(19) **Europäisches Patentamt**

**European Patent Office**

**Office européen des brevets**

(11) **EP 1 005 677 B1**

(12) **EUROPEAN PATENT SPECIFICATION**

(45) Date of publication and mention
of the grant of the patent:
**04.10.2006 Bulletin 2006/40**

(51) Int Cl.:
***G06F 17/00*** (2006.01)

(21) Application number: **98941221.8**

(86) International application number:
**PCT/CU1998/000006**

(22) Date of filing: **24.08.1998**

(87) International publication number:
**WO 1999/010816 (04.03.1999 Gazette 1999/09)**

(54) **SYSTEM AND METHOD FOR THE TOMOGRAPHY OF THE PRIMARY ELECTRIC CURRENT OF THE BRAIN AND THE HEART**

ANLAGE UND VERFAHREN FÜR DIE TOMOGRAPHIE VOM PRIMÄREN ELEKTRISCHEN STROM DES GEHIRNS UND DES HERZENS

SYSTEME ET PROCEDE POUR LA TOMOGRAPHIE DU COURANT ELECTRIQUE PRIMAIRE DU CERVEAU ET DU COEUR

(84) Designated Contracting States:
**AT BE CH CY DE DK ES FI FR GB GR IE IT LI LU MC NL PT SE**

(30) Priority: **22.08.1997 CU 8797**

(43) Date of publication of application:
**07.06.2000 Bulletin 2000/23**

(73) Proprietor: **CENTRO NACIONAL DE INVESTIGACIONES CIENTIFICAS**
**Ciudad de la Habana 12100 (CU)**

(72) Inventors:
 • **VALDES SOSA, Pedro Antonio**
   **Ciudad de la Habana 11600 (CU)**
 • **BOSCH BAYARD, Jorge Francisco**
   **Edificio 131**
   **Ciudad de la Habana 12100 (CU)**
 • **AUBERT VAZQUEZ, Eduardo Francisco**
   **Ciudad de la Habana 10400 (CU)**
 • **VIRUES ALBA, Calle 39, no.273, Apto.3M,**
   **Ciudad de la Habana 10600 (CU)**
 • **MORALES AGUILERA, Frank**
   **Apartamento D**
   **10 de Octubre**
   **Ciudad de la Habana 12300 (CU)**
 • **TRUJILLO BARRETO, Nelson Jesús**
   **Sancti Spiritus 62300 (CU)**
 • **FUENTES MONTERO, Maria Elena**
   **Calle 30 No. 773**
   **Ciudad de la Habana 10600 (CU)**
 • **SOLER MCCOOK, Jorge Miguel**
   **Ciudad de la Habana 11300 (CU)**
 • **RIERA DIAZ, Jorge Javier**
   **Guira de Melena 33600 (CU)**

(74) Representative: **Prins, Adrianus Willem et al**
   **Vereenigde,**
   **P.O.Box 87930**
   **2508 DH Den Haag (NL)**

(56) References cited:
   **EP-A- 0 527 482          US-A- 5 370 126**

 • **PHILIPS J W ET AL: "IMAGING NEURAL ACTIVITY USING MEG AND EEG" IEEE ENGINEERING IN MEDICINE AND BIOLOGY MAGAZINE, vol. 16, no. 3, May 1997, pages 34-42, XP000700726**
 • **DILLENSEGER J L ET AL: "FUNCTIONAL AND MORPHOLOGICAL DATA FUSION IN ELECTROENCEPHALOGRAPHY" PROCEEDINGS OF THE ANNUAL INTERNATIONAL CONFERENCE OF THE ENGINEERI IN MEDICINE AND BIOLOGY SOCIETY, PARIS, OCT. 29 - NOV. 1, 1992, vol. 5, no. CONF. 14, 29 October 1992, page 1022/1023 XP000514524 MORUCCI J P;PLONSEY R; COATRIEUX J L; SWAMY LAXMINARAYAN (ED**

- **CZANNER S ET AL: "Correlation of the Talairach atlas based on the proportional grid system" PROCEEDINGS. TENTH IEEE SYMPOSIUM ON COMPUTER-BASED MEDICAL SYSTEMS (CAT. NO.97CB36083), PROCEEDINGS OF COMPUTER BASED MEDICAL SYSTEMS, MARIBOR, SLOVENIA, 11-13 JUNE 1997, pages 109-113, XP002087782 ISBN 0-8186-7928-X, 1997, Los Alamitos, CA, USA, IEEE Comput. Soc. Press, USA**

## Description

### Technical field

The present invention consists of a system and method for obtaining Tomographic Images of the Primary Electric Currents (PEC) produced by the neurons of the brain and the muscle cells of the heart.

### BACKGROUND

In this invention reference will be made to phrases and terms used of art that will be defined as follows.

Vectors will be denoted with boldface lowercase letters and matrices with uppercase boldface letters. $\mathbf{1}_N$ is the vector of dimension N of ones, $\mathbf{I}_n$ denotes the identity matrix of order n, $\otimes$ the matrix Kronecker product; ".*" the Hadamaard product, and o represents the dyadic product among vectors. If x is a matrix or a vector, $x^t$ denotes x transposed x; $x^*$ denotes x conjugate transposed. The operation vech(X) applied to X consists of forming a vector with the columns of X, eliminating repeated elements or those considered constant in the context of the problem.

Primary Electric current (PEC) $\mathbf{j}_P(\mathbf{r},t)$: is the macroscopic magnitude obtained by the spatial and temporal average of the post-synaptic activity of a group of nervous or heart cells at the position $\vec{r}$ and the instant of time t.

Volume Conductor $\Omega_v$: region inside the body that will be object of study , the head, or the torso.

Generating volume $\Omega_g$: subset of $\Omega_v$ where the PEC originates, consisting of either the brain or the heart.

Lattice of the Volume Conductor $\mathfrak{R}_\mathbf{V}$: discrete group of $N_v$ points $\mathbf{r}_v \in \Omega_v$

lattice of the Generating volume $\mathfrak{R}_\mathbf{g}$: discrete group of $N_g$ points $\mathbf{r}_g \in \Omega_g$.

Analysis interval $\mathfrak{S} = [0, T]$ is the period defined from a reference fixed arbitrarily at the instant 0 until time T.

PEC Tomographic Image (TPEC): vector $\mathbf{j}(t) = \left[\mathbf{j}_P\left(\vec{r}_g, t\right)\right]_{1 \leq g \leq N_g}$ defined at the points $\mathbf{r}_g \in \Omega_g$, and for each instant $t \in \mathfrak{S}$ of time.

Electroencephalogram (EEG) and Electro-cardiogram (EKG): time series obtained by measuring the voltage difference $V_{er}(t)$ that is created in a pair of electrodes localized on the body in the positions $\mathbf{r}_e$ (recording electrode) and $\mathbf{r}_r$ (reference) on the head and the thorax respectively. $V_{er}(t)$ is measured at $N_e$ sites on the body. A vector of measurements of this type will be denoted by v(t).

Magneto-encephalogram (MEG) and Magneto-cardiogram (MKG): time series obtained by measuring the projection $b_{cn}(t)$ of the magnetic field density vector at the center $\mathbf{r}_c$ of a simple coil on $\mathbf{n}_c$, the perpendicular vector to the plane that contains it. These simple coils are connected to Superconducting Quantum interference Devices (dc SQUID) by means of a group of magnetic flow detection transformers. $b_{cn}(t)$ are measured at $N_c$ sites of the body. The vector of measurements of this type will be denoted by b(t).

Anatomical images: type of medical images that offer structural information about the body such as the Computed Axial Tomography (CAT), of Magnetic Resonance Images (MRI), post-mortem sections of the head using cryotomy, etc.

Anatomical atlas: is an anatomical image of the brain or of the heart in a reference system of the head or the torso, respectively. A particular instance of a reference system for the brain is the "Talairach" international system. Possible types of anatomical atlases are:

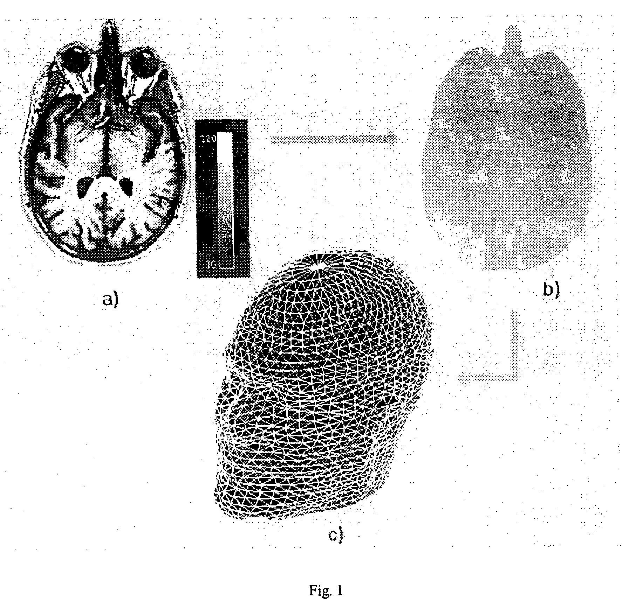

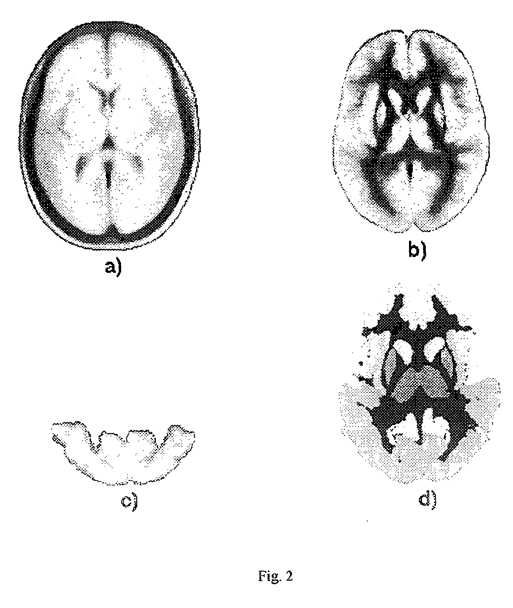

Individual, structural images of the subject under study (Figure 1).
Probabilistic: a composite statistical image that summarizes the inter-individual variability of normal or pathological morphologies of anatomical images in a given population (Valdés P. and Biscay R. The statistical analysis of brain images. In: Machinery of the Mind, E. Roy John et al. the. (ed.), (1990). Birkhauser, pp. 405-434.; Collins DL, Neelin P, Peter TM, Evans AC (1994) Automatic 3D registration of MR volumetric dates in standardized talairach space. J Comput Assist Tomogr 18(2): 192—205, Czanner S et al: "Correlation of the Talairah atlas based on the proportional grid system", Proceeding tenth IEEE Symposium on Computer based medical systems, Maribor Slovenia, 11-13 June 1997, pages 109-113; Evans AC, Collins DL, Mills SR, Brown ED, Kelly RL, Peters TM (1993) 3D statistical neuroanatomical models from 305 MRI volumes. Proc. IEEE-nuclear Science Symposium and Medical Imaging Conference: 1813-1817; Evans, A.C., Collins, D.L., Neelin, P. , MacDonald, D., Kambei, M., and Marret, T.S. (1994) Three dimensional correlative imaging: Applications in human brain mapping. In R. Thatcher, M. Hallet, T. Zeffiro, E. Roy John and M. Huerta (Eds.) Functional neuroimaging technological foundations. Academic Press).

Functional images: types of medical images that offer information on the hemodynamics corporal metabolism such as Functional Magnetic Resonance (fMRI), Positron Emission Tomography (PET) and the Single Photon Emission Tomography (SPECT).

Observations $\mathbf{o}(t) = [\mathbf{o}(t)_m]_{1 \leq m \leq M}$ : the group of M measurements of different modalities (EEG, MEG, and functional image) that will be used for the construction of the TPEC. All the measurements are referred to the reference system of the selected anatomical atlas. The EEG/EKG ($\mathbf{o}_1(t)$) and/or the MEG/MKG ($\mathbf{o}_2(t)$) and optionally the fMRI, PET, and SPECT ($\mathbf{o}_m(t)$, m>2) will be always included in $\mathbf{o}(t)$, being these observations defined for the coordinates $\mathbf{y} \in \Delta_m$, where $\Delta_m$ is the Space of Measurement of the modality m.

Multivariate Z Transform of the vector of random variables x $\Theta_x = \{\mu_x, \Sigma_x\}$, their mean vector and matrix of population covariances is defined $\mathbf{z}^x = (\mathbf{x} - \mu_\mathbf{x}) \cdot \Sigma_\mathbf{x}^{-\frac{1}{2}}$. In this last expression one of the square roots of $\Sigma_x$ is selected and if this is not of complete range a pseudoinverse is used. In case that x is of dimension 1, its Z Transform has the simple expression $z^x = \dfrac{x - \mu_x}{\sigma_x}$.

Generalized Gaussian Distribution $\mathbf{N}_t^{k,p}(\mu_\mathbf{x}, \Sigma_\mathbf{x})$: is a probability density proportional to $\exp\left(-\beta \|\mathbf{x} - \mu_\mathbf{x}\|_{\Sigma_\mathbf{x}}^p\right)$, where k is the dimension of the distribution, p the order of the norm in the exponent $\|\mathbf{x} - \mu_\mathbf{x}\|_{\Sigma_\mathbf{x}}^p = |\mathbf{z}_\mathbf{x}|^p$, $\beta$ a proportionality constant, and t the numeric variable type (R real; C, complex). For example, the notation $\mathbf{x} \sim \mathbf{N}_R^{2,p}(\mu_\mathbf{x}, \Sigma_\mathbf{x})$ is used to express that x is a sample from a multivariate real gaussian distribution.

Functional space: is a set of functions with common properties (Triebel, H. 1990. Theory of Function Spaces II. Basel: Birkhauser). Membership in a function space is used to specify desirable properties in the TPEC under consideration. The function spaces are considered to be consisting of the combination of atoms.

Dictionary of Atoms: is a collection of atoms with properties such that all element of a function space can be expressed as $f = \sum_k F_k \cdot \psi_k$ , where $\psi_k$ are the atoms.

Examples are: "Wavelets", (Meyer, Y. 1992. Wavelets and Operators. Cambridge: Cambridge University Press), the Fourier base of complex exponential, the collection of Dirac deltas in a space of generalized functions, etc. A Megadictionary is being the union of several dictionaries of atoms.

Besov Space $B_{n,s}^m(\Omega \to R^n)$: is a collection of functions defined on the space $\Omega$ and taking values in $R^n$ that possess a degree of smoothness specified by means of the triple (m, n, s) (Triebel, H. 1990. Theory of Function Spaces II. Basel: Birkhauser). The requirement that any given function $f$ belongs to $B_{n,s}^m$ shall be imposed by means of penalization with the metric $\|f\|_{B_{n,s}^m} \approx \|f\|_{b_{n,s}^m} = \sum_k a_k \cdot \|F_k\|^m$, that is to say the norm of weighted combination of the atoms (De Vore R.A. and Popov V. (1988) Interpolation of Besov Spaces. Transactions of the American Mathematical Society 305, 397-414). These spaces allow the modeling of functions with controllable degree of spatial inhomogeneity. Particular cases are:

Sobolev Space $B_{2,2}^m = H^m$ , of maximum smoothness (with respect to derivatives of order m).

The "algebra of bumps" $B_{1,s}^1$ (Meyer Y. (1992) Wavelets and Operators. Cambridge: Cambridge University Press) that allows modeling both of point sources (dipoles) and distributed sources in a common framework.

Reference group: consists of a reference group, the membership to which is to be established for a given TPEC image.

Classical quantitative electrophysiology (qEEG/qEKG)

This invention has as antecedents the work directed to quantify and to make objective the detection of abnormal states of the brain and heart by applying multivariate statistical analysis to the EEG and EKG. These methods are known as electroencephalography and quantitative electrocardiography (abbreviated respectively as qEEG and qEKG). Systems and methods for qEEG were described in the patents US 4,846,190; US 4,913,160; US 5,282,474 and US 5,083,571. Methods for qEKG were described in the US Patent 4,974,598. These patents specify:

Recording of the EEG and/or EKG (v(t)) by means of a plurality of sensors.

Optionally, instead of analyzing the v(t) recorded originally, pre-processed series (PPS) are obtained by means of the calculation of the cross-covariance function of v(t) with respect to a time series s(t), marker of external events. In the case of the EEG, s(t) may be taken as a sequence of Dirac delta functions, indicative of the moments at which certain stimuli are presented to the subject examined. The resulting time series is known as the Average Evoked Potential (AEP). In the case of the EKG s(t) may signal the occurrence of the R wave of the EKG itself and the resulting PPS is the average EKG (AEKG).

The extraction of Descriptive Parameters (DP) from these time series, these being statistical sumarizations of the recorded time series designed to reflect variations in normal and pathological physiologicalal activity.

In the mentioned patents the DP specified for the EEG are the averages in broad bands of the frequency of spectrum, elementary transformations of these DP being included also. The DP of the Broad Band Spectral Analysis (BBSA) are defined formally as follows: let $S_o(\omega)$ be the crosspectral or variance and covariances matrices of the coefficients of Fourier of V(t) (where $\omega$ denotes frequency). The DP-BBSA are: $\int_a^b s_{o,ij}(\omega)d\omega$, where a and b specify the limits of the broad band and $s_{o,ij}(\omega)$ is the element i, j of the matrix $S_o(\omega)$.

In the patents cited the DP for the AEP and AEKG are defined as the coefficients of a Karhunen-Loeve bases. These parameters are known as Factorial Analysis loadings.

In the patents cited, transformations of the DP are carried out to guarantee their gaussianity. In the description of this invention a generic transformed scalar DP will be denoted with the letter x=T(DP) and a vector with the notation **x**=T(DP), after the transformation $\mathbf{x} \sim N_R^{2,p}\left(\mu_\mathbf{x}, \Sigma_\mathbf{x}\right)$.

In the patents cited, a comparison of the DP is carried out with respect to the normal variability (inferred from normative databases) by means of the univariate z transform that expresses the DP as a deviation measured in units of standard deviation $\sigma_x$, with respect to the populational average $\mu_x$. In the calculation of the $z_x$ based on the normative database, the effect of concomitant variables that originate variability that is not of diagnostic interest (as is the case of the subject's age), is eliminated by means of homoscedastic polynomial regression.

The construction of brain and heart Topographic Maps (TM). In these TM the degree of deviation of the subject with respect to the norm is coded by means of a color scale. The TM is an image interpolated between the measurements of the sensors, which represents a schematic two-dimensional projection of the head or of the torso.

The use of cluster analysis, to define groups of similar subjects according to the values of their DP (US Patent 5,083,571).

The use of linear discriminant analysis to classify a subject examined as belonging to a test group or a test group previously defined by cluster analysis on the basis of predefined values of their DP (US Patent 5,083,571).

The measurement of the statistical distances of a subject with respect to his own previous states carried out at selected moments. This offers information on the subject's physiological state, for example, in the following conditions: during an operation, while in intensive therapy, or while in evaluation for the evolution of pathology (US patents 4,545,388; 4,447,270; 4,815,474; 4,844,086 and 4,841, 983). The utility of these methods has been confirmed in several studies (John, E.R.; Harmony, T. Valdés-Sosa and, P. (1987b): The uses of statistics in electrophysiology. In: Gevins, A.S. and Rémond, A. (Eds), Handbook of Electroencephalography and Clinical Neurophisiology. Revised Series. Volume 1, Elsevier, The Netherlands, 497-540 and John, E.R.; Prichep, L.S. and Easton, P. (1987a): Normative data bases and neurometrics. Basic concepts, methods and results of norm construction. In: Gevins, A.S. and Rémond, A. (Eds), Handbook of Electroencephalography and Clinical Neurophisiology. Revised Series. Volume 1, Elsevier, The Netherlands, 449-496). However, these methods have the following limitations:

The DP-BBSA are insufficient as descriptive parameters for the quantification of many types of activities of the EEG. This is due to the loss of resolution caused by the averaging over bands in frequency, with the resulting loss of sensitivity and specificity in diagnoses (Szava, S.; Valdés, P.; Biscay, R.; Galán, L.; Bosch, J.; Clark, I. and Jiménez, J.C.: High Resolution Quantitative EEG Analysis. Brain Topography, Vol. 6, Nr. 3, 1994, pp. 211-219).

The analysis of a group of DP by use of TM is hindered by the high correlation among the variables. To overcome this difficulty, Galán et al. (Galán, L.; Biscay, R.; Valdés, P.; Neira, L. and Virués T. (1994): Multivariate Statistical Brain Electromagnetic Mapping. Brain Topography, vol 7. No.1) introduced the Multivariate TM by means of the use of the multivariate Z transform.

The evaluation of the normality of the TM is carried out by means of univariate statistics without taking into account the need for the control of Type I errors caused by the multiple comparisons for all point of the map. This increases to uncontrollable levels the probability of false anomaly detection.

The procedure for the statistical classification of subjects is based on the assumption that $\mathbf{x} \sim \mathbf{N}_R^{2,P}\left(\mu_{\mathbf{X}}, \Sigma_{\mathbf{X}}\right).$

This implies that the decision frontiers between the groups are linear which is too restrictive. In addition, optimal procedures for the selection of the DPs with highest classification power have not been applied.

The use of magnetic measurements b(t) are not included, in spite of the fact that these add additional information to that provided by $\mathbf{v}$(t).

The analysis of physiological information is limited to v(t), without there being any inference about j(t). Therefore, none of these methods constitutes a modality of TPEC. They are merely an analysis of projections of j(t) on the surface of the body after being distorted by the tissues of the subject interposed between the generators of the neural/cardiac PEC and the sensors. In fact, the relationship between the PEC and the EEG/MEG/EKG/MKG depends on the conductor properties of the head and the torso (e.g. geometry, conductivity, electric and magnetic permeability, etc.).

Cerebral and Heart Multivariate Statistical maps

Some of these limitations were overcome by the US patent 5,282,474 of Pedro Valdés-Sosa et al.. In this patent the use of statistical methods is claimed for the evaluation of the abnormal activity of the brain and of the heart with the following innovations:

> $\mathbf{b}$(t) is added as an optional source of physiological information. In what follows the use of DP obtained both from $\mathbf{v}$(t) and $\mathbf{b}$(t) will be included in the definition of qEEG and qEKG.

Additional components of the time series $\mathbf{s}$(t) are included in the form of markers of external events of more general type such as can be derived from the subject's own voluntary or involuntary reactions, or those obtained by the analysis of their own $\mathbf{o}$(t). The series derived by such a pre-processing will be named the Event Related Components (ERC) derived from $\mathbf{o}$(t).

Expansion of the ERC in tensor products of functional space-time bases. These include not only the basis of Fourier, but also wavelets (for the description of non-stationary processes) among other sets of DP that increase flexibility in the description of physiological processes.

Summarize inclusive of these DP by means of the use of higher order statistical moments and different parametric time series models. In particular the coefficients of linear autorregresive are included.

The introduction as DP of High Resolution Spectral Analysis (HRSA) consistent in the use of $S_o(\omega)$ for all the frequencies $\omega$ according to the one transformed of discrete Fourier.

The introduction in the TM of color scales based on the empirical probability distribution of the global maxima and global minima of the DP. This scale exercises an effective control of the probability of false pathology detection.

The improvements for the evaluation of brain activity introduced in this patent were exemplified:

> By means of the construction of norms of HRSA for the Cuban population from 5 to 97 years (Valdés, P.; Biscay, R.; Galán, L.; Bosch, J.; Szava, S.; and Virues, T.: High Resolution Spectral EEG norms for topography. Brain Topography, 1990, vol. 3, pp. 281-282 and Valdés, P.; Bosch, J.; Serious of it Banks, R.; Hernández, J.L.; Pascual, R. and Biscay, R.: Frequency domain models for the EEG. Brain Topography, 1992a, vol. 4, pp. 309-319).

By the showing that the DP-HRSA achieve a higher sensitivity and specificity than the DP-BBSA for the detection of neurological and psychiatric pathology (Szava, S.; Valdés, P.; Biscay, R.; Galán, L.; Bosch, J.; Clark, I. and Jiménez, J.C.: High Resolution Quantitative EEG Analysis. Brain Topography, Vol. 6, Nr. 3, 1994, pp. 211-219).

However, the construction of DP of o(t) used in the patent cited is limited to such parametric models as the DP-HRSA. These are only a complete description of o(t) for stationary and linear stochastic signals or these with limited types of nonlinearity. The inadequacy of such DP for the study of the EEG of some types of patient (in particular those with epilepsy) was demonstrated by Hernández, Valdés-Sosa and Vila (Hernández, J,L., Valdés, P.A., and Vila, P. (1996) EEG spike and wave modeled by to stochastic limit cycle. NeuroReport, 7: 2246-2250).

qEEG methods and qEKG are not applied to estimates of j(t), for this reason they are not true variants of TPEC.

Tomography of Cerebral and Heart Primary Electric Currents

As in all type of Tomography, the starting point for the development of the TPEC is the establishment of a model that relates the observed data with the quantity to estimate, this model known as the Direct Problem. In the TPEC the direct problem postulates how the **o**(t) are generated the from **j**(t). This model has two components:

The specific model of Volume Conductor assumed, that is to say, of the conductive properties of the head and the torso, in particular their geometry, conductivity, electric and magnetic permeability, etc.

The model that is assumed for **j**(t), or source model.

The properties of the Volume Conductor are summarized in the electric $\mathbf{k}^E(\mathbf{r})$ and magnetic $\mathbf{k}^M(\mathbf{r})$ Lead Fields" (LF) . These are the kernels of Fredholm integral equations of the first kind that establish direct relationships between the PEC $\mathbf{j}_p(\mathbf{r},t)$ with $V_{er}(t)$ and $b_{cn}(t)$.

$$V_{er}(t) = \int_{\Omega_v} \mathbf{k}^E(\mathbf{r}) \cdot \mathbf{j}_P(\mathbf{r},t) d\mathbf{r}^3 \tag{1}$$

$$b_{cn}(t) = \int_{\Omega_v} \mathbf{k}^M(\mathbf{r}) \cdot \mathbf{j}_P(\mathbf{r},t) d\mathbf{r}^3 \tag{2}$$

The discretization of the terms expressed in equations (1) and (2) lead to the following formulation of the direct problem:

$$\mathbf{o}(t) = \mathbf{K} \cdot \mathbf{j}(t) + \mathbf{e}(t) \tag{3}$$

where K is the discretized LF and $\mathbf{e}(t) \sim \mathbf{N}_t^{k,p}(0, \Sigma_{EE})$, being the error introduced at the sensors in the moment of the measurement.

The use of the LFs to formulate the Direct Problem instead of using the classic Green formulation results in remarkable advantages according to Rush and Driscoll (Rush S. and Driscoll D.A. EEG electrode sensitivity-an application of reciprocity. IEEE Trans. on Biomed. Eng., vol. BME-16, pp. 15-22, 1969) and Plonsey (Plonsey R. Capability and limitations of electrocardiography and magnetocardiography. IEEE Trans. Biomed. Eng., vol. BME-19, not. 3, pp. 239-244, 1972). This is mainly because all the conductive properties of the head or the thorax can be summarized in these magnitudes without assuming a specific model for the PEC. However, it is well-known that the conductivity in the body is non homogeneous and non isotropic (Hoeltzell P.B. and Dykes R.W. Conductivity in the somatosensory cortex of the cat. Evidence for cortical anisotropy. Brain Research, 117, pp. 61-82, 1979), a fact that complicates the calculation of the LFs considerably. Nevertheless, a substantial simplification is possible when the conductivity is considered constant and isotropic in each tissue type, which constitute a compartment (Schwan H.P. and Kay C.F. The conductivity of living tissues. Ann. N.Y. Acad. Sci., vol. 65, pp. 1007-1013, 1957). This model of Volume Conductor is denominated Isotropic and Piecewise Homogeneous Volume Conductor (IPHC). In general, two types of model IPHC are in use for the head or the torso:

Spherical: the simplest model that specifies that the surfaces defining the different compartments are concentric spheres. For this model explicit formula exist to evaluate K. This model is easy to evaluate but not very exact, mainly for the temporal regions of the head.

Realistic: in which the surfaces of the compartments are obtained from an anatomical atlas. For this model numeric methods have been developed by Fletcher et al. (Fletcher D.J., Amir A., Jewett D.L. and Fein G. Improve method for the calculation of potentials in a realistic head shape model. IEEE Trans. Biomed. Eng., vol 42, not. 11, pp. 1094-1104, 1995) and Oostendorp and van Oosteroom (Oostendorp T. and van Oosterom A. The potential distribution generated by surface electrodes in homogeneous volume conductive of arbitrary shape. IEEE Trans. Biomed. Eng., vol. BME-38, pp. 409-417, 1991). However, this procedures is based on a boundary element method (BEM) for the calculation of the potential difference on the surfaces that limit the compartments produced by energizing the sensors.

The expression:

$$R_O = \left\| \mathbf{o}(t) - \mathbf{K} \cdot \mathbf{j}(t) \right\|_{\Sigma_{EE}}^{p_o} \tag{4}$$

is the functional of the Direct Problem in the norm $p_o$. $l(\mathbf{o}(t)|\mathbf{j}(t),\Sigma_{EE}) = C_0 \exp[-R_0]$ is defined as the likelihood corresponding to equation (3), being $C_0$ the constant of normalization of the density. In what follows, unless otherwise stated, $p_o$=2.

The central objective of the TPEC is the estimation of $\mathbf{j}(t)$ by means of the minimization of (4), which is the "Inverse Problem". It is well known that (4) lacks, in general, a unique solution since the LF operator has a non-trivial null space, even when simultaneous electric and magnetic measurements are available.

Estimation of the PEC is possible only if a priori information is contributed in the form of a model for $\mathbf{j}(t)$ that impose a series of restrictions. These may be classified as:

Extrinsic: consisting of requiring the compatibility of the desired solution with information provided from other types of neuroimages. About the cerebral anatomical neuroimages typical requirements are:

That j(t) be estimated only inside the spherical Volume Conductor that models the head.

That j(t) be restricted to the cortical surface of the of the subject with an orientation perpendicular to said cortex (Dale, A.M., and Sereno and, M.I, J. Cognit. Neurosc., 1993, 5:2, pp. 162-176).

Intrinsic: consisting of requirements about the shape and extent of areas of activated tissue. It is required that the activated areas are as small as possible while maintaining compatibility of the model with the data (simplicity). As for the variation in the shape in the space, j(t), this requirement has ranged from minimal smoothness (collections of Dirac deltas corresponding to current dipoles) to functions that are maximally smooth.

All the above stated conditions are imposed on the solution in two equivalent ways:

Regularization: Adding to (4) a non-negative functional $R_J = R_J(\mathbf{j}(t)|\Theta)$ and minimizing

$$R_O + \lambda \cdot R_J\left(\mathbf{j}(t)|\Theta\right) \tag{5}$$

Bayesian estimation: Maximizing the a posteriori distribution

$$P\left(\mathbf{j}(t),\Sigma_{EE}|\mathbf{o}(t)\right) \propto l\left(\mathbf{o}(t)|\mathbf{j}(t),\Sigma_{EE}\right) \bullet \pi\left(\mathbf{j}(t)|\Theta\right) \tag{6}$$

where $\pi(\mathbf{j}(t)|\Theta) = C_j \exp[-R_j]$ is the a priori probability of $\mathbf{j}(t)$.

The fundamental differences in the different methods that have been developed to estimate the generators of $\mathbf{o}(t)$ are in the model specified for $\mathbf{j}(t)$. These are reviewed as follows:

When $R_j(\mathbf{j}(t)|\Theta)$ is a functional that guarantees that $N_g < \Theta_o$, being $\Theta_o$ a constant that assures the uniqueness of the solution of the equation (4). This class of models for $\mathbf{j}(t)$ is denominated current dipoles (Scherg, M. and Ebersole, J.S. (1993): Models of Brain Sources. Brain Topography. Vol. 5, Nr. 4, pp. 419-423).

$$\mathbf{j}(r,t) = \sum_{g=1}^{N_d} \alpha_g(t)\mu_g(t)\delta\left(\mathbf{r} - \mathbf{r}_g\right) \tag{7}$$

Where $\alpha_g(t)$ is the activity of the generator g and $\mu_g(t)$ its orientation. A model of this type was described in the US patents 4,949,725; 5,285,385; 5,524,086, and 5,361,774. The advantages of these models are:

They are effective for modeling situations in which a small group of regions is producing PEC, all of small extent.

The formulation leads to simple least squares estimation methods.

The set of resulting DP of the estimates is very compact, comprising the positions and orientations of each current dipole.

Due to the simplicity of the model, it is easy to include additional restrictions to the estimation of this model of PEC (Scherg, M. and Ebersole, J.S. (1993): Models of Brain Sources. Brain Topography. Vol. 5, Nr. 4, pp. 419-423). For example a certain degree of smoothness may be imposed on $\alpha_g(t)$ and the requirement that, $\mu_g(t)=\mu_g$ does not depend on time. These requirements stabilize the estimated DP.

Nevertheless, the dipolar models discussed have the following limitations:

When the number of possible generators of PEC ($N_g$) becomes too large, this type of model requires of an excessive manual intervention of the operator. For this case, the inverse problem becomes again ill determined. In fact, these models do not include a statistical approach for determining $N_g$.

When the different regions of tissue that generate PEC are distributed in extensive areas, the dipolar model estimates an equivalent dipole located at the center of mass of the actual region, and therefore produces an artifact

The smoothness of $\alpha_g(t)$ has been imposed by use of a spline basis, that limits its possible forms of the amplitude of the activity to belong to a Sobolev functional Space $W^2(L^2)$, (second derivative integrable Lebesgue).

This type of model has been used solely to describe the AEP or AEKG.

This model for generators has been applied using spherical and realistic models of the Volume Conductor. However, the LF methodology has not been used, which necessitates the use of numerical algorithms for the realistic case that are not very practical since they require the evaluation of a step of the BEM in each iteration.

A TPEC was proposed for the first time in the US Patent 5,307,807 of Valdés-Sosa et al., giving place to a new modality of medical image. This invention consists on a method and system to create three-dimensional maps of the amplitude, orientation and connectivity of the generators of neural/cardiac PEC with the following innovations:

An estimate of j(t) on a three-dimensional lattice inside the body is carried out.

DPs of the PEC of the CRE is considered.

The information available from other types of Structural Medical images is used to specify the conductive properties of the subject's body. It is also used to define the spatial extent of a lattice for which PEC are defined (thus limiting the possible sites where the PEC are to be estimated) and to determine the orientation of the PEC. The subject's corporal geometric characteristics are incorporated in the TPEC in a quantitative manner with the use of parametric models.

The use of information on the metabolism of the subject under study, available from other types of functional medical images, in order to limit even further the lattice on which **j**(t) is to be estimated.

The generators that are modeled include not only discrete sources (dipoles) but also diffuse generators that desribe background generator noise, in occasions of higher amplitude than the discrete generators.

Frequency domain estimation is introduced for the generators of CRE, in particular the cross-spectral matrix, $S_j(\omega)$, of the generators is introduced.

The improvements for the evaluation of brain activity introduced in this patent were later verified by studies on the origin of abnormal electric activities in patient with focal neurological lesions. See for example Harmony, T.; Fernández-Bouzas, A.; Marosi, E.; Fernández, T.; Valdés, P.; Bosch, J.; Riera, J.; Rodríguez, M.; Reyes, A.; Silva, J; Alonso, M. and Sánchez, J.M. (1995): Frequency Source Analysis in Patients with Brain Lesions. Brain Topography, vol 8. No.2.

However, the clinical works mentioned previously and others (Valdés, P.; Carballo, J.A. ;Alvarez, A.; Dfaz, G.F.; Biscay, R.; Pérez, M.C.; Szava, S.; Virués, T. and Quesada, M.E.: qEEG in to public Health System. Brain Topography, Vol. 4, Nr. 4, 1992b, pp. 259-266) showed that the inventions described in the patents US 5,282,474 and US 5,307,807 did not take into consideration the following aspects, necessary for a more complete methodological elaboration of the TPEC.

The statistical procedures described in the US Patent 5,282,474 were not extended for use in the TPEC. In particular, the sample space of the TPEC is a stochastic field defined on a 4 dimensions space time manifold, thus requiring for its analysis specific methods which were not taken into consideration in said patent.

The regression methods used in the US Patent 5,282,474 to eliminate the effect of experimental concomitant variables are of a parametric global polynomial type. These are not sufficiently flexible for the description of the variations at multiple scales typical for electrophysiologicalal data.

In the case of the US Patent 5,307,807, the only regularization resource consists of the control of the number of dipoles whose parameters are considered. This is an implicit use of the functional $R_J(\mathbf{j}(t)|\Theta)$ that guarantees that $N_g < \Theta_o$, which is not very flexible and requires of an excessive human intervention.

In the case of the US Patent 5,307,807, the solution for the direct problem that relates the activity of the generators with the observable signals is achieved with anatomical deconvolution. This method does not adapt to general configurations of generators and is only an inexact numeric approximation to the optimal solution.

In the case of the US Patent 5,307,807, the parametric description employed to characterize the geometry of the subject's body is based on the use of quantitative descriptors of a functional form that are not sufficiently flexible to adequately describe inter-individual morphometric variability. In particular this method does not allow the inclusion of probabilistic anatomical information in those cases for which the acquisition of structural images of the particular subject under study is either undesirable or impracticable.

In the case of the US Patent 5,307,807, the metabolic information available from other image modalities is only integrated as a restriction to the regions in which active dipoles will be allowed. Thus this information is not used in a statistically optimal estimation procedure.

Since the publication of the two aforementioned patents a series of works have appeared, that while not solving the problems outlined above totally, have contributed to the elaboration of the solutions that are claimed in the present invention. The relevant developments are enumerated next, accompanied by an evaluation of their advantages and inadequacies.

The DPs used in the construction of all the TPEC until the moment are either the raw data or sufficient statistics, derived from the data, almost always some of the lower order statistical moments in the time or frequency domain. Recent works (Hernández, J,L., Valdés, P.A., and Vila, P. (1996) EEG spike and wave modeled by to stochastic limit cycle. NeuroReport, 7: 2246-2250) demonstrate the need for flexible non-parametric descriptions for the temporal sequence of the EEG/MEG/EKG/MKG that allow the description of the dynamic activity, essentially nonlinear, that is characteristic of masses of neural and heart tissue.

The case $R_J\left(\mathbf{j}(t)\,\middle|\,\Theta\right) = \left\|\mathbf{j}(t)\right\|_{\Sigma_{JJ}}^{p_J}$ includes classical regularization, which allows models of $\mathbf{j}(t)$ in which the activated areas can be extensive and with smooth contours. In particular Linear Distributed Solutions are limited to estimate j(t) for each time **t** separately. They assume that $R(t) \sim \mathbf{N}_R^{k,p_0}\left(0, \Sigma_{EE}\right)$ and that $\mathbf{j}(t) \sim \mathbf{N}_R^{k,p_J}\left(0, \Sigma_J\right)$ with $p_o$ and $p_J$=2. In consequence, the estimator of generic TPEC is formulated as a Spline type solution (Riera, J.J., Aubert, E., Valdés, P., Casanova, R. and Lins, O. Discrete Spline Electric-Magnetic Tomography (DSPET) based on Realistic Neuronatomy. To appear in the proceedings of The Tenth International Conference on Biomagnetism, BIOMAG'96, Santa Fe, New Mexico, February 1996) where the smoothness of the solution is specified by the matrix $\Sigma_{JJ}$. The estimator of the PEC has, in this case, an explicit expression that is:

$$\hat{\mathbf{j}}(t) = \Sigma_{JJ} \cdot \mathbf{K}' \cdot \left(\mathbf{K} \cdot \Sigma_{JJ} \cdot \mathbf{K}' + \Sigma_{EE}\right)^{-1} \cdot \mathbf{v}(t) \tag{8}$$

The most significant representatives in this family of TPEC are characterized by assuming that $\Sigma_{EE} = \sigma^2_{EE}\,\mathbf{I}$, where $\sigma^2_{EE}$ is a variance common to the sensors of the same type. The different linear solutions are characterized by the $\Sigma_j$ postulated as indicated below.

Minimum Norm TPEC (MN) for which $\Sigma_J = \sigma_J^2 \cdot \mathbf{I}_{3 \cdot N_g}$. This solution requires that the configuration of generators has minimum energy (Wang J.Z., Williamson S.J. and Kaufman L. Magnetic source images determined by lead-field analysis: the unique minimum-norm least squares estimation. IEEE Trans. Biomed. Eng., 39, 7, 665-667, 1992) compatible with the likelihood. This method produces spatially widespread estimates that do not localize discrete sources correctly. Variants of this methodology were presented in the US Patent 5,228,443.

Weighed Minimum Norm TPEC (WMN), $\Sigma_J = \sigma_J^2 \cdot \mathbf{W}^{-2} \otimes \mathbf{I}_3$, where the matrix $\mathbf{W} = diag(w_i)$

$$w_i = \sqrt{\sum_{d=1}^{N_d} \left|\mathbf{K}\right|_{\bullet,d}^2}$$

**W** is diagonal and it contains weights. These counteract a bias that distributed solutions have of concentrating solutions near the sensors (George, J.S, P.S Lewis, H.A Schlitt, L. Kaplan, I. and C.C Wood (1993) Strategies for source space limitation in tomographic inverse procedures. In: Proc. 9th Int. Conf. On Biomagnetism, Vienna). It has been demonstrated that this method avoids spurious superficial solutions but it does not localize discrete sources with more precision.

Low resolution electric Tomography (LORETA) where:

$$\Sigma_J = \sigma_J^2 \cdot \left( \Lambda_m \cdot L^2 \cdot \Lambda_m \right)^{-1} \otimes I_3$$

$\Lambda_m = W$, $L_3 = L \otimes I_3$, $L$ being the matrix of the discretized Laplacian. This solution imposes the maximum spatial smoothness compatible with the likelihood term (Pascual-Marqui, R.D., 1994. Low resolution electromagnetic tomography: a new method for localizing electrical activity in the brain, Int. J. Psychophysiol. 18:49-65. LORETA). This method localizes a single point source accurately in any part of the brain or heart. LORETA has been extended for the estimation of the cross-spectrum of the generators of the EEG. See paper by Casanova et al. (Casanova, R., Valdés P., Garcia F. M., Aubert E., Riera, J.J., Korin W. and Lins O. Frequency domain distributed inverse solutions. To appear in the proceedings of The Tenth International Conference on Biomagnetism, BIOMAG'96, Santa Fe, New Mexico, February 1996). This is based on the following estimator:

$$S_J^{\frac{1}{2}}(\omega) = \Sigma_J \cdot K' \cdot \left( K \cdot \Sigma_J \cdot K' + \Sigma_E \right)^{-1} \cdot S_v^{\frac{1}{2}} \tag{9}$$

However, (9) it does not correspond to a complete Bayesian model, since an a priori probability is specified for $j(t)$, but not for $\Sigma_j$.

In some cases, additional restrictions can be imposed to obtain the well-known Backus and Gilbert solution as described in the US Patent 4,977,896.

All these Spline methods specify the membership of $j(t)$ to a Sobolev Space $H^q(L^2)$, for integer q, which is too restrictive since the estimation of dipolar types of PEC are not allowed. In fact, the Raleigh limit is valid which states the impossibility of discriminating nearby point sources. This limit is the cause, additionally, of the appearance of "ghost" solutions that are interference artifacts due to the limited resolution of linear methods.

In search of less widespread solutions, several authors have proposed non-linear estimators of $j(t)$. Among these 1) Matsuura and Okabe (Matsuura K. and Okabe Y. (1995) Selective Minimum-Norm Solution of the Biomagnetic Inverse Problem. IEEE Trans. BME Vol. 43 not. 6 pp. 608-615) have varied of $p_o$ and $p_j$ in the Generalized gaussian distributions that define the inverse problem with the consequence that the inverse solution must now be estimated non-linearly. 2) Gorodnitsky and Bhaskar (Gorodnitsky, I. and Bhaskar D.R. 1997. Sparse signal reconstruction from limited data using FOCUSS: a reweighted minimum norm algorithm. IEEE Trans. Signal Proc. Vol. 45 (3) pp 600-616) have proposed to apply iteratively the estimator of equation (5) weighting each lattice point proportionally to the $j(t)$ value estimated in the previous step. Both proposals converge algorithmically to a number of discrete generators equal to the number of sensors, therefore being in effect a more elaborate form of dipole fit. In consequence, they share with these dipolar models the defect that they can not estimate spatially distributed sources.

Philips et al.. (Philips JW, Leahy RM and Mosher JC. MEG based imaging of focal neuronal current sources. (1997). IEEE trans Medical Imaging, Volume 16: 338-348 and Philips JW et al: "Imaging neutral activity using MEG and EEG" IEEE Engineering in medicine and Biology Magazine, vol. 16, no. 3, May 1992, pages 34-42) introduced for the model (6) the functional

$$R_J\left( j(t) | \Theta \right) = \left\| \psi(t) \right\|_{\Sigma_{\Psi\Psi}}^{P_\Psi} + V\left( z(t) \right)$$

$$\tag{10}$$

where $j(t) = \psi(t).*(z(t) \otimes 1_3)$. This functional penalizes the lack of smoothness of $\Psi(t)$ in the metric of $\Sigma_{\Psi\Psi}$. It also imposes the additional requirement that there are few active $Z_j$ (which is controlled by the weights $\alpha_1$) which will be concentrated in neighborhoods $\xi_1$ (whose dispersion is controlled by means of the parameters Q and the weights $\beta_j$). This is accomplished by means of the term $\pi_\mu\left( \mu | i \right) = N_R^{3 \cdot Ng, p}\left( 0, \Lambda_m \cdot D \cdot Z \cdot D \cdot \Lambda_m \right)$, The resulting Bayesian hierarchical model is estimated by using the method of "Mean Field Annealing." This model, although potentially very flexible it has only been described for the case $\Sigma_\Psi = \sigma_\Psi^2 \cdot W^{-2} \otimes I_3$.

None of the methods described above models the temporal dependencies of $j(t)$, which is equal to the use of the implicit assumption that $j(t)$ is independent for each t. This assumption can cause considerable bias in the statistical estimates.

It should be mentioned that numerous works carry out processing of **o**(t) images, sometimes taking into account information contributed by other neuroimage modalities, that however do not have as an objective the estimation and ulterior use of **j**(t). Such is the case of the patent from Finland 925,461 and the US patent 5,331,970. Since these patents do not having as an objective the obtaining of a TPEC, these types of antecedents will not be included in this analysis of the art.

## Description of the Invention

The object of the present invention is to provide a system and a method for calculating an estimate of j(t) based on o(t), said estimate being denoted as TPEC. When referring to the study of the brain, specifically Tomography of the Cerebral Primary Electric Current, the procedure will be denoted (TPECc); and in the case of the heart, as Tomography of the Heart Primary Electric Current, (TPECk).

The map is the inverse solution of the EEG/MEG/EKG/MKG problem based upon: a) the restriction of the solution to structures with high probability of generating electric activity using for this restriction an Anatomical Atlas and b) imposing that the solution belong to a pre-specified functional space, of Besov type or is defined by a Megadictionary. The probability is determined that this map, or a subset of the same, belongs to a test group. For this determination, the spatial and temporal correlations of the map are modeled as well as their dependence on experimental covariables. The resulting probabilities are coded in a pseudocolor scale and they are superimposed on an Anatomical Atlas for their interactive three-dimensional visualization. The Atlases used in the solution of the inverse problem and for visualization, can be individual Structural Neuroimages, Probabilistic Atlases, or plastic deformations from a Probabilistic Atlas to the morphology of an individual.

One of the aspects of the present invention consists then, of a System for the Tomography of the Primary Electric Current (TPEC) of the brain (TPECc) and of the heart (TPECk) comprising the following elements:

- a plurality of external sensors of electric and/or magnetic fields placed in the proximity of the subject's body which are connected to the amplification sub-system, in the case of TPECc said sensors being placed in the proximity of the subject's skull and in the case of TPECk said sensors being placed in the proximity of the subject's torso;

- an amplification sub-system consisting of pre-amplifiers and electronic amplifiers that record the signals detected by said sensors and carry out the pre conditioning of said signals for their analog to digital conversion;

- a sub-system of analog to digital conversion that transforms the amplified signals into a sequence of binary numbers o(t) and stores said sequence in the memory of a general-purpose digital computer that serves as a control unit (CU), in the case of the TPECc the time series obtained from the electric sensors is the Electroencephalogram (EEG) and the time series obtained from the magnetic sensors the magnetoencephalogram(MEG), while in the case of the TPECk, the time series obtained from the electric sensors is the electrocardiogram (EKG) and the time series obtained of the magnetic sensors the magneto-cardiogram (MKG);

- a sub-system of external digital storage coupled to the CU that allows the input of an Anatomical Atlas of the brain or of the heart in a reference system of the head or the torso, respectively, as well as the conductance values, electric and magnetic permitivity associated to the tissues described in this atlas, a particular but not exclusive instance of reference system for the brain being the "Talairach" international system.

- a sub-system of external digital storage coupled to the CU, where the CU allows the input of images of functional states of the brain or heart of a subject obtained by other biophysical procedures such as Functional Magnetic Resonance Imaging (fMRI), Positron Emission Tomography (PET) and Single Photon Emission Tomography Photons (SPECT), said functional images being defined in the reference system of the head or of the torso;

- a device for detecting the position of sensors that emits in digital form, the coordinates of these sensors as well as the coordinates of external anatomical structures of the head or of the subject's torso, These coordinates are defined in the reference system of the head or the torso and they are stored in the CU;

- a plurality of stimulating devices connected with the CU that emit auditory, visual sensorial stimuli, as well as heat, electric, and magnetic pulses for the purpose of stimulating the subject's nervous or cardiovascular system;

- a plurality of sensor devices connected with the CU that measure the motor, physiological and verbal reactions of the subject under study;

• a CU in whose memory a group of instructions, or program, resides for the control of the realization of studies, the field of action of this program being among other purposes, the activation of the stimulating devices according to a preset design, the control of the acquisition of the EEG/MEG/EKG/MKG, the determination of the positions of the sensors, the recording of the activity of the subject under study , the acquisition of the images coming from other biophysical modalities and the digital processing of all the information acquired in a study of a subject for the construction of three-dimensional statistical maps of the temporal evolution of the functional states of the brain or heart.

Additionally the present invention provides a calculation method that will be used in conjunction with the aforementioned system, and that it is comprises the following steps:

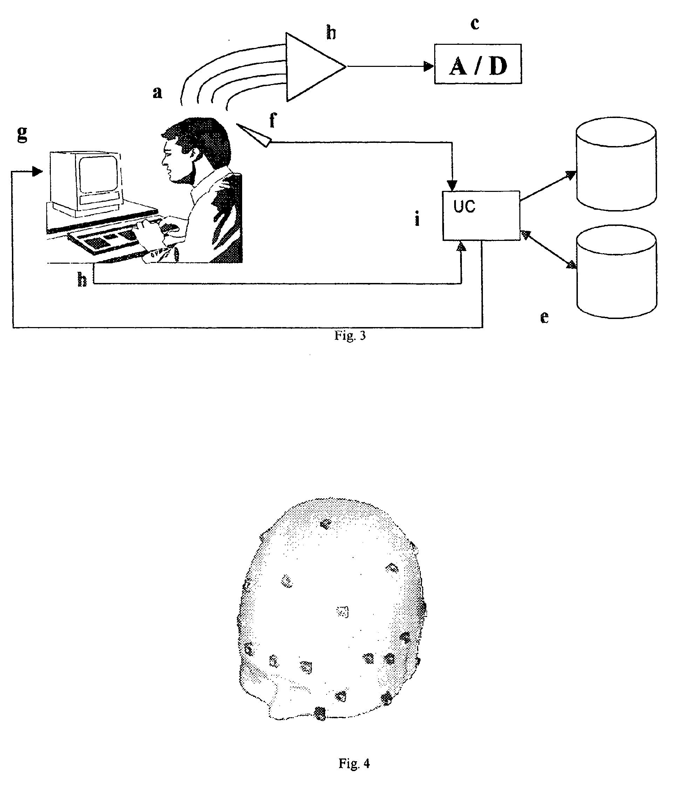

a) Conversion of the coordinates of the sensors to the reference system of the anatomical atlas (Figure 4). This is achieved by means of the application of a viscoelastic deformation, (most generally, non linear) that puts into correspondence the coordinates of the external anatomical markers of the subject with the anatomical atlas stored in the CU.

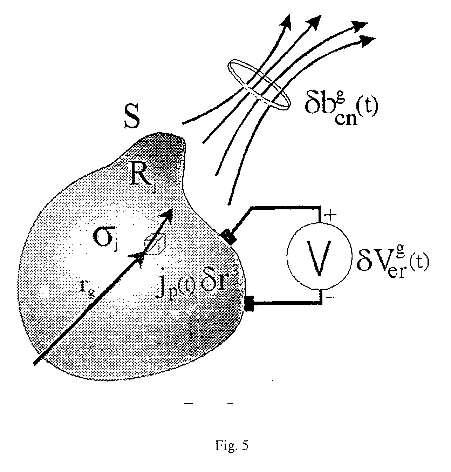

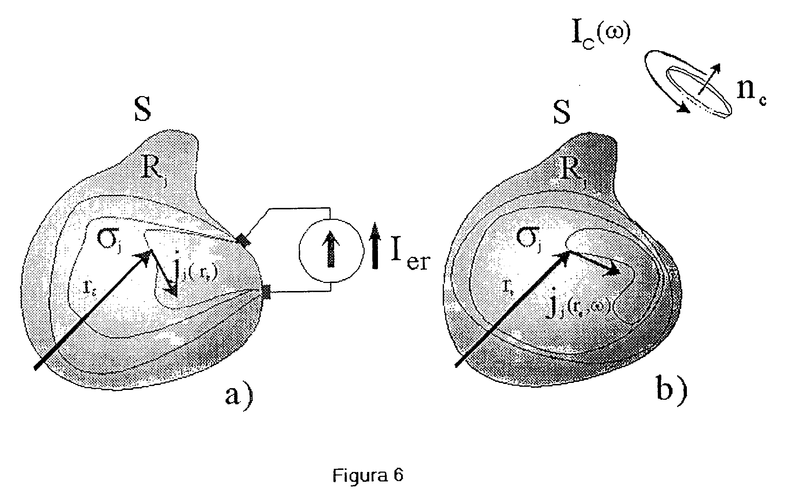

b) Calculation of the linear operators (electric Kernel and magnetic Kernel) that predict the EEG/MEG/EKG/MKG that would be produced in the subject with the presence of PEC in any part of the brain or heart. This Kernel is calculated for the lattice $\Re_V$ using the electric or magnetic principle of reciprocity. This principle establishes a linear relationship between the electric and magnetic LFs with the electric field that appears when the sensors are energized with a direct or alternating current of low frequency, respectively. Therefore, this electric field summarizes all the conductive properties of the head or the thorax independent of the appearance of an arbitrary CEP. Theorem of Reciprocity: Let $\delta\mathbf{r}^3$ be an element of volume inside the conductor $\Omega_V$. A point source of primary current $j_P(t)\delta\mathbf{r}^3$ in the position $\mathbf{r}_g$ inside the conductor is reflected as a difference of elementary potential $\delta V_{er}^g(t)$ in the electrode lead localized on the scalp; and as an elementary projection of the magnetic field $\delta b_{cn}^g(t)$ on the direction normal to an simple coil external to the head (Figure 5). Under passive conditions (PEC equal to zero in the conductor), the two situations enunciated below will produce different distributions of electric field in the whole conductor. 1) For the EEG a direct current $I_{er}$ (leaving the conductor by the recording electrode and entering by the reference) applied to the same lead electrode (Figure 6a). The electric theorem of reciprocity establishes:

$$\frac{\mathbf{e}(\mathbf{r}_g)\cdot\mathbf{j}_P(t)\delta\mathbf{r}^3}{I_{er}} = -\delta V_{er}^g(t).$$ 2) for the MEG an alternating current $I_c(\omega)$ (at a specific frequency $(0\leq\omega\leq100)Hz$)

circulating in the same coil (Figure 6b). The theorem of reciprocity establishes: $\dfrac{\mathbf{e}(\mathbf{r}_g,\omega)\cdot\mathbf{j}_P(t)\delta\mathbf{r}^3}{i\omega I_c(\omega)\Delta S_c} = -\delta b_{cn}^g(t).$

The calculation of the electric fields $\mathbf{e}(\mathbf{r}_g)$ and $\mathbf{e}(\mathbf{r}_g,\omega)$ in the two previously described situations it should be computed in order to finally to obtain the electric and magnetic LFs. This calculation can be carried out on the base of a model of the realistic physical characteristics of the head or torso of the subject as a conductor. The use of the Finite Element Method (FEM) or Finite Difference (FDM) (Ramírez, S., Eisenberg, J. Lehr and F. Schoen. Effects of cardiac configuration, paddle placement, and paddle size on defibrillation current distribution: A finite element mode. Med. Biol. Eng. Comput., vol. 27, pp. 587-594, 1989; Fahy, J., Kim Y. and Ananthaswamy A.. Optimal electrode configurations for external cardiac pacing and defibrillation: An inhomogeneous study. IEEE Trans. Biomed. Eng., vol. BEM-34, pp. 743-748, 1987), allows modeling a Volume Conductor which is inhomogeneous and anisotropic. For this case, $\Re_V$ consists of tetrahedrons. If the Volume Conductor is considered a IHPC, the Boundary Element Method is used (BEM) (Oostendorp T. and van Oosterom A. The potential distribution generated by surface electrodes in homogeneous volume conductive of arbitrary shape. IEEE Trans. Biomed. Eng., vol. BME-38, pp. 409-417, 1991). For this particular case tetrahedrons are also used to discretize the volume, but with the difference that these tetrahedrons, when intersecting the surfaces separating successive compartments, form triangles, these surfaces being discretized by means of a lattice. All these methods have been developed based on the formulation of a scalar boundary problem for the electric potential. These lead to an ill-posed problem (from the physical point of view) due to the uncertainty in determining the electric potential up to a spatial constant. Once the electric potential is calculated, then some type of numerical gradient is used to calculate the electric potential in the situations enunciated previously. The method proposed by Fletcher et al.. (Fletcher D.J., Amir A., Jewett D.L., and Fein G. Improve method for calculation of potentials in to realistic head shape model. IEEE Trans. Biomed. Eng., vol 42, not. 11, pp. 1094-1104, 1995), denominated Lead Field Analysis (LFA), is also based on the solution of a BEM problem for the electric potential. The main difficulty of this methodology is that the matrices originated by all these methods are singular and therefore the use of a method of multiple deflations is needed to obtain a particular solution (Lynn M.S. and Timlake W. P. The uses of multiple deflations in the numerical solution of singular systems of equations, with application to potential theory. SIAM J. Numer. Anal., vol. 5, not. 2, 1968). In addition, when increasing or diminishing the ratio between the conductivity of the skin and of the bone, the resulting matrices become very ill conditioned. Numerical errors introduced by said ill conditioning have been reduced when using the formulation of the isolated problem Hamalainein and Sarvas (Hamailainen M.S. and Sarvas J. 1989. Realistic conductivity model of the head for interpretation of neuromagnetic dates. IEEE Trans. Biomed. Eng. Vol. 36, pp. 165-171). All these methods can be extended to the calculation of these electric fields under the formulation of a vector boundary problem for the ohmic current density originated in both situations, leading to vector versions of the FEM, FDM, and BEM. Contrary to the scalar versions the matrices that originate from discretization of the equations are not singular, the use of the method of multiple deflations being avoided this way. The "sparse" ill conditioning (when increasing or diminishing the ratios among the conductivities) is reflected in said matrices by the appearance of a block structure. It being possible for the vector version of BEM to calculate the inverses by blocks, something that is not possible in the scalar case since these inverses are not defined. As an example, the case of a vector BEM is developed in detail below. Said procedure originates systems of algebraic equations of a large scale. The use of multi-resolution analysis based on the principle of thresholding allow the conversion of these enormous algebraic systems of equations into a sparse format, allowing in this way a considerable reduction of the computational cost and of the errors introduced by rounding (D. M. Bond and S. A. Vavasis. Fast wavelet transforms for matrices arising from boundary element methods, 1994 and Glowinski R., Bread T.W., Wells R. O. and Zhou X.. Wavelet and finite element solutions for Neumann problem using fictitious domains, 1994).

c) Determination of the possible sites of presence of generators of PEC in the head or the thorax by means of the use of an Anatomical Atlas.

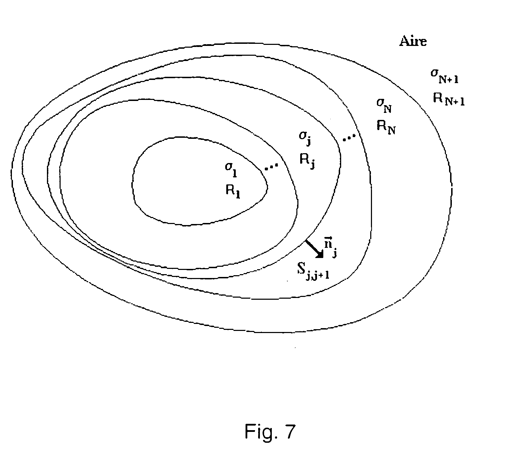

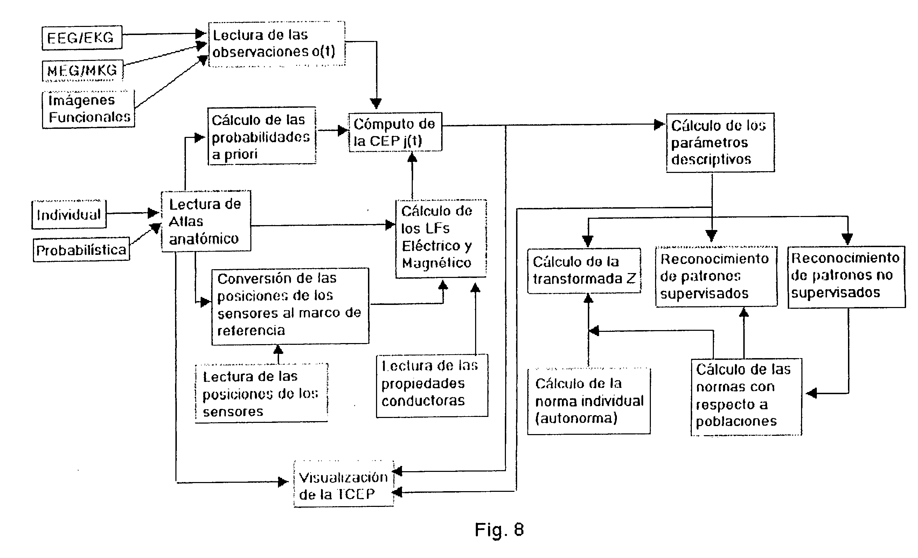

In the particular case of an isotropic piecewise homogeneous volume conductor (IPHC), said IPHC consists of N compartments $(R_1, R_2, \cdots, R_N)$ that do not intersect and are ordered so that said compartments contain each other. The air is,, $R_{N+1}$ by definition. Additionally, $\sigma_j$, represents the value of the conductivity in the compartment $R_j$, ($\sigma_{N+1} = 0$). The surface $S_{j,j+1}$ is the frontier that separates the compartments $R_j$ and $R_{j+1}$. The vector $\mathbf{n}_j(\mathbf{r})$ denotes the normal $\mathbf{r}$ to the surface in the position (Figure 7)

1) The ohmic current density, $\mathbf{j}_j(\mathbf{r})$, in each compartment $R_j$ for the situation (1) corresponds to a vector boundary problem and it can be calculated on the basis of the second Green vector formula:

$$4\pi \mathbf{j}_j(\mathbf{r}) = 4\pi \mathbf{j}_\infty(\mathbf{r}) + \sum_{k=1}^{N} \frac{(\sigma_k - \sigma_{k+1})}{\sigma_k} \oint_{S_{k,k+1}} (\nabla' \times G(\mathbf{r},\mathbf{r}')) \cdot (\mathbf{n}_k(\mathbf{r}) \times \mathbf{j}_k(\mathbf{r}')) d\mathbf{r}'^2$$

$$(11)$$

where:

$$\mathbf{j}_\infty(\mathbf{r}) = -\frac{I_{er}}{4\pi} \nabla \cdot (G(\mathbf{r},\mathbf{r}_e) - G(\mathbf{r},\mathbf{r}_r))$$

$$(12)$$

2) The ohmic current density, $\mathbf{j}_j(\mathbf{r})$, in each compartment $R_j$ for the situation (2) also corresponds to a vector boundary problem and can be calculated directly on the basis of the second Green vector formula:

$$4\pi \mathbf{j}_j(\mathbf{r},\omega) = 4\pi \mathbf{j}_\infty(\mathbf{r},\omega) + \sum_{k=1}^{N} \frac{(\sigma_k - \sigma_{k+1})}{\sigma_k} \oint_{S_{k,k+1}} (\nabla' \times G(\mathbf{r},\mathbf{r}')) \cdot (\mathbf{n}_k(\mathbf{r}) \times \mathbf{j}_k(\mathbf{r}',\omega)) d\mathbf{r}'^2$$

$$(13)$$

where:

$$\mathbf{j}_\infty(\mathbf{r},\omega)=\frac{i\omega}{4\pi}\sum_{k=1}^{N}(\sigma_k-\sigma_{k+1})\oint_{S_{k,k+1}}G(\mathbf{r},\mathbf{r}')\cdot(\mathbf{n}_k(\mathbf{r}')\times\mathbf{b}_c(\mathbf{r}',\omega))d\mathbf{r}'^2$$

(14)

In addition, the vector magnetic field density produced by a coil in an infinite and homogeneous medium is calculated according to the expression:

$$\mathbf{b}_c(\mathbf{r},\omega)=\frac{\mu_o I_c(\omega)\Delta S_c}{4\pi}\nabla\times\nabla\times(G(\mathbf{r},\mathbf{r}_c)\cdot\mathbf{n}_c)$$

(15)

Equations (11) and (13) are solved analytically if the symmetry of the IPHC allows a representation in a system of curvilinear coordinates that allows a separation of variables for the solution of the Laplace vector equation. Otherwise, these they should be transformed to Cartesian coordinates and discretized. Notice that for a fixed frequency $\omega=\omega_c$ in (13) these equations only differ in the independent term $\{\mathbf{j}_\infty(\mathbf{r}),\mathbf{j}_\infty(\mathbf{r},\omega_c)\}$. Let us denote the general vector field as $\mathbf{c}_k(\mathbf{r})=\{\mathbf{j}_k(\mathbf{r}),\mathbf{j}_k(\mathbf{r},\omega_c)\}$. One can then write that for an independent term $\mathbf{c}_\infty(\mathbf{r})$ this general vector field satisfies the equation:

$$\mathbf{c}_j(\mathbf{r})=\mathbf{c}_\infty(\mathbf{r})-\sum_{k=1}^{N}\frac{(\sigma_k-\sigma_{k+1})}{\sigma_k}\mathbf{q}_k(\mathbf{r})$$

(16)

Where: $\mathbf{q}_k(\mathbf{r})=-\dfrac{1}{4\pi}\oint_{S_{k,k+1}}(\nabla'g(\mathbf{r},\mathbf{r}'))\times(\mathbf{n}_k(\mathbf{r}')\times\mathbf{c}_k(\mathbf{r}'))d\mathbf{r}'^2$

A vector BEM based on this equation obtains when the limit $\mathbf{r}\rightarrow S_{j,j+1}$ is calculated and when the resulting Fredholm integral equation of second type is discretized. This leads to a set of algebraic linear equations:

$$\mathbf{Dc}=\mathbf{c}_\infty-\Gamma\mathbf{c}$$

(17)

Where $\mathbf{c}_\infty=(\mathbf{c}_{1\infty};...;\mathbf{c}_{N\infty})$, $\mathbf{c}=(\mathbf{c}_1;...;\mathbf{c}_N)$, and the matrices:

$$\Gamma=\frac{1}{4\pi}\begin{pmatrix}\frac{(\sigma_1-\sigma_2)}{\sigma_1}\Gamma_{11} & \frac{(\sigma_2-\sigma_3)}{\sigma_2}\Gamma_{12} & \cdots & \Gamma_{1N}\\ \frac{(\sigma_1-\sigma_2)}{\sigma_1}\Gamma_{21} & \frac{(\sigma_2-\sigma_3)}{\sigma_2}\Gamma_{22} & \cdots & \Gamma_{2N}\\ \vdots & \vdots & \ddots & \vdots\\ \frac{(\sigma_1-\sigma_2)}{\sigma_1}\Gamma_{N1} & \frac{(\sigma_2-\sigma_3)}{\sigma_2}\Gamma_{N2} & \cdots & \Gamma_{NN}\end{pmatrix}\qquad D=\begin{pmatrix}\alpha_1 I_{N_{1,2}} & 0 & \cdots & 0\\ 0 & \ddots & 0 & \vdots\\ \vdots & 0 & \alpha_{N-1}I_{N_{N-1,N}} & 0\\ 0 & \cdots & 0 & I_{N_{N,N+1}}\end{pmatrix}$$

The $(3N_{j,j+1}\times1)$ vectors $\mathbf{c}_{j\infty}$ and $\mathbf{c}_j$ are formed by the evaluation of $\mathbf{c}_{j\infty}(\mathbf{r})$ and $\mathbf{c}_j(\mathbf{r})$ at the centers of mass of the triangles of the surface $S_{j,j+1}$. $\mathbf{c}_k$ is a $(3N_{k,k+1}\times1)$ vector built by the evaluation of in the centers of mass of the triangles of the surface $S_{k,k+1}$. The $(3N_{j,j+1}\times3N_{k,k+1})$ matrix $\Gamma_{jk}$ contains the numerical evaluation of $\Gamma_n^k(\mathbf{r})$ at the centers of masses of the triangles of the surface $S_{j,j+1}$.

$$\Gamma_{jk} = \begin{pmatrix} \Gamma_1^k\left(\mathbf{r}_1^j\right) & \cdots & \Gamma_{N_{k,k+1}}^k\left(\mathbf{r}_1^j\right) \\ \vdots & \ddots & \vdots \\ \Gamma_1^k\left(\mathbf{r}_{N_{J,j+1}}^j\right) & \cdots & \Gamma_{N_{k,k+1}}^k\left(\mathbf{r}_{N_{J,j+1}}^j\right) \end{pmatrix}$$

Where $\Gamma_n^k(\mathbf{r}) = \Omega_n^k(\mathbf{r})\mathbb{I}_3 - \left(\mathbf{n}_n^k \circ \lambda_n^k(\mathbf{r})\right)$:

The solution of the system of algebraic equations (17) is obtained inverting the matrix of the system: $\mathbf{c}_{est} = (\mathbf{D} + \Gamma)^{-1}\mathbf{c}_\infty$. This system of algebraic equations does not have problems of singularity since the ohmic current density does not depend on specifying any reference.

The value of $\mathbf{c}_j(\mathbf{r})$ at an interior point r of the compartment $R_J$ is obtained by substituting, the general vector field estimated on the surfaces, into the following expression:

$$\mathbf{c}_j(\mathbf{r}) = \mathbf{c}_\infty(\mathbf{r}) - \frac{1}{4\pi}\sum_{k=1}^{N}\frac{\left(\sigma_k - \sigma_{k+1}\right)}{\sigma_k}\sum_{n=1}^{N_{k,k+1}}\Gamma_n^k(\mathbf{r})\mathbf{c}_{n_{est}}^k$$

*Independent terms EEG/MEG*

For the EEG

Keeping in mind the fact that the current injected is considered constant over the triangles marked on the scalp, corresponding to the electrodes the following is obtained:

$$\mathbf{c}_\infty(\mathbf{r}) = \frac{I_{er}}{4\pi}\left(\frac{(\mathbf{r} - \mathbf{r}_e)}{|\mathbf{r} - \mathbf{r}_e|^3} - \frac{(\mathbf{r} - \mathbf{r}_r)}{|\mathbf{r} - \mathbf{r}_r|^3}\right)$$

For the MEG

If at the fixed frequency $\omega = \omega_c$ it is considered that the magnetic field vector density is constant on each triangle and equal to its value at the center of mass, we obtain:

$$\mathbf{c}_\infty(\bar{r}) = \mathbf{j}_\infty(\mathbf{r}, \omega_c) = -\frac{i\omega_c}{4\pi}\sum_{k=1}^{N}(\sigma_k - \sigma_{k+1})\sum_{n=1}^{N_{k,k+1}}\mathbf{n}_n^k\times\mathbf{b}_{cn}^k\eta_n^k(\mathbf{r})$$

where:

$$\eta_n^k(\mathbf{r}) = \int_{\Delta_n^k}\frac{d\mathbf{r'}^2}{|\mathbf{r} - \mathbf{r'}|}$$

Note that it is necessary to specify the transversal component of the magnetic field vector density in each one of the triangles that form each surface. For this, it is only necessary to evaluate the expression mentioned below in each one of the centers of masses of the triangles.

$$\mathbf{b}_{cn}^k = \mathbf{b}_c\left(\mathbf{r}_n^k, \omega_c\right) = \frac{\mu_o I_c(\omega_c)\Delta S_c}{4\pi}\left(\frac{3\mathbf{n}_c \cdot \left(\mathbf{r}_n^k - \mathbf{r}_c\right) \circ \left(\mathbf{r}_n^k - \mathbf{r}_c\right)}{\left|\mathbf{r}_n^k - \mathbf{r}_c\right|^5} - \frac{\mathbf{n}_c}{\left|\mathbf{r}_n^k - \mathbf{r}_c\right|^3}\right)$$

Calculation of the inverse solution

The solution to the inverse problem is based on formulating a Hierarchical Bayesian model for image fusion (Hum, 1996; Mardia, 1996). At the most basic level in the model the existence of a non-observable brain or heart tissue activation, A($t$), is postulated.

The model comprises the following components:

• Likelihood terms $l_{o_m}$ for each modality m based on the corresponding direct problem

$$\mathbf{o}_m(t) = \mathbf{K}_m \cdot \mathbf{f}_m(t) + \mathbf{e}_m(t) \tag{18}$$

Expression (18) relates the observations $\mathbf{o}_m$($t$) with the functional indicators, $\mathbf{F}_m(\mathbf{x}, t)$, these being degraded by convolution with, $\mathbf{K}_m$, a Kernel that captures the spatial and temporal resolution of the image modality m. An additive instrumental error, $\mathbf{e}_m(t) \sim l_{o_m}(\mathbf{o}_m(t) - \mathbf{K}_m *\mathbf{f}_m(t)|\Theta_{e_m})$, is also assumed. The notation $\mathbf{f} = [\mathbf{f}_m]_{1 \leq m \leq M}$ shall be used. Note that for the EEG and MEG, $\mathbf{f}_m(t) = \mathbf{j}(t)$.

• a priori Probabilities, $\pi_{\mathbf{f}_m}$, for each functional activity $\cdot \mathbf{F}_m(\mathbf{x},t)$, conditioned to a degree of cerebral activation A(x, t) according to the relationship

$$\mathbf{f}_m(t) = \mathbf{H}_m \circ \mathbf{A}(t) + \beta_m(t) \tag{19}$$

that expresses the form in which $\mathbf{A}(\mathbf{x},t)$ is projected according to some mechanism $\mathbf{H}_m$ to produce the functional indicators. Random variations of the functional indicators, $\beta_m(t) \sim \pi_{\mathbf{f}_m}(\mathbf{f}_m(t) - \mathbf{H}_m \circ \mathbf{A}(t)|\Theta_{\beta_m})$, are also assumed. The notation $\Theta_\beta = {}_L\Theta_{\beta_m}{}^{\rfloor}{}_{1 \leq m \leq M}$ will be used.

• A priori probability $\pi_A$ of the brain or heart activation $\mathbf{A}(t) \sim \pi_\mathbf{A}(\mathbf{A}(t)|\Theta_\mathbf{A})$.

• A priori probabilities for the hyperparameters

$$\Theta_\mathbf{A} \sim \pi_{\Theta_\mathbf{A}}(\Theta_\mathbf{A})$$

$$\Theta_{\beta_m} \sim \pi_{\Theta_{\beta_m}}(\Theta_{\beta_m}).$$

$$\Theta_{e_m} \sim \pi_{\Theta_{e_m}}(\Theta_{e_m})$$

All of which leads to the following basic a posteriori probability:

$$P(\mathbf{A},\Theta_A,\mathbf{f},\Theta_\beta,\Theta_e|\mathbf{o}) \propto$$
$$\prod_{m=1}^{M} l_{\mathbf{o}_m}\left(\mathbf{o}_m(t) - \mathbf{K}_m * \mathbf{f}_m(\mathbf{x},t)|\Theta_{e_m}\right) \times$$
$$\prod_{m=1}^{M} \pi_{\mathbf{t}_m}\left(\mathbf{f}_m(t) - \mathbf{H}_m \circ \mathbf{A}(t)|\Theta_{\beta_m}\right) \times$$
$$\prod_{m=1}^{M}\left\{\pi_{\Theta_{e_m}}(\Theta_{e_m}) \cdot \pi_{\Theta_{\beta_m}}(\Theta_{\beta_m})\right\} \times$$
$$\pi_\mathbf{A}\left(\mathbf{A}(t)|\Theta_\mathbf{A}\right)$$

(20)

The estimation of all the parameters $\mathbf{A},\Theta_A,\mathbf{F},\Theta_\beta,\Theta_E$ of the model will be carried out by the Maximum method a Posteriori (MAP) method. For some embodiments of the invention, explicit estimators will exist. Otherwise the estimate will be obtained by one of the following iterative methods, taking as initial values those estimated of a simpler model or simply random values:

Successive Maximization of (20) for subsets of the parameters $\mathbf{A},\Theta_A,\mathbf{F},\Theta_\beta,\Theta_E$, while maintaining fixed all the others, "Iterated Conditional Maximization" (ICM; Winkler, G. (1995) Image Analysis, Random Fields and Dynamic Carlo Methods Mounts. Springer).

A. Successive Maximization of (20) for subsets of the parameters., fixing the other parameters to their expected value, "Expectation Maximization" (EM; Tanner, 1996).
B. Choosing the mode of the Monte Carlo's distribution of (20) obtained by means of Monte Carlo Markov Chain methods (MCMC, Tanner M. 1996 Tools for Statistical Inference. Third Edition. Springer).

The advantage of the formulation (20) is that it allows the combination, in a statistically optimal manner, of information provided by imaging modalities with different temporal and spatial resolution. The weight that should be assigned to each modality is obtained in an automatic. In particular, it is possible to find the optimal relative weights with which the EEG and MEG should be combined.
The form of the likelihood is specified by means of the form of the Risk

$$R_{|\Theta_{E_m}}\left[\mathbf{e}_m|\Theta_{e_m}\right] = -\ln\left[l_{\mathbf{o}_m}\left(\mathbf{e}_m(t)|\Theta_{e_m}\right)\right] + \ln[C_\mathbf{o}]$$

(21)

The following notation is introduced for any set of variable x. Let the most important parameters of the distribution be the mean and the matrix of covariances $\Theta_x = \{\mu_x, \Sigma_x\}$. Then $R_\mathbf{x}^p\left[\mathbf{X}|\Theta_\mathbf{x}\right]_{\varepsilon,p} = \sum_{i=1}^{N_\mathbf{x}} C_{\varepsilon,p}\left[\mathbf{z}_i^\mathbf{x}\right]$ defines a polynomial risk of order p, ($\varepsilon$ insensitive) with

$$C[y]_{\varepsilon,p} = \begin{cases} 0 & |y| \le \varepsilon \\ \left|\dfrac{z^p}{p}\right| - \varepsilon & |y| > \varepsilon \end{cases}.$$

(22)

This class of risks includes the norms ($L_p$) of order p>1 used previously:

$$R_{\mathbf{x}}^{L^p}\left[\mathbf{x}|\Theta_{\mathbf{x}}\right]=\left\|\mathbf{x}-\mu_{\mathbf{x}}\right\|_{\Sigma_{\mathbf{x}}}^p=\sum_{i=1}^{N_{\mathbf{x}}}C_{0,p}\left[z_i^{\mathbf{x}}\right].$$

$$(23)$$

In addition, risks that are more general $R_{\mathbf{x}}^C\left[\mathbf{x}|\Theta_{\mathbf{x}}\right]=\sum_{i=1}^{N_{\mathbf{x}}}C_{\varepsilon,p,\sigma}\left[\mathbf{Z}_{i\bullet}^{\mathbf{x}}\right]$ will be used where

$$C[w]_{\varepsilon,p,\sigma}=\begin{cases}0 & |w|\leq\varepsilon\\c_{p,\sigma}\left(|w|-\varepsilon\right) & |w|>\varepsilon\end{cases}\quad\text{and}\quad c_{p,\sigma}(z)=\begin{cases}\sigma^{1-p}\cdot\dfrac{z^p}{p} & z\leq\sigma\\\left(z+\left(\dfrac{1}{p}-1\right)\sigma\right) & z>\sigma\end{cases}$$

$$(24)$$

This type of risk includes many particular cases used in other contexts. When $\varepsilon=0$ and $p=2$ the risk correspond to that of Huber that defines robust estimators. The case $\varepsilon=0$ y $\sigma=0$ reduces to the norm $L_1$ mentioned previously. Finally if $\sigma=0$ the norm is the $\varepsilon$ insensitive risk of Vapnik (1995).

In order to obtain the MAP estimator of (20) using ICM, EM, or MCMC an essential step is the minimization of expressions of the type $R_0+\lambda\cdot R_J(\mathbf{j}(t)|\Theta)$ (equation 5). We define $R_o$ as $R_{\mathbf{x}}^P\left[\mathbf{x}|\Theta_{\mathbf{x}}\right]_{\varepsilon,p}$ or $R_{\mathbf{x}}^C\left[\mathbf{x}|\Theta_{\mathbf{x}}\right]$ and $R_J(\mathbf{j}(t)|\Theta)$ as $\left\|\mathbf{x}-\mu_{\mathbf{x}}\right\|_{\Sigma_{\mathbf{x}}}^p$. The partial solution (Smola, 1966) is equivalent to regression with Support Vector Machines" (SVM). Besides allowing an efficient minimization, stated as a quadratic programming problem, also the solutions obtained are robust to data contaminated with outliers. Additionally the estimated PEC will be function of a reduced number of elements of $K_m$, which guarantees simplicity of the TPEC.

For both the EEG and MEG $\mathbf{f}_m(t)=\mathbf{j}(t)$, the PEC. The model for the PEC is further detailed as

$$\mathbf{j}\left(\mathbf{r}_g,t\right)=\mu\left(\mathbf{r}_g\right)\cdot\gamma\left(\mathbf{r}_g,t\right)+\xi\left(\mathbf{r}_g,t\right)$$

$$(25)$$

where $\mu(\mathbf{r}_g)$: $\Omega g\to R^3$ is the field of orientations of the generators of the PEC, $\gamma(\mathbf{x},t)$: $\Omega_g\times\mathfrak{I}\to R$ the field of intensity of the generators of the PEC and $\xi(t)$: $\Omega_g\times\mathfrak{I}\to R^3$ the additive random component $\beta_m(\mathbf{x},t)$ associated with $\mathbf{j}(t)$. Said random component, which without lost of generality in a particular embodiment, will be specified as $\xi(\mathbf{x},t)\sim\mathbf{N}_R^{3,2}\left(0,\sigma_\xi^2\cdot\mathbf{I}_3\right)$. Equation (25) extends to the TPEC the spatiotemporal modeling initially introduced for cerebral dipoles (equation 7; Scherg, 1993):

$$\mathbf{j}(t)=\mathbf{M}\cdot\mathbf{G}(t)+\xi(t)$$

The specification of the orientation and intensity of the generators is carried out defining the following a priori probabilities:

$$\mu=vech(\mathbf{M})\sim\pi_\mu\left(\mathbf{A},B_{n,s}^m\left(\Omega_g\to R^3\right)\Theta_\mu\right)$$

$$(26)$$

$$\gamma(t) \sim \pi_{\gamma}\left(\mathbf{A}, B_{n,s}^{m}(\Omega \to R), \Theta_{\gamma}\right)$$

$$(27)$$

such that compatibility is enforced with the cerebral activation A, and a functional smoothness predefined by a Besov space . Selecting in this case a Besov Space with index (1,1,s) leads to a Variable Resolution TPEC (VARETA). VARETA allows the estimation of both point source generators as well as distributed source generators in the same framework. Thus, the absolute dichotomy between both types of modeling, existent up to now is eliminated. In VARETA, it is allowed that the quantity of smoothness enforced in the solution be variable from one point to another inside the generating volume. Thereby the name "Variable Resolution." This variable resolution allows spatially adaptive nonlinear estimates of the current sources that eliminate those "ghost" solutions present in the usual linear distributed solutions. It also allows the achievement of "super-resolution," the capacity to distinguish discrete sources very near one to the other. Particular, but not exclusive realizations, of the a priori probabilities of the equation (26) are:

1. $\log \pi_{\mu} \propto \left\| \Lambda_s \cdot \mu \cdot \Lambda_m \right\|_{B_{n,s}^m}$ where $\Lambda_s$. specifies the degree of smoothness that will be imposed at each point of the lattice and $\Lambda_m = \mathbf{W} \cdot p_g$, where W has already been defined and $p_g$ is a matrix in whose diagonal the probabilities are placed that gray matter exists in the point of the lattice.

2. $\pi_{\mu}(\mu|\mathbf{i}) = N_R^{3 \cdot Ng, p}\left(0, \Lambda_m \cdot \mathbf{D} \cdot \mathbf{Z} \cdot \mathbf{D} \cdot \Lambda_m\right)$, where the values in the diagonal of D take large values $\sigma_A^2$ or small values $\sigma_A^2$ according to the value of an indicator variable i defined for the lattice such that said indicator takes the value 1 if activity is admitted in a point and 0 otherwise. In other words a mixture model is taken for the marginal distribution such that $\pi_{\mu}(\mu_t|\mathbf{i}) = i \cdot N(0, \sigma_A^2) + (1 - i) \cdot N(0, \sigma_B^2)$, and, in turn, the i are obtained as samples of a multinomial with probabilities $p_g$.

Similar expressions for the equation (27) are evident. Note that the dependence on $p_g$ allows the probabilistic restriction of estimators for PEC to points of the lattice due to a priori our knowledge . In particular if $p_g$ only take values 0 and 1 this restriction for the localization of the PEC becomes deterministic.

Statistical evaluation

Determination of the membership to a Test Group

Once made, for a given subject, the estimate of $\mathbf{j}(\mathbf{x}, t)$ and $\gamma(\mathbf{x},t)$ and of the hyperparameters, a selection of these are composed in a vector of DP. This vector is used to determine if the TPEC obtained may be classified, using methods of non-parametric statistics (Ripley B. D. (1966) Pattern Recognition and Neural Networks. Cambridge University Press), in one of a multiplicity of test groups. Test groups that represent different embodiments of this invention are:

- Groups of subjects defined as normal by means of criteria external to their TPEC. These groups will be specified implicitly by means of a sample of DP of TPEC of normal subjects contained in a database. The objective of the statistical analysis in this case, is to determine the probability that the DP of the TPEC evaluated is obtained from a healthy subject.
- Groups of subjects defined as having pathologies predefined by criteria external to the TPEC will be specified implicitly by means of samples of DPs of TPEC of patients with the contained pathologies of interest in a database. The objective of the statistical analysis in this case is to determine the probability that the TPEC is obtained from a subject with the specified illnesses.
- Groups determined by methods of Unsupervised Pattern Recognition the basis of the DP of the TPEC of a sample of normal and/or pathological subjects have in common physiological characteristics of interest. The objective of the statistical analysis in this case is to determine the probability that the TPEC is obtained a subject with the physiological characteristics.
- Groups consisting of a sample of DP of TPEC obtained in previous moments from the subject under study. The group of DPs for each analysis moment constitutes a vector time series . The objective of the statistical analysis of this vector time series is twofold

1. To determine the changes with time in the functioning of predetermined parts of the brain or heart, as reflected in changes of the DP, with the objective, for example, to monitor the states of the brain during an operation.
2. To carry out predictions about the probability of that a vector of DP that reflect the functional state of the brain or heart take on a given set of values at a moment posterior to the current examination.

For the calculation of the previous probabilities, it is required to have the mean $\mu_x(v)$, and variance $\sigma_x(v)$, of each test Group for each one of the parameters at each point of the lattice of the generating volume. These means and variances can be expressed as a function of control variables, **v**, by means of non-parametric regression (Hastie, T.J. and Tibshirani, R.J. (1990): Generalized additive models. Chapman and Hall, Pp 18-20; ISBN 0-412-34390-8 and Fan J. and Gibjels. I. Local Polynomimal Modeling and its Applications, 1996):

$$T(x) = \mu_x(v) + \varepsilon_x(v),$$

$$(28)$$

The following transformations are carried out, also, on the DP :

1. Transformation toward a target distribution by means of a non-linear function x = T(DP), to ensure that the vector x has a distribution close to one selected, f, specified beforehand.
2. Calculation of the $z_x$ transformation using the univariate variant.
3. Calculation of the probability that all or part of $z_x$ reaches a prespecified maximum or minimum value . This calculation should be carried out with the correction of the effect of the multiple spatial and temporal correlations. These are as much a consequence of the estimation process as of the physiology of the brain and heart, the over all effect being of increase the type error I in the identification of pathological states.

The complexity of this problem rests in the nature of $z_x$ as a random function defined on a four dimensional manifold, three dimensions for space and one for time (defined so without loss of generality, though the time dimension may refer to an expansion in a basis of a Megadictionary; thus also possibly defining a frequency or time frequency dimension). Statistical methods have been developed for this type of random variable defined as a stochastic processes on a n-dimensional manifold (Worsley, K.J., Marrett, S., Neelin, P., Vandal, A.C., Friston, K.J., and Evans, A.C. (1995): A unified statistical approximation for determining significant signals in images of cerebral activation. Human Brain Mapping, 4: 58-73). These statistical procedures have been applied to the study of structural images (CAT, MRI), as well as functional images (fMRI, SPECT, and PET). Said methods have not been applied yet to the field qEEG/qEKG or to the TPEC. In particular these results calculate the probability P that $z_{max}$, the maximum value of z observed in a region of interest C (of arbitrary shape), exceeds a given threshold U.

$$P_{f,max}(z_{max} > U) \sum_{d=0}^{D} \rho_{max}(f)_d \cdot R_d(C)$$

$$P_{f,min}(z_{max} < U) \sum_{d=0}^{D} \rho_{min}(f)_d \cdot R_d(C)$$

where $\rho_{max}(f)_d$ y $\rho_{min}(f)_d$ depend on the type f of random field that is postulated (Gaussian, t, F, $\chi^2$, etc.) and the $R_d$ characterizes the geometry of C being expressed in terms of the FWHM of the stochastic field.

$FWHM = \sqrt{4 \log_e 2} (\det(\Lambda))^{-1/2D}$, where $\Lambda = Var(\frac{\partial Z}{\partial x})$ is the variance of the derivative of the stochastic field, and D is the dimension of C. Notice that, in the general case, the probability previously expressed only will depend on the type of random field that is being evaluated but also on the degrees of freedom of the statistical image. $z_x$ will be plotted in the following ways

- As one-dimensional graph, where $z_x$ will be plotted as a function for a fixed point of the lattice. This graph type is denominated a z curve (or, in the case of the frequency domain, z spectrum of the TPEC).
- As a two or three-dimensional image, where $z_x$ will be plotted for a fixed time instant. This graph type is denominated z image of the TPEC. The plot will be carried out according to a color code that indicates the degree of deviation of the TPEC from normative values. This plot is superimposed on an anatomical atlas, which constitutes the background of the image.

For the clearer statistical evaluation of the z image the following transformation is defined:

$$\begin{cases} z_w = F^{-1}\big(P_{f,\max}(z)\big) \ \text{si} \ \ z \geq U_{\max} \\ z_w = 0 \hspace{2.5cm} \text{si} \ \ U_{\min} < z < U_{\max} \\ z_w = F^{-1}\big(P_{f,\min}(z)\big) \ \text{si} \ \ z \leq U_{\min} \end{cases}$$

where the region of interest selected is the complete image, $F^{-1}$ is the inverse Gaussian distribution function. $U_{\max}$, $U_{\min}$ are limits chosen by the user, a possible selection being $U_{\min} = - U_{\max}$. The resultant is a scale of probability corresponding to a univariate gaussian distribution. The advantage of this is that the user can make an evaluation without having to keep in mind the distribution type f or the corresponding degrees of freedom.

*Examples of preferred embodiments*

TPECc in the frequency domain

One preferred, but non-exclusive, embodiment of this invention will be the TPECc in the frequency domain for the EEG. This embodiment is developed for situations in situations in which it is not convenient to obtain an individual MRI and therefore the use of Probabilistic Brain Atlas becomes necessary. The observations are taken now as the EEG for the individual i, segment j, which is modeled (Valdés, P.; Bosch, J.; Grave de Peralta, R.; Hernández, J.L.; Pascual, R. and Biscay, R.: Frequency domain models for the EEG. Brain Topography, 1992a, vol. 4, pp. 309-319) as:

$$\mathbf{o}_{i,j}(t) = \kappa_i \cdot \mathbf{v}_{i,j}(t) - \beta_{i,j}(t) \cdot \mathbf{1}_{N_d},$$

$$(29)$$

In other words it is assumed that the observed EEG is the distortion of an ideal recorded EEG $\mathbf{v}_{i,j}(t)$ by multiplication with a scale factor $\kappa_i$ (individual and random) and also by subtraction of the activity of the reference electrode $\rho_{ij}(t)$. By means of visual inspection, it is guaranteed that the segments conform to the assumptions of stationarity and "mixing" (Brillinger, D.R, (1975): Time series Date Analysis and Theory.). This guarantees that the Fourier transformed of the segments $\mathbf{o}_{i,j}(\omega) \sim N_C^{N_d}\big(\mathbf{0}, \Sigma_{\mathbf{o}}(\omega)\big)$ where $\omega$ indicates frequency and the crosspectral or covariance matrix of the EEG which is estimated as:

$$\mathbf{S}_{\mathbf{O},i}(\omega) = \frac{1}{N_{seg}} \sum_{j=1}^{N_{seg}} \mathbf{o}_{i,j}(\omega) \cdot \mathbf{o}_{i,j}(\omega)^{*},$$

$$(30)$$

Valdés-Sosa (Valdés-Sosa, P. (1984): Statistical Bases. Chapter 7. Neurometric assessment of brain dysfunction in neurological patients. In: T. Harmony. Functional Neuroscience, vol. 3, Lawrence Erlbaum Associates, Hilsdale, New Jersey, pp. 141-254). Consequently the sample estimator of the matrix of covariances $\Sigma_1(\omega)$ is:

$$\mathbf{S}_{\mathbf{V},i}(\omega) = \frac{1}{\kappa_i^2} \mathbf{H} \cdot \mathbf{S}_{\mathbf{O},i}(\omega) \cdot \mathbf{H},$$

$$(31)$$

where H denotes the centering matrix that transforms the data to the average reference (Mardia KV, Kent JT and Bibby JM. (1979): Multivariate Analysis. Academic Press Inc. London Ltd.)and the Maximum Likelihood estimator of $\kappa$ is described in Hernández et al. (Hernández, J.L.; Valdés, P.; Biscay, R.; Virués, T.; Száva, S.; Bosch, J.; Riquenes, A. and Clark, I. (1995): A Global Scale Factor in Brain Topography. Intern. Journal of Neuroscience, 76:267-278).

In this case the object of primary interest is the estimation of $\Sigma_{J_i}(\omega)$ which is carried out by choosing the appropriate a priori probabilities. In this case, the MAP estimation procedure is equivalent to minimize the following expression:

$$\sum_{r=1}^{N_{seg}} \left( (\mathbf{v}_{ir}(\omega) - \mathbf{K} \cdot \mathbf{j}_{ir}(\omega))^t \cdot \Sigma_{E_i}^{-1}(\omega) \cdot (\mathbf{v}_{ir}(\omega) - \mathbf{K} \cdot \mathbf{j}_{ir}(\omega)) + \mathbf{j}_{ir}^{\ t} \cdot \Sigma_{J_i}^{-1} \cdot \mathbf{j}_{ir} \right)$$
$$+ (m + N_{seg}) \cdot \ln\left|\Sigma_{J_i}(\omega)\right| + \frac{1}{\tau_i^2(\omega)} Tr\left\{\Sigma_{J_i}^{-1}(\omega) \cdot \mathbf{G}\right\}$$

$$(32)$$

which is achieved by calculating iteratively for the step k

$$1. - \mathbf{A}_{J_i}(\omega)^{(k)} = \hat{\Sigma}_{J_i}(\omega)^{(k-1)} \cdot \mathbf{K}^t \cdot \left(\mathbf{K} \cdot \hat{\Sigma}_{J_i}^{(k-1)} \cdot \mathbf{K}^t + \Sigma_{E_i}(\omega)\right)^{-1} \cdot \mathbf{S}_i^{\frac{1}{2}}(\omega)$$
$$2. - \hat{\Sigma}_{J_i}(\omega)^{(k)} = \frac{N_{seg} \mathbf{A}_{J_i}(\omega)^{(k)} \cdot \mathbf{A}_{J_i}(\omega)^{(k)} + \frac{1}{\tau_i^2(\omega)^2} \mathbf{G}}{m + N_{seg}}$$

$$(33)$$

These steps are repeated until the estimator converges.

In equation (32) the first term corresponds to the likelihood, the second term $\sum_{r=1}^{N_{seg}} \mathbf{j}_{ir}(\omega)^t \cdot \Sigma_{J_i}^{-1}(\omega) \cdot \mathbf{j}_{ir}(\omega),$ to the usual regularizing term with the hyperparameter matrix. The third term corresponds to the natural conjugate a priori for the Wishart Matrix for the hyperparameter $\Sigma_{J_i}(\omega)$, proportional to:

$$\mathbf{G} = \left(\Lambda_m \cdot \mathbf{L}^t \cdot \Lambda_s^2 \cdot \mathbf{L} \cdot \Lambda_m\right)^{-1} \otimes \mathbf{I}_3$$

where the diagonal matrix $\Lambda_s$ specifies the degree of smoothness that will be enforced in each point of the lattice, in this case zero for the borders of the gray matter. $\Lambda_m$ weights the permissible magnitude of the PEC at each point of the lattice $\Lambda_m = \mathbf{W} \cdot \Lambda_G^{\frac{1}{2}}$, where the diagonal matrix $\Lambda_G$ contains an estimator of the probability that any normal brain has gray matter at that position. These probabilities for example can be obtained using a Braini Probabilistic Atlas (Evans, A. C., Collins, D.L., Neelin, P., MacDonald, D., Kambei, M., and Marret, T.S. (1994) Three dimensional correlative imaging: Applications in human brain mapping. In R. Thatcher, M. Hallet, T. Zeffiro, E. Roy John and M. Vegetable garden (Eds.) Functional neuroimaging technological foundations. Academic Press; Mazziotta JC, Gown TO, Evans AC, Fox P, Lancaster J (1995) A probabilistic atlas of the human brain: Theory and rationale for its development. Neuroimage 2: 89-101).

In a particular embodiment of this invention, the diagonals of all $\Sigma_{j_i}(\omega)$ are taken as DP, denoted as $\mathbf{S}_j(\omega)$. Equation (28) is applied to each one of these descriptive parameters using the natural logarithm as T. The mean and variance by frequency and for each point of the lattice are obtained previously by the regression model:

$$1n\left(\mathbf{S}_{j,i}(\omega)\right) = \mu_i(\omega; age) + \varepsilon_i(\omega; age),$$

$$(34)$$

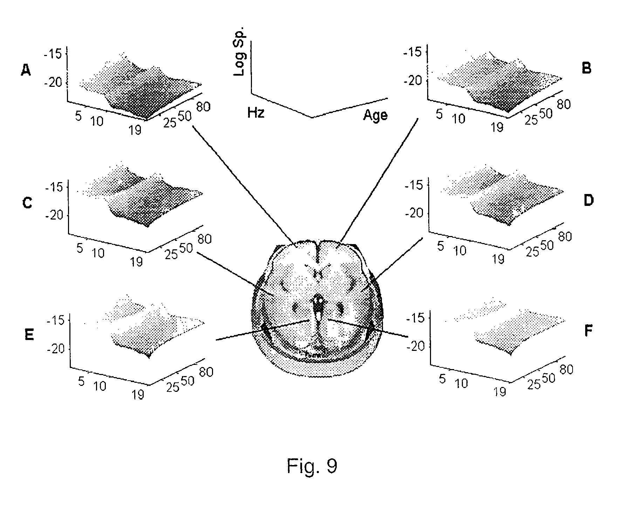

where the population the mean $\mu_i(\omega; age)$ and standard deviation $\sigma_i(\omega; age)$, for each point of the lattice j and each age, are obtained by heteroscedastic non-parametric regression (Hastie, T.J. and Tibshirani, R.J. (1990): Generalized additive models. Chapman and Hall, Pp 18-20; ISBN 0-412-34390-8; Fan J. and Gibjels. I. Local Polynomimal Modeling and its Applications. Chapman and Hall). The complete set of $\mu_i(\omega; age)$ constitutes then a generalization of the developmental surfaces described in Valdés et al. (Valdés, P.; Biscay, R.; Galán, L.; Bosch, J.; Szava, S.; and Virues, T.: High Resolution Spectral EEG norms for topography. Brain Topography, 1990, vol. 3, pp. 281-282) and now extended to the TPEC. In Figure 9 these regression surfaces (obtained from the Cuban Normative EEG Data Base) can be observed. In the figure, three-dimensional surfaces are shown for several points of the generator lattice, in which the z-axis is $\mu_i(\omega; age)$ the y-axis is frequency and the x-axis is age.

The two or three dimensional plots of the z values obtained this way from $\mathbf{S}_J(\omega)$ constitute an example of z images of the TPEC. The plotting is carried out with a color code that indicates the deviation of the calculated image from normative values. This plot is superimposed on a cerebral atlas in Talairach coordinates, which constitutes the background of the image. For the statistical evaluation of z images of it is assumed that they are samples of a four dimensional gaussian random field (3 space and 1 frequency dimensions). In this case the theory described by Worsley et al. is applied (Worsley, K.J., Marrett, S., Neelin, P., Vandal, A.C., Friston, K.J., and Evans, A.C. (1995): A unified statistical approximation for determining significant signals in images of cerebral activation. Human Brain Mapping, 4:58-73). This allows the evaluation of the probability that a maximum or minimum of the image z takes a given value. The $z_w$ transformation described above is adopted for the yielding a scale of probability corresponding to a univariate gaussian distribution.

*Clinical example 1.*

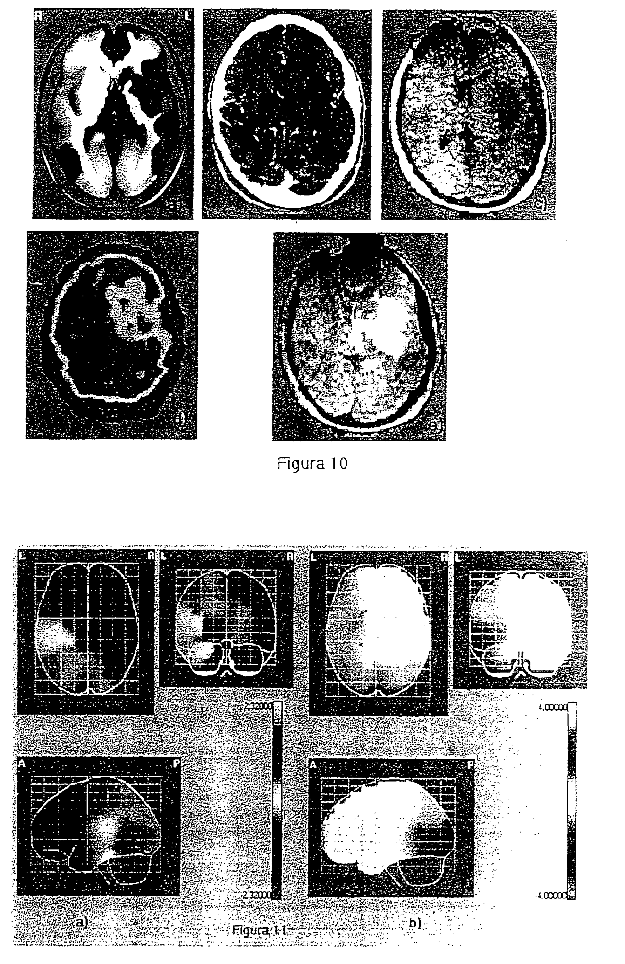

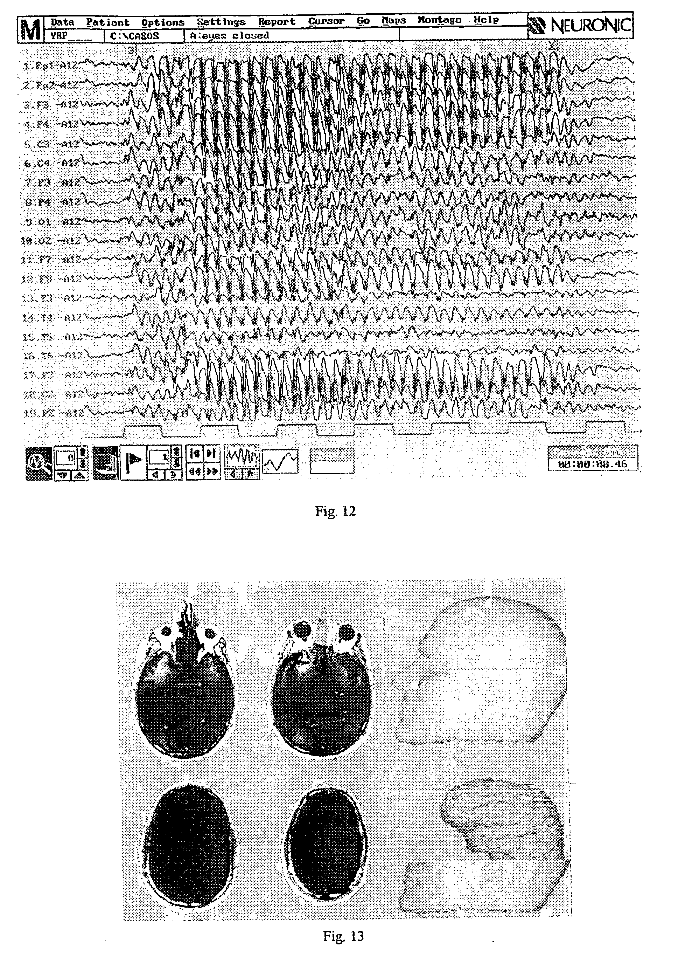

In Figure 10 a) the Z image of the TPECc in the frequency domain is shown, said image being obtained from a 27-year-old female patient for whom an astrocytoma in the left fronto-parietal region had been diagnosed. For purposes of comparison also shown in this figure are the b) spiral CAT, c) T1 MRI, d) SPECT, and and e) T2 MRI.

*Clinical example 2*

A group of 11 neurological patients of recent admission were selected based on a diagnosis of Cerebrovascular accidents (CVA) of ischemic origin, due to occlusion of the Left (LCMA) or Right (RCMA) Medial Cerebral Artery. TCEPc the frequency domain and the CAT were applied to all patients. Examples of the obtained TPECc-FD are shown in Figure 11, there being a complete correspondence with the clinical diagnosis. In Table1 all the results obtained are summarized. One can observe that in 7 cases the TPECc-DF was superior to the CAT, in 3 equal and only in 1 worse in terms of localization accuracy. The CAT is to date the imaging method of election for evaluation of CVA in spite of its invasive nature.

**Table 1**

| PATIENT | AGE IN YEARS | MOMENT OF THE STUDY IN HOURS | I DIAGNOSE | TPECc-DF | CAT |
|---|---|---|---|---|---|
| 01-Fg. 11a | 48 | 12 | LCMA | YES | NO |
| 021 | 75 | 24 | LCMA | YES | NO |
| 03 | 44 | 48 | LCMA | YES | NO |
| 04 | 71 | 24 | LCMA | YES | NO |
| 05 | 49 | 48 | LCMA | YES | NO |
| 06 | 65 | 22 | RCMA | YES | NO |
| 07-Fig. 11b | 72 | 16 | RCMA | YES | NO |
| 08 r | 62 | 24 | RCMA | YES | YES |
| 09 | 69 | 48 | LCMA | YES | YES |
| 10 | 48 | 48. | LCMA | YES | YES |

(continued)

| PATIENT | AGE IN YEARS | MOMENT OF THE STUDY IN HOURS | I DIAGNOSE | TPECc-DF | CAT |
|---------|--------------|------------------------------|------------|----------|-----|
| 11* | 66 | 24 | LCMA | NO | YES |

Non-linear Analysis of $\gamma(\mathbf{x},t)$

Another preferred, but not exclusive, embodiment of this invention is the TPECc in the time domain. In this case the method is used to identify activated cerebral regions and their functional connections when one has an individual anatomical brain atlas for the subject under study. Once the $\gamma(\mathbf{x},t)$ are estimated they are analyzed as non-linear time series. The values of $\gamma(\mathbf{x},t)$ are grouped in a vector $\gamma_t = [\gamma(\mathbf{x}_i,t)]_{1\leq i\leq N_g}$ to which a Nonparametric Nonlinear Autoregressive Model is fitted (NNAM);

$$\gamma_t = \mathbf{F}\left(\Gamma_t^q, t\right) + \varepsilon\left(\Gamma_t^q, t\right)$$

where $\Gamma_t^q = \left[\gamma_{t-k}\right]_{1\leq k\leq q}$ is the matrix of pasts which constitutes the previous state of the Markovian system (q is the maximal order of the past included). Both the Autoregressive function F as well as the matrix of covariances, $\Sigma_\gamma\left(\Gamma_t^q, t\right)$ of $\varepsilon\left(\Gamma_t^q, t\right)$ are dependent both on the previous state as well as on the time instant t, for which reason the model is also non-stationary and heteroscedastic. Both the Autoregressive function F as well as the matrix of covariances, $\Sigma_\gamma\left(\Gamma_t^q, t\right)$ are estimated using non parametric methods (Hastie and Tibshirani, 1990) in the form of Splines (Wahba, 1990), local polynomials (Fan J. and Gibjels. I., Local Polynomimal Modeling and its Applications. Chapman and Hall), SVM (Smola A., (1996) Regression Estimation with Support Vector Machines. Ph.D. Dissertation Physik Department, Technische Department, Technische Universität München) or regression in Megadictionaries.

The evaluation of F on the support of the series of time $\gamma_t$ generates a vector $\mathbf{x}_\gamma^{NL}$ of DP that contains information about the morphology of the grapho-elements of the physiological series that has been demonstrated to be more informative than the one provided by polyespectra for the recognition of the activity of ictal activity in the EEG of epileptics (Hernández, J,L., Valdés, P.A., and Vila, P. (1996) EEG spike and wave modeled by to stochastic limit cycle. NeuroReport, 7: 2246-2250). The cluster analysis and pattern recognition of the EEG is carried out now with $\mathbf{x}_\gamma^{NL}$, using for this purpose not only simple linear methods but also non Parametric methods (Ripley B. D. (1966) Pattern Recognition and Neural Networks. Cambridge University Press).

Additionally, the estimate of F allows the calculation of estimates of Granger causality among generators of PEC by means of the following non-linear and non-stationary generalization of influence measures introduced by Geweke (1984). Let us denote for A, B and C as subsets (with an empty intersection) of the time series of that comprise $\gamma_t$. Let us define the matrix of covariances $\Sigma_\gamma\left(\Gamma_t^q, t; A|B.C\right)$ as the one obtained from A when the influence of B is eliminated by non parametric regression conditional on C. Then the (directed) influence of B on A, conditional to C as the natural logarithm of the ratios of determinants:

$$I_{B\to A/C} = \ln\left(\frac{\left|\Sigma_\gamma\left(\Gamma_t^q, t; A|\{\ \}C\right)\right|}{\left|\Sigma_\gamma\left(\Gamma_t^q, t; A|B.C\right)\right|}\right)$$

The potential utility of the influence measures combined with the TPECc in the evaluation of the epileptic pathology is now illustrated. Estimators of $\gamma_t$ were obtained based on the EEG recording of a patient with a complex partial seizure (of focal origin and secondary propagation resulting in loss of consciousness). The patient was an 11 year-old girl whose recording is shown in Figure 12. In Figure 13 the estimated variance of $\gamma_t$, is shown it being possible to observe four active sites (indicated with the letters A to D in the figure). The calculation of the influence measures indicated the direction of flow of the information among presumed epileptic foci. As can be seen focus A influenced all the other presumed foci and others did not influence it. The place of greatest activity and presumed primary origin of the crisis was considered as A, which corresponded to the clinical diagnosis.

BRIEF DESCRIPTION OF THE FIGURES

Figure 1
Cerebral anatomical atlas based on an individual structural image. a) Axial slice of a MRI, to which a procedure of contour detection is applied; b) Outline of the detected skin; c) 3D reconstruction of the triangulation of the surface of the skin using the contours detected in all the slices.

Figure2
Cerebral anatomical atlas based on a probabilistic structural image in the Talairach reference system. a) Axial slices of the average image; b) image of the probability of appearance of gray matter; c) image of the probability of appearance of the gray matter of the occipital lobe; d) segmentation of different structures of the gray matter represented by a discrete scale of colors.

Figure3
This figure is a diagram showing a System for obtaining the TPEC, comprising the following parts presented here, without loss of generality, for the particular embodiment for the TPECc. These elements are those described next and designated by the letters used for their identification: a) A plurality of external sensors of electric and/or magnetic fields which are placed in the proximity of the subject's body. In the case of TPECc, said sensors are placed in the proximity of the subject's head. In the case of TPECk, said sensors are placed in the proximity of the subject's torso. These sensors are connected to the amplification sub-system (b) consisting of electronic pre-amplifiers and amplifiers that record the signals picked up by said sensors and that carry out the pre-conditioning of these signals for their later analog to digital conversion. The amplification sub-system is connected to the sub-system of analog to digital conversion (1-c) that transforms the amplified signals into a sequence of binary numbers. Said analog to digital conversion sub-system is connected to a general-purpose digital computer that acts as a control unit (CU). (i) The digitized signals are stored in the memory of the CU, which is, in turn, coupled to external sub-systems for digital storage (d) and that allow the reading and storage of:

- Anatomical atlas of the brain or of the heart in the reference system of the head or the torso, respectively.

- Functional images of the brain or heart of a subject obtained by other biophysical procedures and defined in the reference system of the head or of the torso.

The system also comprises a device for detecting the position of sensors (f) that emits in digital form, the coordinates of these sensors as well as the coordinates of external anatomical structures of the subject's head or torso. These coordinates are defined in said reference system, and are stored in the CU.. Additionally, the system comprises a plurality of stimulating devices (g) connected with the CU that emit auditory, visual sensorial stimuli, as well as heat, electric, and magnetic pulses for the purpose of stimulating the subject's nervous or cardiovascular system. To the CU are also connected a plurality of sensor devices (h) that measure the motor, physiological and verbal reactions of the subject under study (Responses of the subject). The CU (i) is a general-purpose computer in whose memory a group of instructions resides (Programs) for the control of the realization of studies. The activities of the system for TPEC that are controlled by this program include: the activation of the stimulating devices according to a preset design (conceived with the purpose of exploring reactions of the subject); the control of the acquisition of the EEG/MEG/EKG/MKG; the determination of the positions of the sensors; the recording of the activity of the subject under study in the same time reference system as that of the EEG/MEG/EKG/MKG; the acquisition of the functional images coming from other biophysical modalities; and the digital processing of all the information acquired in a study of a subject for the construction of three-dimensional statistical maps of the temporal evolution of the functional states of the brain.

Figure4
Electrodes of the standard 10/20 system for the recording of the EEG shown on the skin of the probabilistic brain atlas in Talairach reference system.

Figure5
Illustration of the principle of reciprocity for a compartment $R_j$ surrounded by a surface S of arbitrary shape. Elemental

voltage difference $\delta V_{er}^{g}(t)$ and projection of the magnetic vector field $\delta b_{cn}^{g}(t)$ that appears due to the presence of a point PEC $\mathbf{j}_{P}(t)\delta\mathbf{r}^3$ inside the compartment $R_j$.

Figure6
Illustration of the principle of reciprocity for a compartment surrounded by a surface S of arbitrary shape. Densities of ohmic currents that appear inside the $R_j$ compartment in the absence of active PEC, as a result of energizng the: a) electric sensors with a direct current $I_{er}$ and b) the magnetic sensors with an alternating current $I_c(\omega)$ of low frequency.

Figure7
Graphical representation of an isotropic piecewise homogeneous volume conductor (IPHC).

Figure8
Sequence of operations with the subject. The use of the system described for obtaining TPEC consists of the following operations: Placement of the subject in seated or lying position in the proximity of the system described. Positioning of a group of sensors (electrodes and magnetic sensors) in order to detect and to record the electric and magnetic signals that represent the cerebral and heart physiological activity in the form of recordings of EEG/MEG/EKG/MKG. Use of the device for the measurement of the coordinates of the sensors and the external anatomical structures of the head or the torso for their storage in the memory of the CU. Initiation of the collection of data of the subject, controlled by the programs stored in the memory of the CU. Gathering and analog to digital conversion of the electric and/or magnetic activity of the brain or heart of the subject by the use of the systems for amplification and analog to digital conversion. This step may optionally be accompanied by the presentation of stimuli using the devices described and the acquisition of the Responses of the subject. The picked up signals are stored in form of chronological series of EEG/MEG/EKG/MKG. Removal of the subject from the system. Pre-processing of the signals recorded from the subject for the elimination of artifacts and of non-physiological frequency components.

Sequence of operations used by the Program.

Input of the observations (EEG/EKG; MEG/MKG; optionally of functional Images such as fMRI, PET, SPECT).

Input of the positions of the sensors.

Input of an anatomical atlas, this may be either an individual or a probabilistic structural image.

Conversion of the position of the sensors to the reference system of the anatomical atlas.

Calculation of the a priori probabilities obtained from the anatomical atlas.

Input of the conductive properties.

Calculation of the electric and magnetic LFs using the positions of the modified sensors.

Calculation of the PEC j(t).

Calculation of observable descriptive parameters (DP) of the preprocessed signals already. These DP are sufficient statistics of models of stochastic processes. Particular, but not exhaustive, instances of these statistics are:

- Averages, variances and covariances or higher order cumulants calculated as invariant in the time or, alternatively, as non-stationary.

- cross polyspectra of all the channels of recorded EEG/EKG and MEG/MKG, calculated as invariant in the time or, alternatively, as non-stationary;

- Multivariate Autoregressive functions fitted to the EEG/EKG and MEG/MKG, both parametric (linear, quadratic) as well as non-parametric.

- Correlation measures, mutual information, Granger causality between channels of EEG/EKG and MEG/MKG.

- Regression coefficients of any one of the previous types of parameters with respect either to the stimuli emitted by the system or to the subject's answers.

Figure9
Illustrates the equations that describe the normal variation of the TPECc in the frequency domain . Each sub plot shows, for points of the representative cerebral lattice, the regression surface of the logarithm of the spectrum of the PEC (axis z) with regard to frequency (x) and age (z).

Figure 10
Z image of the TPECc in the frequency domain of a 27 year old female patient who was diagnosed as having an astrocytoma in the left fronto-parietal region of the brain. For purposes of comparison other imaging modalities for this patient are shown in b) spiral CAT, c) T1 MRI, d) SPECT and) T2 MRI.
*Figure11*
Maximum Intensity Projection of the Z transform of the logarithm of the TPECc in the frequency domain of two patients with Cerebrovascular Accident of the a) Left and b) Right Middle Cerebral Artery.
*Figure 12*
EEG recording of a patient with complex partial epilepsy. The recording presents spike and wave activity.
*Figure 13*
TPECc in the time domain of the recording of Figure 12 in which the major sources of PEC are indicated as well as the direction of flow of information as determined by the measures of non-linear influence, not stationary.

**Claims**

1. A Method of obtaining a Tomography of the Primary Electric Current (PEC) from the signals originated either from the brain or the heart using an array of external electrical and biomagnetic sensors and a set of anatomical and medical images, the method comprising the steps of:

   a) Measuring the electrical and/or magnetic signals of a test subject from the sensor array, defining observations for a common time t, designating the EEG/EKG as $\mathbf{o}_1(t)$ and the MEG/MKG as $\mathbf{o}_2(t)$ respectively, obtaining as a result a vector time series defined on the time interval $\mathfrak{I} = [0,T]$.
   b) Specifying the coordinates of the sensors in the sensor array for the test subject as with regard to the reference system of the head or torso said set of coordinates being designated $\aleph_1$ and $\aleph_2$ for the EEG/EKG and MEG/MKG respectively.
   c) Selecting an anatomical image, be it of the test subject or a suitable reference image, and specifying the Cartesian coordinates, in the common reference system, of its constituent voxels as the lattice of the volume conductor $\mathfrak{R}_V$.
   d) Specifying for each point $\mathbf{r}_v$ of $\mathfrak{R}_V$ a given tissue type "$s$" thus defining an Anatomical Atlas.
   e) Labeling each point $\mathbf{r}_V$ of the lattice of the volume conductor with the tissue type.
   f) Specifying for each point $\mathbf{r}_v$ of the lattice of the volume conductor $\mathfrak{R}_V$ the conductivity value corresponding to the tissue label $s_v$, said values being taken from a predefined set of conductivities, designated as the conductivity profile σ.
   g) Selecting the points of the generating Rv in order to define the set $\mathfrak{R}_g$ of $N_g$ with points $\mathbf{r}_g$, the generating volume.
   h) Calculating the electric $\mathbf{K}_1$ and magnetic $\mathbf{K}_2$ lead field matrices by the vector versions of the Finite Element Method (FEM) or Finite Difference Method (FDM) that for an inhomogeneous and anisotropic volume conductor such that given σ, $\aleph_1$, $\aleph_2$, $\mathfrak{R}_g$ and $\mathfrak{R}_V$ the following equations hold: $\mathbf{o}_1(t) = \mathbf{K}_1\mathbf{f}_1(t)$, $\mathbf{o}_2(t) = \mathbf{K}_2\mathbf{f}_2(t)$, where $\mathbf{f}_1(t)$ and $\mathbf{f}_2(t)$ are used interchangeably to denote the PEC.
   i) Selecting, when available, one or more of the images of the following functional modalities: Functional magnetic resonance (fMRI) Positron Emission Tomography (PET) and Single Photon Emission Computed Tomography (SPECT) defining observations $\mathbf{o}_3(t)$, $\mathbf{o}_4(t)$ and $\mathbf{o}_5(t)$ respectively, being those of the test subject or some suitable reference image.
   j) Specifying voxel positions as the set of Cartesian coordinates, in the common reference system, for each selected functional image, said set of positions being designated $\aleph_3$, $\aleph_4$, and $\aleph_5$ for fMRI, PET and SPECT respectively.
   k) Specifying the time instants for which each selected functional image m has been sampled, in a common time scale with for fMRI, PET and SPECT respectively
   l) Calculating the kernel $\mathbf{K}_m$ that expresses our knowledge that the ideal functional indicator $\mathbf{f}_m(t)$ associated to the m-th image modality ($m$=3,4,5) defined on $\mathfrak{R}_V$ and for the time instants sampled is modified by the image formation process according to the transformation $\mathbf{o}_m(t) = \mathbf{K}_m * \mathbf{f}_m(t)$ which:

- Reduces the spatial resolution of $\mathbf{f}_m(t)$ from that of $\mathfrak{R}_V$ to that of $\aleph_m$
- Reduces the temporal resolution of $\mathbf{f}_m(t)$ from that of electrophysiological data (m=1) to that of any other modality m.

m) Calculating the physiological operator $H_m$ that expresses our knowledge that the neural or cardiac tissue activation A(t) defined on $\mathfrak{R}_g$ and $\mathfrak{I}_m$ produces physiological processes associated to the m-th image modality (m=1,2,3,4,5) according to the transformation $\mathbf{f}_m(t) = \mathbf{H}_m \circ \mathbf{A}(t)$.

n) Calculating estimates of the PEC by joint estimation of all the $\mathbf{f}_m(t)$ and of $\mathbf{A}(t)$ for all observed m, said estimation comprising steps of:

    i. Assigning arbitrary initial values to the $\mathbf{f}_m(t)$ and $\mathbf{A}(t)$
    ii. Iteratively modifying the values of $\mathbf{f}_m(t)$ and $\mathbf{A}(t)$
    iii. Calculating a probability measure $p$ which increases when values of $\mathbf{f}_m(t)$ and $\mathbf{A}(t)$ simultaneously

        - Reconstruct the $\mathbf{o}_m(t)$ with small error as quantified by a risk function and
        - Have a small norm in a given Besov space.

    iv. Continuing step ii until the measure $p$ does not increase.

o) Calculating the following descriptive parameters

    - $\gamma_m(t) = \mathbf{f}_m(t)$ m=1,2,3,4,5 and $\gamma_6(t) = \mathbf{A}(t)$): for $\mathbf{r}_i \in \mathfrak{R}_g$
    - A value $I_{B \to A/C}$ that quantifies the directed influence of brain or heart region $B$ on region A that is not due to the activity coming from region $C$.

p) Specifying reference groups of subjects for which descriptive parameters have been summarized statistically and calculating:

    - The probabilities $p(\gamma_m)$ that the descriptive parameters measured obtained in claim 1-o for the test subject are typical of a given reference groups.

    - The probabilities $p\left(I_{A \to B/C}^m\right)$ that the descriptive parameters measured obtained in claim 1-o for the test subject and are typical of a given reference groups.

q) Coding the values of $p(\gamma_m)$ and $p\left(I_{A \to B/C}^m\right)$ by means of a color scale and displaying said codes overlaid on the selected anatomical image highlighting statistically significant regions by thresholding to zero those values beneath a chosen significance level to obtain a statistical parametric map for the Tomography of PEC.

2. The method defined in claim 1, wherein the common reference system is the Talairach coordinate system.

3. The method defined in claim 1, wherein the anatomical atlas is obtained according to one of the following variants:

    a) By the extraction of tissue probabilities of anatomical images obtained for the specific subject under study being these any one of the following types: Computed Axial Tomography (CAT), Magnetic Resonance Images (MRI), post-mortem sections of the head obtained by cryotomy, etc. said atlas being designated as an Individual Anatomical Atlas.
    b) By a rigid or elastic transformation of the subject's volume conductor lattice in order to ensure the best correspondence possible to a canonical volume conductor lattice for which a tissue probability distribution has obtained from a sample of normal or pathological anatomical images, said atlas being designated as a Probabilistic Anatomical Atlas.

4. The method defined in claim 1, wherein the computation of the Electric and Magnetic Lead Fields matrices by the vector versions of the Finite Element Method (FEM) or Finite Difference Method (FDM) for an inhomogeneous and anisotropic volume conductor comprising the following steps:

a) Specifying the positions of the sensors for the lattice of EEG/EKG and MEG/MKG modality $\aleph_1$ and $\aleph_2$, respectively as defined in claim 1-b.

b) Specifying the positions $\mathbf{r}_g$ and $\mathbf{r}_V$ in the lattice of the generating volume $\mathfrak{R}_g$ and of the volume conductor $\mathfrak{R}_V$, respectively as defined in claims 1-c and 1-f.

c) Specifying the conductivity profile as defined in claim 1-g using an approximation such that each set of tissue labels constitutes a collection of N embedded regions for which the conductivity value is constant, $\sigma=\{\sigma_1,\cdots,\sigma_N\}$.

d) Calculating numerically the ohmic current densities $\mathbf{j}_{kv} = \mathbf{j}_k(\mathbf{r}_V)$ and $\mathbf{j}_{kV}^{\omega} = \mathbf{j}_k(\mathbf{r}_V,\omega)$ on each point $\mathbf{r}_V$ of the lattice of volume conductor $\mathfrak{R}_V$, that belongs to the surfaces limiting the regions by means of the following Linear Algebraic Systems of Equations: $\mathbf{Dc}=\mathbf{c}_\infty -\Gamma\mathbf{c}$; wherein $\mathbf{c}=(\mathbf{c}_1;...;\mathbf{c}_N)$; said $\mathbf{c}_k =\{\mathbf{j}_{kv},V=1,\cdots,N_{k,k+1}\}$ for the EEG/EKG or $\mathbf{c}_k = \left\{\mathbf{j}_{kV}^{\omega},V=1,\cdots,N_{k,k+1}\right\}$ for the MEG/MKG; $N_{k,k+1}$ is the number point $\mathbf{r}_v$ that belong to the surface separating the regions k and k+1. The matrices $\Gamma$ and $\mathbf{D}$ are defined as:

e)

$$\Gamma = \frac{1}{4\pi} \begin{pmatrix} \dfrac{(\sigma_1-\sigma_2)}{\sigma_1}\Gamma_{11} & \dfrac{(\sigma_2-\sigma_3)}{2}\Gamma_{12} & \cdots & \Gamma_{1N} \\[2ex] \dfrac{(\sigma_1-\sigma_2)}{\sigma_1}\Gamma_{21} & \dfrac{(\sigma_2-\sigma_3)}{\sigma_2}\Gamma_{22} & \cdots & \Gamma_{2N} \\[2ex] \vdots & \vdots & \ddots & \vdots \\[2ex] \dfrac{(\sigma_1-\sigma_2)}{\sigma_1}\Gamma_{N1} & \dfrac{(\sigma_2-\sigma_3)}{\sigma_2}\Gamma_{N2} & \cdots & \Gamma_{NN} \end{pmatrix}$$

$$\mathbf{D} = \begin{pmatrix} \alpha_1\mathbf{I}_{N_{1,2}} & 0 & \cdots & 0 \\ 0 & 0 & & \vdots \\ \vdots & & \ddots & 0 \\ 0 & \cdots & 0 & \alpha_N\mathbf{I}_{N_{N,N}} \end{pmatrix};$$

f) where $\alpha_j = \dfrac{(2\sigma_j+\sigma_{j+1})}{3\sigma_j}$; $\Gamma_{jk} = \begin{pmatrix} \Gamma_1^k(\mathbf{r}_1^j) & \cdots & \Gamma_{N_{k,k+1}}^k(\mathbf{r}_1^j) \\ \vdots & \ddots & \vdots \\ \Gamma_1^k(\mathbf{r}_{N_{J,J+1}}^j) & \cdots & \Gamma_{N_{k,k+1}}^k(\mathbf{r}_{N_{J,J+1}}^j) \end{pmatrix}$; $\mathbf{r}_i^j$ labels the *i-th* point of

$\mathfrak{R}_V$ that belong to j-th surface; $\mathbf{c}_\infty = \left(\mathbf{c}_\infty^1;...;\mathbf{c}_\infty^N\right)$, with $\mathbf{c}_\infty^k = \left\{\mathbf{j}_{kV}^\infty,V=1,\cdots,N_{k,k+1}\right\}$ for the EEG/EKG

and $\mathbf{c}_\infty^k = \left\{\mathbf{j}_{kV}^{\omega\,\infty},V=1,\cdots,N_{k,k+1}\right\}$; for the MEG/MKG. The magnitudes $\mathbf{j}_{kV}^\infty = \mathbf{j}_{k\infty}(\mathbf{r}_V)$ and

$\mathbf{j}_{kV}^{\omega\,\infty} = \mathbf{j}_{k\infty}(\mathbf{r}_V,\omega)$ are evaluated from the expressions:

g) $\mathbf{j}_{k\infty}(\mathbf{r}) = -\dfrac{I_{er}}{4\pi}\nabla\cdot\left(g(\mathbf{r},\mathbf{r}_e)-g(\mathbf{r},\mathbf{r}_r)\right)$

h) $\mathbf{j_{k\infty}}(\mathbf{r},\omega) = \dfrac{i\omega}{4\pi}\sum\limits_{k=1}^{N}(\sigma_k - \sigma_{k+1})\oiint\limits_{S_{k,k+1}} g(\mathbf{r},\mathbf{r}')\cdot\big(\mathbf{n}_k(\mathbf{r}')\times\mathbf{b}_c(\mathbf{r}',\omega)\big)d\mathbf{r}'^2$, Being $\mathbf{b}_c(\mathbf{r}',\omega)$ the mag-

netic field generated by the low frequency ac current passing through a single coil in a infinite and homogeneous volume conductor.

i) The function $\Gamma_n^k(\mathbf{r}) = \int\limits_{\Delta_n^k}\nabla'g(\mathbf{r},\mathbf{r}')\cdot d\mathbf{r}'^2 - \int\limits_{\Delta_n^k}\nabla'g(\mathbf{r},\mathbf{r}')\circ d\mathbf{r}'^2$, where $g(\mathbf{r}_g,\mathbf{r}')$ is the Green Function of the infinite domain.

j) Calculating the Electric and Magnetic Lead Field in each compartment of an IPHC by using the reciprocity

theorems: $\mathbf{k}_{eg} = -\dfrac{\mathbf{j}_j(\mathbf{r}_g)}{\sigma_j I_{er}}$ at the lead electrode "er"; and $\mathbf{k}_{cg} = -\dfrac{\mathbf{j}_j(\mathbf{r}_g,\omega)}{i\omega\sigma_j I_c(\omega)\Delta S_c}$ at the coil "co",

respectively. The ohmic current densities are evaluated at any point $\mathbf{r}_g$ of the lattice of the generating volume $\mathfrak{R}_g$ by the use of the following expressions:

k) Electric Lead Field: $\mathbf{j}_j(\mathbf{r}_g) = \mathbf{j}_{k\infty}(\mathbf{r}_g) - \dfrac{1}{4\pi}\sum\limits_{k=1}^{N}\dfrac{(\sigma_k - \sigma_{k+1})}{\sigma_k}\sum\limits_{V=1}^{N_{k,k+1}}\Gamma_V^k(\mathbf{r}_g)\mathbf{j}_{kV}$

l) Magnetic Lead Field: $\mathbf{j}_j(\mathbf{r}_g,\omega) = \mathbf{j}_{k\infty}(\mathbf{r}_g,\omega) - \dfrac{1}{4\pi}\sum\limits_{k=1}^{N}\dfrac{(\sigma_k - \sigma_{k+1})}{\sigma_k}\sum\limits_{V=1}^{N_{k,k+1}}\Gamma_V^k(\mathbf{r}_g)\mathbf{j}_{kV}^\omega$

m) Calculating the Electric $\mathbf{K}_1 = \{\mathbf{k}_{eg}^t\}$ and Magnetic $\mathbf{K}_2 = \{\mathbf{k}_{cg}^t\}$ Lead Fields matrices specifying a row of said matrices for each sensor (EEG/EKG $e = 1,\cdots, N_e$ and MEG/MKG $c = 1,\cdots, N_c$); and a column for each point of the lattice of the generating volume $\mathbf{r}_g \in \mathfrak{R}_g$.

**5.** The method defined in claim 1, wherein the calculation of the kernel operator $\mathbf{K}_m$ is specified being with the spatial and temporal resolution associated to the m-th image modality (i=3,4,5).

**6.** The method defined in claim 1, wherein the estimation of the PEC f, (t) for a given subject comprises steps of:

a) Assigning initial values to the following sets of variables:

1. Tissue activation $\mathbf{A}(t) = \big[A(\mathbf{r}_g,t)\big]_{\mathbf{r}_g\in\mathfrak{R}_g}$ for each point $\mathbf{r}_g$ of $\mathfrak{R}_g$

2. Functional indicators $\mathbf{f}_m(t) = \big[\mathbf{f}_m(\mathbf{r}_g,t)\big]_{\mathbf{r}_g\in\mathfrak{R}_g}$ for each point $\mathbf{r}_g$ of $\mathfrak{R}_g$ where $\mathbf{f}(t) = [\mathbf{f}_m(t)]_{1\leq m\leq M}$ denote

the totality of measurement modalities obtained for the subject.
3. Hyperparameter of the activation $\Theta_A$,
4. Hyperparameter of the intrinsic noise of the functional indicators $\Theta_\beta = [\Theta_{\beta_m}]_{1\leq m\leq M}$,
5. Hyperparameter of the instrumental noise $\Theta_e = [\Theta_{e_m}]_{1\leq m\leq M}$

b) Computing the a posteriori probability:

$$P\left(\mathbf{A}(t),\Theta_{\mathbf{A}},\mathbf{f}(t),\Theta_{\beta},\Theta_{\mathbf{e}}\big|\mathbf{o}(t)\right) \propto$$

$$\prod_{m=1}^{M} l_{\mathbf{o}_m}\left(\mathbf{o}_m(t)-\mathbf{K}_m * \mathbf{f}_m(t)\big|\Theta_{\mathbf{e}_m}\right) \times$$

$$\prod_{m=1}^{M} \pi_{\mathbf{f}_m}\left(\mathbf{f}_m(t)-\mathbf{H}_m \circ \mathbf{A}(t)\big|\Theta_{\beta_m}\right) \times$$

$$\prod_{m=1}^{M} \left\{\pi_{\Theta_{\mathbf{e}_m}}\left(\Theta_{\mathbf{e}_m}\right)\cdot\pi_{\Theta_{\beta_m}}\left(\Theta_{\beta_m}\right)\right\} \times$$

$$\pi_{\mathbf{A}}\left(\mathbf{A}(t)\big|\Theta_{\mathbf{A}}\right)\times\pi_{\Theta_{\mathbf{A}}}\left(\Theta_{\mathbf{A}}\right)$$

that comprises the following multiplicative terms:

    1) Likelihood terms $l_{o_m}$ for each modality m $l_{o_m}$ ($\mathbf{o}_m(t)$ - $\mathbf{K}_m$ * $\mathbf{f}_m(t)|\Theta_{e_m}$)
    2) The a priori probabilities for the functional indicators

$$\pi_{\mathbf{f}_m}\left(\mathbf{f}_m(t)-\mathbf{H}_m \circ \mathbf{A}(t)\big|\Theta_{\beta_m}\right)$$

    3) The a priori probability for the activation $\pi_A(\mathbf{A}(t)|\Theta_A)$
    4) The a priori probability for the hyperparameters of the activation $\pi_{\Theta A}(\Theta_A)$

    5) The a priori probabilities for the hyperparameters of the intrinsic noise of the functional indicators

$$\pi_{\Theta_{\beta_m}}\left(\Theta_{\beta_m}\right)$$

    6) The a priori probabities for the hyperparameters of the instrumental noise $\pi_{\Theta_{e_m}}\left(\Theta_{e_m}\right)$

c) Iteratively modifying the parameters $\mathbf{A}(t),\Theta_{\mathbf{A}},\mathbf{f}(t),\Theta_{\beta},\Theta_e$ until a maximum value of the a posteriori probability is achieved, said modification being carried out according to one of the following schemes:

    1. Successive Maximization of $P(\mathbf{A}(t),\Theta_{\mathbf{A}},\mathbf{f}(t),\Theta_{\beta},\Theta_e|\mathbf{o}(t))$ for subsets of the parameters, while maintaining fixed all the others, "Iterated Conditional Maximization" (ICM).
    2. Successive Maximization of $P(\mathbf{A}(t),\Theta_{\mathbf{A}},\mathbf{f}(t),\Theta_{\beta},\Theta_e|\mathbf{o}(t))$ for subsets of the parameters, fixing the other parameters to their expected value, "Expectation Maximization" (EM).
    3. Choosing the mode of the distribution of $P(\mathbf{A}(t),\Theta_{\mathbf{A}},\mathbf{f}(t),\Theta_{\beta},\Theta_e|\mathbf{o}(t))$ obtained by means of Monte Carlo Markov Chain methods (MCMC).

**7.** The method in claim 6 wherein the electrophysiological observations $\mathbf{o}_m(t)$ m=1,2 for the estimation of $\mathbf{f}_1(t)$ comprises any one of the following combinations: EEG alone, MEG alone, EEG and MEG together.

**8.** The method in claim 6 wherein either

    • None of the the functional images $\mathbf{o}_m(t)$ m=3,4,5 is used for the estimation of $f_1(t)$ or, alternatively,
    • Some of these measure are used in any one of the following combinations: fMRI alone, PET alone, SPECT alone, fMRI and SPECT, fMRI and SPECT, PET and SPECT.

**9.** The method in claim 6 wherein the calculation of the likelihoods $l_{o_m}(\mathbf{o}_m(t) - \mathbf{K}_m * f_m(t)|\Theta_{em})$ comprise the following steps:

    a) Selecting the use of either ε insensitive polynomial risk or a generalized polynomial risk $R_x$ [$\mathbf{x}|\Theta_x$] for the specific purpose of evaluating non gaussian likelihoods.
    b) Calculating the discrepancy vector $\Delta = \mathbf{o}_m(t) - \mathbf{K}_m * f_m(t)$
    c) Rescaling the $\Delta$ as a function of the hyperparameters $\Theta_{em}$

d) Calculating $R_x^C\left[\Delta\middle|\Theta_x\right]$, the risk of $\Delta$

e) Calculating $l_{0_m}\left(\Delta\middle|\Theta_{c_m}\right)=\exp\left(-B\ R_x^C\left[\Delta\middle|\Theta_x\right]\right)$ where $B$ is a preselected constant

**10.** The method in claim 6 wherein the calculation of the a priori probability $\pi_{t_m}$ ($\mathbf{f}_m(t)$ - $\mathbf{H}_m$∘$\mathbf{A}(t)|\Theta_{\beta m}$) for the functional indicators comprises the following steps:

a) Selecting the indices indices m,n,s of the Besov space $B_{n,s}^{m}$ with the purpose of defining the smoothness of the estimated functional indicator, a particular but not exclusive choice being $m=1$, $n=1$, $s=1$ which defines the "algebra of bumps".
b) Calculating the discrepancy vector $\Delta = \mathbf{f}_m(t)$ - $\mathbf{H}_m \circ \mathbf{A}(t)$
c) Rescaling the $\Delta$ as a function of the hyperparameters $\Theta_{\beta m}$

d) Calculating $\left\|\Delta\right\|_{B_{n,s}^m}$, the norm of the $\Delta$ in $B_{n,s}^{m}$, said calculation being carried out by first expanding $\Delta$ as

$\Delta = \sum_k F_k \cdot \psi_k$ where the $\psi_k$ belong to a Dictionary of Atoms (particular but not exhaustive instances of which are the Wavelet dictionary, Fourier dictionary, Dirac delta dictionary or a mega-dictionary defined by the union of several dictionaries) and then calculating $\left\|\Delta\right\|_{B_{n,s}^m} \approx \sum_k a_k \cdot \left\|F_k\right\|^m$

e) Calculating $\pi_{f_m}\left(\Delta\middle|\Theta_{\beta_m}\right)=\exp\left(-C\ \left\|\Delta\right\|_{B_{n,s}^m}\right)$ where C is a preselected constant.

**11.** The method in claim 6 wherein the calculation of the a priori probability of the activation $\pi_A(\mathbf{A}(t)|\Theta_A)$ comprises the following steps:

a) Calculating a non decreasing function $h(\square)$ of the probabilistic atlas $h(p_g)$

b) Calculating the norm $\left\|\mathbf{A}(t)\right\|_{B_{n,s}^m}$ as specified in claim 10

c) Calculating $\pi_A\left(\mathbf{A}(t)\middle|\Theta_A\right)=\exp\left(-D\ h\left(p\left(s,\mathbf{r}_g\right)\right)-E\left\|\mathbf{A}(t)\right\|_{B_{n,s}^m}\right)$ where $D$ and $E$ are prespecified constants.

**12.** The method in claim 6 wherein the of the a priori probability for the PEC takes the following specific form:

$$\pi_{f_l}\left(\mathbf{f}_l(t)-\mathbf{H}_l\circ\mathbf{A}(t)\middle|\Theta_{\beta_l}\right)=\pi_1\left(\mathbf{f}_l(t)-\mathbf{M}\ \mathbf{A}(t)\middle|\Theta_1\right)\pi_2\left(\mu\middle|\Theta_2\right)$$

where $\pi_1$ and $\pi_2$ are a priori probabilities specified as in claim 10,

$$\mathbf{M} = \begin{bmatrix} \mu_1 & 0 & \cdots & 0 & 0 \\ 0 & \mu_2 & \cdots & 0 & 0 \\ \cdots & \cdots & \mu_1 & \cdots & \cdots \\ 0 & 0 & \cdots & \mu_{N_g-1} & 0 \\ 0 & 0 & \cdots & 0 & \mu_{N_g} \end{bmatrix}, \quad \mu = \begin{bmatrix} \mu_1 \\ \mu_2 \\ \cdots \\ \mu_{N_g} \end{bmatrix}$$

where the $\mu_g$ are the orientations of the PEC for each point of $\mathfrak{R}_g$.

**13.** The method in claim 6 wherein the a priori probability $\pi_2(\mu|\Theta_2)$ defined in claim 12 is calculated by either of the following expressions:

- $\pi_2\left(\mu|\Theta_2\right) = \exp\left(-\left\|\Lambda_s \cdot \mu \cdot \Lambda_m\right\|_{B_{n,s}^m}\right)$ where $\Lambda_s \cdot$ is a diagonal matrix that specifies the degree of smoothness that will be imposed at each point of $\mathfrak{R}_g$ and $\Lambda_m = \mathbf{W} \cdot \mathbf{P}$, where $\mathbf{W}$ is a prespecified weighting matrix and $\mathbf{P}$ is a diagonal matrix containing $p_g$ for all $\mathbf{r}_g$.

- By means of successive evaluation of the marginal distributions of

$\pi_2\left(\mu_g|\Theta_2\right) = p_g \cdot N\left(0,\sigma_A^2\right) + \left(1-p_g\right) \cdot N\left(0,\sigma_B^2\right)$ with $N(0,\sigma^2)$ the univariate gaussian distribution with mean zero and standard deviation $\sigma$., $\sigma_A^2$ and $\sigma_B^2$ being constants selected to be large and small respectively with respect to the expected variation of the orientations.

**14.** The method in claim 6 wherein the calculations are carried out for $\mathbf{o}_1(t)$ and/or $\mathbf{o}_2(t)$ in the frequency domain by specifying:

- That the electrical signals have been subjected to the Fourier transform, t now denoting frequency.

- $l_{O_m}\left(\mathbf{o}_m(t) - \mathbf{K}_m * \mathbf{f}_m(t)|\Theta_{e_m}\right) = \mathbf{N}_R^{2,p}\left(0,\Sigma_e\right)$ where $\mathbf{N}_R^{2,p}\left(\mu_X,\Sigma_X\right)$ denotes the multivariate real gaussian distribution with mean and variance $(\mu_X,\Sigma_X)$.

- $\pi_{f_m}\left(\mathbf{f}_m(t) - \mathbf{H}_m \circ \mathbf{A}(t)|\Theta_{\beta_m}\right) = \mathbf{N}_R^{2,p}\left(0,0\right), \mathbf{H_m} = \mathbf{I}$, the identity matrix

- $\pi_A\left(\mathbf{A}(t)|\Theta_A\right) = \mathbf{N}_R^{2,p}\left(0,\Sigma_A\right)$

- $\pi_{\Theta_A}\left(\Theta_A\right) = \left\|\Sigma_A\right\|^{-\alpha/2}\exp\left(-\frac{1}{2}Tr\left(\mathbf{Q}\,\Sigma_A^{-1}\right)\right)$ a natural conjugate a priori distribution for complex Wishart matrices where $\alpha$ and Q are prespecified in accordance with prior knowledge.

- $\pi_{\Theta_{\beta_m}}\left(\Theta_{\beta_m}\right)$ and $\pi_{\Theta_{e_m}}\left(\Theta_{e_m}\right)$ are constants defining improper priors.

**15.** The method defined in claim 1, wherein the calculation of the element $I_{B\rightarrow A/C}$ that quantifies the directed influence of brain or heart region $B$ on region A that is not due to the activity coming from region $C$ comprises the steps of:

a) Defining regions $A, B, C$ as sets of arbitrary voxels $\mathbf{r}_i \in \mathfrak{R}_g$ chosen from the generating volume.

b) Defining for any given image modality m the matrix of pasts as $\Gamma_i^q = \left[\gamma_m(t-k)\right]_{1\leq k\leq q}$.

c) Calculating the residuals $\varepsilon\left(\Gamma_i^q,t\right)$ of an autoregressive model over any given brain by fitting the model

$\gamma_t = \mathbf{F}\left(\Gamma_i^q,t\right) + \varepsilon\left(\Gamma_i^q,t\right)$ by means of a nonparametric function F and using the expression

$$\varepsilon\left(\Gamma_i^q, t\right) = \mathbf{F}\left(\Gamma_i^q, t\right) - \gamma_t$$

d) Calculating the matrix $\Sigma_\gamma\left(\Gamma_i^q, t; A|B.C\right)$ as the covariance of the residuals of the nonparametric autoregressive model over the set union of the regions A, B and C.

e) Calculating the matrix $\Sigma_\gamma\left(\Gamma_i^q, t; A|B.C\right)$ as the covariance of the residuals of the nonparametric autoregressive model over the set union of the regions A and C, excluding B.

f) Calculating the influence of region A on B when the influence of region C is eliminated as the expression:

$$I_{B \to A/C} = \ln\left(\frac{\left|\Sigma_\gamma\left(\Gamma_i^q, t; A|\{\ \}.C\right)\right|}{\left|\Sigma_\gamma\left(\Gamma_i^q, t; A|B.C\right)\right|}\right)$$ as the log ratio of the determinants of the matrices

$\Sigma_\gamma\left(\Gamma_i^q, t; A|B.C\right)$ and $\Sigma_\gamma\left(\Gamma_i^q, t; A|\{\ \}.C\right)$.

**16.** The method defined in claim 1, wherein the calculations involved comprise the following steps:

a) Specification of reference groups of subjects for which descriptive parameters $\gamma_m(t)$ have been obtained and for which certain covariates have been measured. Said covariates (an specific but not exclusive example of which is age) are denoted by $\bar{\omega}$.
b) Transformation towards Gaussianity of the descriptive parameters of the both the test subject as well as the subjects in the reference groups by means of the expression: $\gamma'_m(t) = \mathbf{T}(\gamma_m(t))$ in which the descriptive

parameters $\gamma_m(t)$ is transformed by a non-linear function T to obtain the vector $\gamma'_m(t)$.

c) Calculation of the conditional mean $\mu\left(\gamma'_m; \varpi\right)$ and variance, $\sigma\left(\gamma'_m; \varpi\right)$, of each of the descriptive parameters as a function of the control parameters $\bar{\omega}$ in each reference group by means of a non parametric regression;

d) Calculation of the z transform of each descriptive parameter by means of the expression:

$$z = \left(\frac{\gamma'_m - \mu\left(\gamma'_m; \varpi\right)}{\sigma\left(\gamma'_m; \varpi\right)}\right)$$ where each quantitity is evaluated at each point of the generating lattice $\Re_g$ ;

e) - Calculation of the probabilities that $z_{max}$ and $z_{min}$, the maximum and minimum value of z in a region of interest C respectively, exceeds a thresholds Umax, $U_{min}$; respectively said $U_{max}$, $U_{min}$ are thresholds chosen in accordance to prior knowledge;
f) - Calculation of the following transformed quantities:

$$\begin{cases} z' = F^{-1}\left(P(z)\right) & \text{if } z \geq U_{max} \\ z' = 0 & \text{si } U_{min} < z < U_{max} \\ z' = F^{-1}\left(P(z)\right) & \text{si } z \leq U_{min} \end{cases},$$

in which $F^{-1}$ is the inverse gaussian function;

g) Display of the z curves, defined as on dimensional graphs of z' plotted for a fixed point of the generating lattice $\mathfrak{R}_g$ and as a function of either time or frequency; the latter case being designated a z spectrum of the tomography of the PEC.

- Display of the z' values as as two or three-dimensional images for fixed time or frequency" said image being denominated z image of the tomography of the PEC the image being rendered according to a color code that indicates the degree of deviation of the values from the reference gorups, said image being superimposed an anatomical atlas, which constitutes the background of the image.

**Patentansprüche**

1. Verfahren zum Erhalten einer Tomographie des elektrischen Primärstromes (Primary Electric Current) (PEC) aus den Signalen, die entweder von dem Gehirn oder dem Herz stammen, unter Verwendung einer Anordnung von externen elektrischen und biomagnetischen Sensoren und einer Menge von anatomischen und medizinischen Bildern, wobei das Verfahren die Schritte umfasst:

a) Messen des elektrischen und/oder magnetischen Signals eines Testsubjektes mit der Sensoranordnung, wodurch Messungen in einer gemeinsame Zeit t definiert werden, die des EEG/EKG als $\mathbf{o}_1(t)$ bzw. des MEG/MKG als $\mathbf{o}_2(t)$ bezeichnet werden und als ein Ergebnis eine in dem Zeitintervall $\mathfrak{I}$ = [0,$T$] definierte Vektor-Zeit-Reihe erhalten wird.

b) Festlegen der Koordinaten der Sensoren in der Sensoranordnung für das Testsubjekt mit Bezug auf das Referenzsystem des Kopfes oder des Torsos, wobei die Menge der Koordinaten mit $\aleph_1$ und $\aleph_2$ für das EEG/EKG bzw. MEG/MKG bezeichnet werden.

c) Auswählen einer anatomisches Bilds, sei es von dem Testsubjekt oder einer geeigneten Referenzbilds, und Festlegen, in dem gemeinsamen Referenzsystem, der kartesischen Koordinaten seiner Voxelbestandteile als das Gitter des Volumenleiters $\mathfrak{R}_V$ .

d) Festlegen eines gegebenen Gewebetyp "***s***" für jeden Punkt $\mathbf{r}_V$ von $\mathfrak{R}_V$ , um so einen anatomischen Atlas zu definieren.

e) Bezeichnen jedes Gitterpunktes $\mathbf{r}_v$ des Volumenleiters mit dem Gewebetyp.

f) Festlegen, für jeden Gitterpunkt $\mathbf{r}_V$ des Volumenleiters $\mathfrak{R}_V$ , des der Gewebebezeichnung $\mathbf{s}_V$ entsprechenden Leitfähigkeitswertes, wobei die Werte aus einer vorbestimmten Menge von Leitfähigkeiten entnommen werden und als das Leitfähigkeitsprofil σ bezeichnet werden.

g) Auswählen der Punkte der erzeugenden Rv , um die Menge $\mathfrak{R}_g$ von $N_g$ mit Punkten $\mathbf{r}_g$ als das zu erzeugenden Volumen zu definieren.

h) Berechnen der elektrischen $\mathbf{K}_1$ und magnetischen $\mathbf{K}_2$ Führungsfeldmatrizen (lead field matrices) durch die Vektorversionen der Finite Elementen Methode (FEM) oder der Finite Differenzen Methode (FDM) für einen inhomogenen und anisotropen Volumenleiter, so dass bei gegebenen σ, $\aleph_1$, $\aleph_2$, $\mathfrak{R}_g$ und $\mathfrak{R}_V$ die folgenden Gleichungen gelten: $\mathbf{o}_1(t)=\mathbf{K}_1\mathbf{f}_1(t)$, $\mathbf{o}_2(t)=\mathbf{K}_2\mathbf{f}_2(t)$, wobei $\mathbf{f}_1(t)$ und $\mathbf{f}_2(t)$ austauschbar verwendet werden, um den PEC zu bezeichnen.

i) Auswählen, wenn verfügbar, eines oder mehrerer der Bilder der folgenden funktionellen Modalitäten: Funktionale Magnetische Resonanz (Functional magnetic resonance) (fMRI), Positronen-Emissions-Tomographie (Positron Emission Tomography) (PET), Einzel Photonen Emissions-Tomographie (Single Photon Emission Computed Tomography) (SPECT), wodurch Messungen $\mathbf{o}_3(t)$, $\mathbf{o}_4(t)$ bzw. $\mathbf{o}_5(t)$ definiert werden, die diejenigen des Testsubjekts oder eines geeigneten Referenzbilds sind.

j) Festlegen von Voxelpositionen als die Menge der kartesischen Koordinaten in dem gemeinsamen Referenzsystem, für jedes ausgewählte funktionelle Bild, wobei die Menge der Positionen als $\aleph_3$, $\aleph_4$ und $\aleph_5$ für fMRI, PET bzw. SPECT bezeichnet werden.

k) Festlegen der Zeitpunkte, an denen jedes ausgewählte funktionelle Bild m abgetastet worden ist, in einer gemeinsamen Zeitskala für fMRI, PET bzw. SPECT.

l) Berechnen des Kernels $\mathbf{K}_m$, der unser Wissen ausdrückt, dass der ideale funktionelle Indikator $\mathbf{f}_m(t)$, zuge-

ordnet der *m*-ten Bildmodalität (*m* =3,4,5), im $\mathfrak{R}_V$ definiert und für die abgetasteten Zeitmomente aufgenommen, durch den Bilderzeugungsprozess modifiziert wird gemäß der Transformation $\mathbf{o}_m(t)=\mathbf{K}_m{}^*\mathbf{f}_m(t),$ die

- die räumliche Auflösung von $\mathbf{f}_m(t)$ von der von $\mathfrak{R}_V$ auf die von $\aleph_m$ reduziert;
- die zeitliche Auflösung von $\mathbf{f}_m(t)$ von der der elektrophysiologischen Daten (*m* = 1) zu der einer anderen Modalität m reduziert.

m) Berechnen des physiologischen Operators $\mathbf{H}_m$, der unser Wissen ausdrückt, dass die Aktivierung $\mathbf{A}(t)$ des Gehirn- oder Herzgewebes, die in $\mathfrak{R}_g$ und $\mathfrak{I}_g$ definiert ist, physiologische Prozesse erzeugt, die der m -ten Bildmodalität (*m* =1,2,3,4,5) gemäß der Transformation $\mathbf{f}_m(t) = \mathbf{H}_m \circ \mathbf{A}(t)$ zugeordnet sind.

n) Berechnen von Schätzwerten des PEC durch gemeinsame Schätzung aller $\mathbf{f}_m(t)$ und $\mathbf{A}(t)$ für alle beobachteten *m*, wobei die Schätzung die Schritte umfasst:

i. Zuordnen von willkürlichen Anfangswerten zu dem $\mathbf{f}_m(t)$ und $\mathbf{A}(t)$ und
ii. Iteratives Modifizieren der Werte von $\mathbf{f}_m(t)$ und $\mathbf{A}(t)$
iii. Berechnen eines Wahrscheinlichkeitsmaßes *p*, das sich erhöht, wenn die Werte von $\mathbf{f}_m(t)$ und $\mathbf{A}(t)$ gleichzeitig

- die $\mathbf{o}_m(t)$ mit einem geringen Fehler rekonstruieren, der durch eine Risikofunktion quantifiziert wird und
- eine kleine Norm in einem gegebenen Besov-Raum aufweisen. iv) Fortsetzen des Schrittes ii, bis das Maß p nicht weiter ansteigt.

o) Berechnen der folgenden beschreibenden Parameter

- $\gamma_m(t) = \mathbf{f}_m(t)$ *m* = 1,2,3,4,5 und $\gamma_6(t) = \mathbf{A}(t)$: für $\mathbf{r}_i \in \mathfrak{R}_g$
- Ein Wert $I_{B \to A/C}$, der den gerichteten Einfluss einer Gehirn- oder Herzregion *B* auf eine Region *A,* die nicht aus der Aktivität aus einer Region *C* resultiert.

p) Festlegen von Referenzgruppen von Subjekten, für die beschreibende Parameter statistisch gesammelt worden sind und berechnen:

- der Wahrscheinlichkeiten $p(\gamma_m)$, dass die gemessenen beschreibenden Parameter, die in Schritt 1-o für das Testsubjekt erhalten worden sind, für eine gegebene Referenzgruppe typisch sind.

- der Wahrscheinlichkeiten $p(I^m_{A \to B/C})$, dass die gemessenen beschreibenden Parameter, die in Schritt 1-o für das Testsubjekt erhalten wurden, typisch für die gegebenen Referenzgruppen sind.

q) Codieren der Werte von $p(\gamma_m)$ und $p(I^m_{A \to B/C})$ mit Hilfe einer Farbskala und Anzeigen der überlagerten Codes auf dem ausgewählten anatomischen Bild, wobei statistisch signifikante Regionen hervorgehoben werden, indem diejenigen Werte unter einem ausgewählten Signifikanzniveau auf Null gesetzt werden, um eine statistische Parameterkarte für die Tomographie des PEC zu erhalten.

2. Verfahren nach Anspruch 1, wobei das gemeinsame Referenzsystem das Talairach Koordinatensystem ist.

3. Verfahren nach Anspruch 1, wobei der anatomische Atlas durch eine der folgenden Varianten erhalten wird:

a) durch die Extraktion von Gewebewahrscheinlichkeiten der anatomischen Bilder, die für das bestimmte zu untersuchende Subjekt durch eine der folgenden Typen erhalten wird: Berechnete Axiale Tomographie (Computed Axial Tomography) (CAT), Magnetische Resonanzabbildungen (Magnetic Resonance Images) (MRI), post-mortem-Sektionen des Kopfes, die durch Cryotomy usw. erhalten wurden, wobei der Atlas als ein individueller anatomischer Atlas bezeichnet wird.
b) durch eine starre oder elastische Transformation des Volumenleitergitters des Subjektes, um die bestmögliche Zuordnung zu einem kanonischen Volumenleitergitter zu gewährleisten, für die eine Gewebewahrscheinlichkeitsverteilung aus einem Muster eines normalen oder pathologischen anatomischen Bilds erhalten wurde, wobei der Atlas als ein Probabilistischer Anatomischer Atlas bezeichnet wird.

4.  Verfahren nach Anspruch 1, wobei die Berechnung der elektrischen und magnetischen Führungsfeldmatrizen durch die Vektorversionen der Finite Elemente Methode (FEM) oder Finite Differenzen Methode (FDM) für einen inhomogenen und anisotropischen Volumenleiter die folgenden Schritte umfasst:

a) Festlegen der Positionen der Sensoren für das Gitter der EEG/EKG und MEG/MKG Modalität als $\aleph_1$ bzw. $\aleph_2$, wie in Schritt 1-b definiert ist.

b) Festlegen der Positionen $\mathbf{r}_g$ und $\mathbf{r}_V$, in dem Gitter des erzeugenden Volumens $\Re_g$ bzw. des Volumenleiters $\Re_V$, wie in den Schritten 1-c und 1-f definiert.

c) Festlegen des Leitfähigkeitsprofils, wie in Schritt 1-g definiert, mit Hilfe einer Näherung, so dass jede Menge der Gewebebezeichnungen eine Sammlung von N eingebetteten Bereichen bildet, für die der Leitfähigkeitswert konstant ist, $\sigma = \{\sigma_1;...;\sigma_N\}$ .

d) Numerisches Berechnen der ohmschen Stromdichten $\mathbf{j}_{kV} = \mathbf{j}_k(\mathbf{r}_V)$ und $\mathbf{j}_{kV}^\omega = \mathbf{j}_k(\mathbf{r}_V,\omega)$ an jedem Punkt $\mathbf{r}_V$ des Gitters des Volumenleiters $\Re_V$, der zu den die Bereiche begrenzenden Oberflächen gehört, mit Hilfe der folgenden linearen algebraischen Gleichungssysteme: $\mathbf{Dc} = \mathbf{c}_\infty - \Gamma\mathbf{c}$; wobei $\mathbf{c} = (\mathbf{c}_1;...;\mathbf{c}_N)$; wobei das $\mathbf{c}_k = \{\mathbf{j}_{kV}, V =1,\cdots,N_{k,k+1}\}$ für das EEG/EKG oder $\mathbf{c}_k = \{\mathbf{j}^\omega kV, V =1,\cdots,N_{k,k+1}\}$ für das MEG/MKG; wobei $N_{k,k+1}$ der Nummernpunkt $\mathbf{r}_V$ ist, der zu der die Bereiche $k$ und $k+1$ trennenden Oberfläche gehört. Die Matrizen $\Gamma$ und $\mathbf{D}$ sind definiert als:

e)

$$\Gamma = \frac{1}{4\pi}\begin{pmatrix} \dfrac{\sigma_1 - \sigma_2}{\sigma_1}\Gamma_{11} & \dfrac{\sigma_2 - \sigma_3}{\sigma_2}\Gamma_{12} & \cdots & \Gamma_{1N} \\ \dfrac{\sigma_1 - \sigma_2}{\sigma_1}\Gamma_{21} & \dfrac{\sigma_2 - \sigma_3}{\sigma_2}\Gamma_{22} & \cdots & \Gamma_{2N} \\ \vdots & \vdots & \ddots & \vdots \\ \dfrac{\sigma_1 - \sigma_2}{\sigma_1}\Gamma_{N1} & \dfrac{\sigma_2 - \sigma_3}{\sigma_2}\Gamma_{N2} & \cdots & \Gamma_{NN} \end{pmatrix}$$

$$\mathbf{D} = \begin{pmatrix} \alpha_1\mathbf{I}_{N_{1,2}} & 0 & \cdots & 0 \\ 0 & 0 & & \vdots \\ \vdots & & \ddots & 0 \\ 0 & \cdots & 0 & \alpha_N\mathbf{I}_{N_{N,N}} \end{pmatrix}$$

f) wobei $\alpha_j = \dfrac{(2\sigma_j + \sigma_{j+1})}{3\sigma_j}$; $\Gamma_{jk} = \begin{pmatrix} \Gamma_1^k(\mathbf{r}_1^j) & \cdots & \Gamma_{N_{k,k+1}}^k(\mathbf{r}_1^j) \\ \vdots & \ddots & \vdots \\ \Gamma_1^k(\mathbf{r}_{N_{j,j+1}}^j) & \cdots & \Gamma_{N_{k,k+1}}^k(\mathbf{r}_{N_{j,j+1}}^j) \end{pmatrix}$; $\mathbf{r}_i^j$ bezeichnet den $i$-ten

Punkt von $\Re_V$, der zu der $j$-ten Oberfläche gehört; $\mathbf{c}_\infty = (\mathbf{c}_\infty^1;\cdots;\mathbf{c}_\infty^N)$, mit $\mathbf{c}_\infty^k = \{\mathbf{j}_{kv}^\infty, V = 1,\cdots,N_{k,k+1}\}$ für das EEG/EKG und $\mathbf{c}_\infty^k = \{\mathbf{j}_{kv}^{\omega\infty}, V = 1,\cdots,N_{k,k+1}\}$ für das MEG/MKG. Die

Amplituden $\mathbf{j}_{kV}^{\infty} = \mathbf{j}_{k\infty}(\mathbf{r}_V)$ und $\mathbf{j}_{kV}^{\omega\infty} = \mathbf{j}_{k\infty}(\mathbf{r}_V,\omega)$

werden aus den Ausdrücken ermittelt:

g) $\quad \mathbf{j}_{k\infty}(\mathbf{r}) = -\dfrac{I_{er}}{4\pi} \nabla \cdot (g(\mathbf{r},\mathbf{r}_e) - g(\mathbf{r},\mathbf{r}_r))$

h) $\quad \mathbf{j}_{k\infty}(\mathbf{r},\omega) = \dfrac{i\omega}{4\pi} \sum_{k=1}^{N} (\sigma_k - \sigma_{k+1}) \oint_{S_{k,k+1}} g(\mathbf{r},\mathbf{r'}) \cdot (\mathbf{n}_k(\mathbf{r'}) \times \mathbf{b}_c(\mathbf{r'},\omega)) d\mathbf{r'}^2$, wobei $\mathbf{b}_c(\mathbf{r'},\omega)$ dem magne-

tischen Feld entspricht, das durch den niederfrequenten Wechselstrom, der durch eine einzelne Spule in einem infiniten und homogenen Volumenleiter fließt, erzeugt wird.

i) Die Funktion $\Gamma_n^k(\mathbf{r}) = \int_{\Delta_n^k} \nabla' g(\mathbf{r},\mathbf{r'}) \cdot d\mathbf{r'}^2 - \int_{\Delta_n^k} \nabla' g(\mathbf{r},\mathbf{r'}) \circ d\mathbf{r'}^2$, wobei $g(\mathbf{r}_g,\mathbf{r'})$ der Green Funktion der infiniten Domäne entspricht.

j) Berechnen des elektrischen und magnetischen Führungsfeldes in jedem Kompartment eines IPHC mit Hilfe

des Reziprozitäts-Theorems: $\quad \mathbf{k}_{eg} = -\dfrac{\mathbf{j}_j(\mathbf{r}_g)}{\sigma_j I_{er}} \quad$ an der Führungselektrode "*er*" bzw.

$k_{cg} = -\dfrac{\mathbf{j}_j(\mathbf{r}_g,\omega)}{i\omega\sigma_j I_c(\omega)\Delta S_c} \quad$ an der Spule *"co"*. Die ohmschen Stromdichten werden an jedem Punkt $\mathbf{r}_g$

des Gitters des erzeugenden Volumens $\Re_g$ mit Hilfe der folgenden Ausdrücke ermittelt:

k) Elektrisches Führungsfeld: $\mathbf{j}_j(\mathbf{r}_g) = \mathbf{j}_{k\infty}(\mathbf{r}_g) - \dfrac{1}{4\pi} \sum_{k=1}^{N} \dfrac{(\sigma_k - \sigma_{k+1})}{\sigma_k} \sum_{V=1}^{N_{k,k+1}} \Gamma_V^k(\mathbf{r}_g) \mathbf{j}_{kV}$

l) Magnetisches Führungsfeld:

$$\mathbf{j}_j(\mathbf{r}_g,\omega) = \mathbf{j}_{k\infty}(\mathbf{r}_g,\omega) - \dfrac{1}{4\pi} \sum_{k=1}^{N} \dfrac{(\sigma_k - \sigma_{k+1})}{\sigma_k} \sum_{V=1}^{N_{k,k+1}} \Gamma_V^k(\mathbf{r}_g) \mathbf{j}_{kV}^{\omega}$$

m) Berechnung der elektrischen $\mathbf{K}_1 = \left\{ \mathbf{k}_{eg}^t \right\}$ und magnetischen $\mathbf{K}_2 = \left\{ \mathbf{k}_{cg}^t \right\}$ Führungsfeldmatrizen, die eine Reihe besagter Matrizen für jeden Sensor (EEG/EKG $e = 1,..., N_e$ und MEG/MKG $c = 1,..., N_c$) und eine Spalte für jeden Punkt des Gitters des erzeugenden Volumens festlegen $\mathbf{r}_g \in \Re_g$.

5. Verfahren nach Anspruch 1, wobei die Berechnung des Kerneloperators $K_m$ mit der räumlichen und zeitlichen Auflösung, die der m-ten Bildmodalität (i = 3, 4, 5) zugeordnet ist, festgelegt wird.

6. Verfahren nach Anspruch 1, wobei die Schätzung des PEC $f_1(t)$ für ein gegebenes Subjekt die Schritte umfasst:

a) Zuordnen von Anfangswerten zu den folgenden Mengen von Variablen:

1. Gewebeaktivierung $\mathbf{A}(t) = [\mathbf{A}(\mathbf{r}_g,t)]_{\mathbf{r}_R \in \Re_g}$ für jeden Punkt $\mathbf{r}_g$ von $\Re_g$

2. Funktionelle Indikatoren $\mathbf{f}_m(t) = [\mathbf{f}_m(\mathbf{r}_g,t)]$ für jeden Punkt $\mathbf{r}_g$ von $\Re_g$, wobei $\mathbf{f}(t) = [\mathbf{f}_m(t)]_{1 \leq m \leq M}$ die Gesamtheit der Messmodalitäten, die für ein Subjekt erhalten wird, bezeichnet.

3. Hyperparameter der Aktivierung $\Theta_A$,

4. Hyperparameter des intrinsischen Rauschens der funktionellen Indikatoren $\Theta_\beta = [\Theta_{\beta_m}]_{1 \leq m \leq M}$

5. Hyperparameter des instrumentellen Rauschens $\Theta_c = [\Theta_{c_m}]_{1 \leq m \leq M}$

b) Berechnung der a Posteriori-Wahrscheinlichkeit:

$$P\Big(\mathbf{A}(t), \Theta_A, \mathbf{f}(t), \Theta_\beta, \Theta_c \Big| \mathbf{o}(t)\Big) \propto$$

$$\Pi_{m=1}^M l_{\mathbf{o}_m}(\mathbf{o}_m(t) - \mathbf{K}_m * \mathbf{f}_m(t) \mid \Theta_{c_m}) \times$$

$$\Pi_{m=1}^M \pi_{\mathbf{f}_m}(\mathbf{f}_m(t) - \mathbf{H}_m \circ \mathbf{A}(t) \mid \Theta_{\beta_m}) \times$$

$$\Pi_{m=1}^M \Big\{\pi_{\Theta_{cm}}(\Theta_{c_m}) \cdot \pi_{\Theta_{\beta_m}}(\Theta_{\beta_m})\Big\} \times$$

$$\pi_A(\mathbf{A}(t) \mid \Theta_A) \times \pi_{\Theta_A}),$$

die die folgende multiplikativen Ausdrücke umfasst:

> 1. Wahrscheinlichkeitsterme $l_{o_m}$ für jede Modalität m $l_{o_m}(\mathbf{o}_m(t)-\mathbf{K}_m*\mathbf{f}_m(t) \mid \Theta_{\mathbf{c}m})$;
> 2. die a priori Wahrscheinlichkeiten der funktionellen Indikatoren $\pi_{fm}(\mathbf{f}_m(t)-\mathbf{H}_m \circ \mathbf{A}(t) \mid \Theta_{\beta_m})$;
> 3. die a priori Wahrscheinlichkeiten für die Aktivierung $\pi_A(\mathbf{A}(t) \mid \Theta_A)$
> 4. die a priori Wahrscheinlichkeit für die Hyperparameter der Aktivierung $\pi_{\Theta_A}(\Theta_A)$;
>
> 5. die a priori Wahrscheinlichkeiten für die Hyperparameter des intrinsischen Rauschens der funktionellen
>
> Indikatoren $\pi_{\Theta_{\beta_m}}(\Theta_{\beta_m})$;
>
> 6. die a priori Wahrscheinlichkeiten für die Hyperparameter des instrumentellen Rauschens $\pi_{\Theta_{c_m}}(\Theta_{c_m})$;

c) Iteratives Modifizieren der Parameter $\mathbf{A}(t)$, $\Theta_A$, $\mathbf{f}(t)$, $\Theta_\beta$, $\Theta_c$, bis ein Maximalwert der a posteriori Wahrscheinlichkeit erreicht ist, wobei die Modifikation gemäß einem der folgenden Schemata ausgeführt wird:

> 1. Aufeinanderfolgende Maximierung von $P(\mathbf{A}(t), \Theta_A, \mathbf{f}(t), \Theta_\beta, \Theta_c | \mathbf{o}(t))$ für Untermengen der Parameter, während die anderen auf ihrem Wert festgehalten werden, "Iterierte konditionelle Maximierung" (Iterated Conditional Maximization) (ICM)
> 2. Aufeinanderfolgende Maximierung von $P(A(t), \Theta_A, \mathbf{f}(t), \Theta_\beta, \Theta_c | \mathbf{o}(t))$ für die Untermenge der Parameter, wobei die anderen Parameter auf ihrem Erwartungswert gehalten werden "Erwartungswert-Maximierung" (Expected Maximization) (EM)
> 3. Wählen des Verteilungsmodus von $P(\mathbf{A}(t), \Theta_A, \mathbf{f}(t), \Theta_\beta, \Theta_c | \mathbf{o}(t))$, der mit Hilfe des Monte Carlo Markov-Ketten-Verfahrens (MCMC) erhalten wird.

**7.** Verfahren nach Anspruch 6, wobei die elektrophysiologischen Messungen $\mathbf{o}_m(t)$ m= 1, 2 für die Abschätzung von $\mathbf{f}_1(t)$ eine der folgenden Kombinationen umfasst: EEG alleine, MEG alleine, EEG und MEG gemeinsam.

**8.** Verfahren nach Anspruch 6, wobei entweder

> • keines der funktionellen Bilder $\mathbf{o}_m(t)$ m = 3, 4, 5 für die Abschätzung von $\mathbf{f}_1(t)$ verwendet wird oder alternativ
> • eine oder mehrere dieser Messungen in einer der folgenden Kombinationen verwendet werden: fMRI alleine, PET alleine, SPECT alleine, fMRI und SPECT, fMRI und SPECT, PET und SPECT.

**9.** Verfahren nach Anspruch 6, wobei die Berechnung der Wahrscheinlichkeiten $l_{o_m}(\mathbf{o}_m(t) - \mathbf{K}_m * \mathbf{f}_m(t) | \Theta_{e_m})$ die folgenden Schritte umfasst:

a) Wählen der Verwendung von entweder ε unempfindlichen polynomischen Risiken oder eines verallgemeinerten polynomischen Risikos $R_{\mathbf{x}}^C[\mathbf{x}|\Theta_{\mathbf{x}}]$ für den bestimmten Zweck des Bewertens von nicht Gaußverteilten Wahrscheinlichkeiten.
b) Berechnen des Diskrepanzvektors $\Delta = (\mathbf{o}_m(t)-\mathbf{K}_m{}^*\mathbf{f}_m(t))$
c) Umskalieren von $\Delta$ als eine Funktion der Hyperparameter $\Theta_{c_m}$

d) Berechnen von $R_{\mathbf{x}}^{C}\left[\Delta\middle|\Theta_{\mathbf{x}}\right]$ , dem Risiko von $\Delta$

e) Berechnen von $l_{0_m}\left(\Delta\middle|\Theta_{c_m}\right) = \exp\left(- B\ R_{\mathbf{x}}^{C}\left[\Delta\middle|\Theta_{\mathbf{X}}\right]\right)$, wobei $B$ eine vorgewählte Konstante ist.

**10.** Verfahren nach Anspruch 6, wobei die Berechnung der a priori Wahrscheinlichkeit $\pi_{r_m}(\mathbf{f}_m(t)\text{-}\mathbf{H}_m \circ \mathbf{A}(t)|\Theta_{\beta_m})$ für die funktionellen Indikatoren die folgenden Schritte umfasst:

a) Auswählen der Indizes $m, n, s$ des Besov-Raumes $B_{n,s}^{m}$ zum Zweck des Definierens der Glattheit des abgeschätzten funktionellen Indikators, wobei eine bestimmte aber nicht ausschließliche Wahl $m$=1, $n$=1, $s$=1 beträgt, die die "Algebra von Erhebungen" (algebra of bumps) definiert.
b) Berechnung des Diskrepanzvektors $\Delta = \mathbf{f}_m(t)$ - $\mathbf{H}_m$ o A(t)
c) Umskalieren des $\Delta$ als einer Funktion der Hyperparameter $\Theta_{\beta_m}$

d) Berechnen von $\left\|\Delta\right\|_{B_{n,s}^{m}}$ , die Norm von $\Delta$ in $B_{n,s}^{m}$ , wobei die Berechnung durch die erste Expansion des $\Delta$ als $\Delta = \Sigma_k F_k \cdot \Psi_k$ ausgeführt wird, wobei das $\Psi_k$ zu einem Verzeichnis von Atomen gehört, (dessen bestimmte jedoch nicht erschöpfende Beispiele dem Wavelet-Verzeichnis, dem Fourier-Verzeichnis, dem Dirac-delta-Verzeichnis oder einem Mega-Verzeichnis, das durch die Gesamtheit von mehreren Verzeichnissen definiert ist, entspricht) und dann Berechnung von $\left\|\Delta\right\|_{B_{n,s}^{m}} \approx \sum_{k} \alpha_k \cdot \left\|F_k\right\|^{m}$

e) Berechnung von $\pi_{f_m}\left(\Delta\middle|\Theta_{\beta_m}\right) = \exp\left(- C\ \left\|\Delta\right\|_{B_{n,s}^{m}}\right)$ , wobei C eine vorgewählte Konstante ist.

**11.** Verfahren nach Anspruch 6, wobei die Berechnung der a priori Wahrscheinlichkeit der Aktivierung $\pi_A(\mathbf{A}(t)|\Theta_A)$ die folgenden Schritte umfasst:

a) Berechnen einer nicht abnehmenden Funktion $h(\ )$ des probabilistischen Atlasses $h(p_g)$

b) Berechnen der Norm $\left\|\mathbf{A}(t)\right\|_{B_{n,s}^{m}}$ wie in Anspruch 10 angegeben ist

c) Berechnung von $\pi_A\left(\mathbf{A}(t)\middle|\Theta_A\right) = \exp\left(- D\ h\left(p(s,\mathbf{r}_g)\right) - E\left\|\mathbf{A}(t)\right\|_{B_{n,s}^{m}}\right)$ , wobei D und E vorbestimmte Konstanten sind.

**12.** Verfahren nach Anspruch 6, wobei die a priori Wahrscheinlichkeit für den PEC die folgende bestimmte Form annimmt:

$$\pi\left(\mathbf{f}_1(t) - \mathbf{H}_1 \circ \mathbf{A}(t)\middle|\Theta_{\beta_1}\right) = \pi_1\left(\mathbf{f}_1(t) - \mathbf{M}\ \mathbf{A}(t)\middle|\Theta_1\right)\pi_2\left(\mu\middle|\Theta_2\right)$$

wobei $\pi_1$ und $\pi_2$ den a priori Wahrscheinlichkeiten entsprechen, die in Anspruch 10 spezifiziert sind,

$$\mathbf{M} = \begin{bmatrix} \mu_1 & 0 & \cdots & 0 & 0 \\ 0 & \mu_2 & \cdots & 0 & 0 \\ \cdots & \cdots & \mu_i & \cdots & \cdots \\ 0 & 0 & \cdots & \mu_{N_g-1} & 0 \\ 0 & 0 & \cdots & 0 & \mu_{N_g} \end{bmatrix}, \ \mu = \begin{bmatrix} \mu_1 \\ \mu_2 \\ \cdots \\ \mu_{N_g} \end{bmatrix}$$

wobei $\mu_g$ den Orientierungen der PEC für jeden Punkt von $\Re_g$ entspricht.

**13.** Verfahren nach Anspruch 6, wobei die a priori Wahrscheinlichkeit $\pi_2(\mu|\Theta_2)$, die in Anspruch 12 definiert ist, anhand einer der folgenden Ausdrücke berechnet wird:

- $\mu_2\left(\mu|\Theta_2\right) = \exp\left(-\left\|\Lambda_s \cdot \mu \cdot \Lambda_m\right\|_{B_{n,s}^m}\right)$, wobei $\Lambda_s$ ·einer diagonalen Matrix entspricht, die den Grad der Glattheit spezifiziert, der an jedem Punkt von $\Re_g$ und $\Lambda_m = \mathbf{W} \cdot \mathbf{P}$ vorliegt, wobei W eine vorspezifizierte Gewichtungsmatrix und P eine diagonale Matrix ist, die $p_g$ für alle $\mathbf{r}_g$ enthält.

- Mit Hilfe der nacheinander folgenden Schätzung der Randverteilungen von

$$\pi_2\left(\mu_g|\Theta_2\right) = p_g \cdot N\left(0, \sigma_A^2\right) + \left(1 - p_g\right) \cdot N\left(0, \sigma_B^2\right),$$ wobei $N(0,\sigma^2)$ der eindimensionalen Gaußverteilung mit dem Mittelwert Null entspricht, und die Standardabweichung $\sigma$ ., $\sigma_A^2$ und $\sigma_B^2$, Konstanten entsprechen, die groß bzw. klein mit Bezug auf die erwartete Variation der Orientierungen gewählt sind.

**14.** Verfahren nach Anspruch 6, wobei die Berechnungen für $\mathbf{o}_1(t)$ und/oder $\mathbf{o}_2(t)$ in der Frequenzdomäne ausgeführt werden, indem festgelegt ist:

- dass mit den elektrischen Signalen die Fourier-Transformation ausgeführt wurde, wobei t nun die Frequenz angibt.

- $l_{\mathbf{o}_m}\left(\mathbf{o}_m(t) - \mathbf{K}_m * \mathbf{f}_m(t)|\Theta_{e_m}\right) = \mathbf{N}_R^{2,p}\left(0, \Sigma_e\right)$ wobei $\mathbf{N}_R^{2,p}\left(\mu_x, \Sigma_x\right)$ die mehrdimensionale reale Gaußverteilung mit dem Mittelwert und der Varianz $(\mu_x, \Sigma_x)$ angibt.

- $\pi_{\mathbf{f}_m}\left(\mathbf{f}_m(t) - \mathbf{H}_m \circ \mathbf{A}(t)|\Theta_{\beta_m}\right) = \mathbf{N}_R^{2,p}\left(0,0\right), \mathbf{H}_m = \mathbf{I}$, die Identitätsmatrix

- $\pi_{\mathbf{A}}\left(\mathbf{A}(t)|\Theta_{\mathbf{A}}\right) = \mathbf{N}_R^{2,p}\left(0, \Sigma_a\right)$

- $\pi_{\Theta_{\mathbf{A}}}\left(\Theta_{\mathbf{A}}\right) = \left\|\Sigma_{\mathbf{A}}\right\|^{-\alpha/2} \exp\left(-\tfrac{1}{2} Tr\left(\mathbf{Q}\,\Sigma_{\mathbf{A}}^{-1}\right)\right)$ ein natürliches Konjugat einer a priori Verteilung für komplexe Wishart-Matrizen ist, wobei $\alpha$ und $\mathbf{Q}$ gemäß einem Vorwissen festgelegt sind.

- $\pi_{\Theta_{\beta_m}}\left(\Theta_{\beta_m}\right)$ und $\pi_{\Theta_{e_m}}\left(\Theta_{e_m}\right)$ Konstanten sind, die ungenaue Vorgänger definieren.

**15.** Verfahren nach Anspruch 1, wobei die Berechnung des Elementes $I_{B \to A/C}$, das den direkten Einfluss des Gehirn- oder Herzbereiches *B* auf den Bereich A quantifiziert, die nicht von der von dem Bereich C stammenden Aktivität abhängt, die folgenden Schritte umfasst:

a) Definieren der Bereiche *A,B,C* als Mengen von festgelegten Voxel $\mathbf{r}_i \in \Re_g$, die aus dem erzeugenden Volumen gewählt sind.

b) Definieren für jede gegebene Bildmodalität m die Matrix von Vorgängern als $\Gamma_t^q = \left[\gamma_m(t-k)\right]_{1\leq k\leq q}$.

c) Berechnen der Reste $\varepsilon\left(\Gamma_t^q,t\right)$ eines autoregressiven Modells über jedes vorgegebene Gehirn durch An-

passung des Modells $\gamma_t = \mathbf{F}\left(\Gamma_t^q,t\right)+\varepsilon\left(\Gamma_t^q,t\right)$ mit Hilfe einer nicht parametrischen Funktion F und mit Hilfe

des Ausdrucks $\varepsilon\left(\Gamma_t^q,t\right) = \mathbf{F}\left(\Gamma_t^q\right)-\gamma_t$

d) Berechnen der Matrix $\sum_{\gamma}\left(\Gamma_t^q,t;A|B.C\right)$ als die Kovarianz der Abweichungen des nicht parametrischen autoregressiven Modells über der Vereiningungsmenge der Bereiche A,B,C.

e) Berechnen der Matrix $\sum_{\gamma}\left(\Gamma_t^q,t;A|B.C\right)$ als die Kovarianz der Abweichungen des nicht parametrischen autoregressiven Modells über der Vereiningungsmenge der Bereiche A und C, ausgenommen B.

f) Berechnen des Einflusses des Bereiches A auf B, wenn der Einfluss von Bereich C als Ausdruck:

$$I_{\mathbf{B}\rightarrow\text{A/C}} = \ln\left(\frac{\left|\sum_{\gamma}\left(\Gamma_t^q,t;A|\{\ \}.C\right)\right|}{\left|\sum_{\gamma}\left(\Gamma_t^q,t;A|B.C\right)\right|}\right),$$ da das Logarithmenverhältnis der Determinanten der Matrizen

$\sum_{\gamma}\left(\Gamma_t^q,t;A|B.C\right)$ und $\sum_{\gamma}\left(\Gamma_t^q,t;A\{\ \}.C\right)$ entspricht.

**16.** Verfahren nach Anspruch 1, wobei die betroffenen Berechnungen die folgenden Schritte umfassen:

a) Bestimmen der Referenzgruppen der Subjekte, für die die beschreibenden Parameter $\gamma_m(t)$ erhalten worden sind und für die die bestimmten Kovariate gemessen worden sind. Die Kovariate (wobei ein bestimmtes jedoch nicht ausschließendes Beispiel das Alter ist) werden durch $\varpi$ bezeichnet.
b) Transformation in Richtung der Gaußianität der beschreibenden Parameter von sowohl dem Testsubjekt als auch den Subjekten in der Referenzgruppe mit Hilfe des Ausdrucks: $\gamma_m'(t) = T(\gamma_m(t))$, wobei die beschrei-

benden Parameter $\gamma_m(t)$ durch eine nichtlineare Funktion T transformiert werden, um den Vektor $\gamma_m'(t)$ zu

erhalten.

c) Berechnen des Mittelwertes $\mu\left(\gamma_m';\varpi\right)$ und der Varianz $\sigma\left(\gamma_m';\varpi\right)$ von jedem der beschreibenden Parameter als Funktion des Steuerparameters $\varpi$ in jeder Bezugsgruppe mit Hilfe einer nicht parametrischen Regression;

d) Berechnung der z Transformation von jedem beschreibenden Parameter mit Hilfe des Ausdrucks:

$$z = \left(\frac{\gamma_m' - \mu\left(\gamma_m';\varpi\right)}{\sigma\left(\gamma_m';\varpi\right)}\right),$$ wobei jede Größe an jedem Punkt des erzeugenden Gitters $\mathfrak{R}_g$ ermittelt wird;

e) Berechnung der Wahrscheinlichkeiten, dass $z_{max}$ und $z_{min}$, der Maximal- bzw. der Minimalwert von z in dem Interessensbereich C, Schwellwerte $U_{max}$, $U_{min}$ übersteigen, wobei $U_{max}$, $U_{min}$ Schwellwerte sind, die gemäß einem Vorwissen gewählt sind;
f) Berechnung der folgenden transformierten Größen:

$$\begin{cases} z' = F^{-1}\big(P(z)\big) \; wenn\, z \ge U_{\max} \\ \quad z' = 0 \qquad\qquad wenn\, U_{\min} < z < U_{\max} \\ z' = F^{-1}\big(P(z)\big) \; \; wenn \; z \le U_{\min} \end{cases}$$

wobei $F^{-1}$ die inverse Gaußfunktion ist;

g) Anzeigen von z Kurven, die als dimensionale Graphen von z' definiert sind, und die für einen festgelegten Punkt des erzeugenden Gitters $\mathfrak{R}_g$ und als eine Funktion von entweder der Zeit oder der Frequenz aufgezeichnet werden; was im letztgenannten Fall ein z Spektrum der Tomographie des PEC als bezeichnet wird.

- Anzeigen der z' Werte als das zwei- oder dreidimensionale Bild für eine feste Zeit oder Frequenz, wobei das Bild als z Bild der Tomographie des PEC bezeichnet wird, wobei das Bild gemäß einem Farbcode gerendert wird, der den Grad der Abweichung der Werte von den Referenzgruppen angibt, wobei die Bild einem anatomischen Atlas überlagert wird, der den Hintergrund des Bilds bildet.

**Revendications**

1. Procédé d'obtention d'une Tomographie du Courant Electrique Primaire (CEP) à partir des signaux provenant du cerveau ou bien du coeur, par l'intermédiaire d'un réseau de capteurs électriques et biomagnétiques externes et d'un ensemble d'images anatomiques et médicales, le procédé comprenant les étapes qui consistent à :

a) mesurer les signaux électriques et/ou magnétiques d'un sujet examiné à partir du réseau de capteurs, définir des observations pour un instant commun t, désigner respectivement l'EEG/ECG par $o_1(t)$ et le MEG/MCG par $o_2(t)$, obtenir en résultat une série de vecteurs temps définie sur l'intervalle de temps $\mathfrak{I}$ = [0, $T$] ;

b) spécifier les coordonnées des capteurs du réseau de capteurs pour le sujet examiné par rapport au système de référence de la tête ou du torse, ledit ensemble de coordonnées étant respectivement désigné $\aleph_1$ et $\aleph_2$ pour l'EEG/ECG et pour le MEG/MCG ;

c) sélectionner une image anatomique, soit une image du sujet examiné, soit une image de référence appropriée, et spécifier les coordonnées cartésiennes, dans le système de référence commun, de ses voxels constitutifs en tant que treillis du conducteur volumique $\mathfrak{R}_V$ ;

d) spécifier pour chaque point $r_v$ de $\mathfrak{R}_V$ un type de tissu donné "s", en définissant ainsi un Atlas Anatomique ;

e) étiqueter chaque point $r_v$ du treillis du conducteur volumique avec le type de tissu ;

f) spécifier pour chaque point $r_v$ du treillis du conducteur volumique $\mathfrak{R}_V$ la valeur de conductivité correspondant à l'étiquette du tissu $s_v$, lesdites valeurs étant prises dans un ensemble prédéfini de conductivités, désigné comme le profil de conductivité $\sigma$;

g) sélectionner les points du $R_v$ de génération afin de défmir l'ensemble $\mathfrak{R}_g$ de $N_g$ avec les points $r_g$, le volume de génération ;

h) calculer les matrices de champ électrique évoqué $K_1$ et de champ magnétique évoqué $K_2$, par les versions vectorielles de la Méthode des Eléments Finis (MEF) ou la Méthode des Différences Finies (MDF), ce pour un conducteur volumique non homogène et anisotrope, de telle sorte que, avec des $\sigma$, $\aleph_1$, $\aleph_2$, $\mathfrak{R}_g$ et $\mathfrak{R}_V$ donnés, les équations suivantes s'appliquent : $o_1(t) = K_1\, f_1(t)$, $o_2(t) = K_2\, f_2(t)$, où $f_1(t)$ et $f_2(t)$ sont utilisés de manière interchangeable pour désigner le CEP ;

i) sélectionner, lorsqu'elles sont disponibles, une ou plusieurs des images des modalités fonctionnelles suivantes : la résonance magnétique fonctionnelle (IRMf), la tomographie par émission de positrons (TEP) et la tomographie par émission de photon unique (TEPU), en définissant les observations respectives $o_3(t)$, $o_4(t)$ et $o_5(t)$ comme étant celles du sujet examiné ou d'une certaine image de référence appropriée ;

j) spécifier les positions des voxels en tant qu'ensemble de coordonnées cartésiennes, dans le système de référence commun, pour chaque image fonctionnelle sélectionnée, ledit ensemble de position étant respecti-

vement désigné $\aleph_3$, $\aleph_4$, et $\aleph_5$ pour les modalités IRMf, TEP et TEPU ;

k) spécifier les instants auxquels chaque image fonctionnelle sélectionnée m a été échantillonnée, dans une échelle temporelle commune avec les modalités respectives IRMf, TEP et TEPU ;

l) calculer le noyau $K_m$ qui exprime notre connaissance du fait que l'indicateur fonctionnel idéal $f_m(t)$, qui est associé à la m$^{ième}$ modalité d'imagerie (m = 3, 4, 5), défini sur $\mathfrak{R}_V$ et pour les instants échantillonnés, est modifié par le processus de formation d'image selon la transformation $o_m(t) = K_m * f_m(t)$, qui

- réduit la résolution spatiale de $f_m(t)$ de celle de $\mathfrak{R}_V$ à celle de $\aleph_m$,
- réduit la résolution temporelle de $f_m(t)$ de celle des données électrophysiologiques (m = 1) à celle de l'une quelconque des autres modalités m ;

m) calculer l'opérateur physiologique $H_m$ qui exprime notre connaissance du fait que l'activation des tissus neuronaux ou cardiaques A(t) qui est définie sur $\mathfrak{R}_g$ et $\mathfrak{I}_m$ produit des processus physiologiques associés à la m$^{ième}$ modalité d'imagerie (m = 1, 2, 3, 4, 5) selon la transformation $f_m(t) = H_m \circ A(t)$ ;

n) calculer des estimations du CEP par estimation conjointe de tous les $f_m(t)$ et A(t) pour toutes les m observées, ladite estimation comprenant les étapes qui consistent à :

i. attribuer des valeurs initiales arbitraires aux $f_m(t)$ et A(t) ;
ii. modifier de manière itérative les valeurs de $f_m(t)$ et A(t) ;
iii. calculer une mesure de probabilité $p$ qui augmente lorsque les valeurs de $f_m(t)$ et A(t), simultanément :

- permettent une reconstruction de $o_m(t)$ avec une petite erreur telle que quantifiée par une fonction de risque ; et
- ont une petite norme dans un espace de Besov donné ;

iv. continuer l'étape ii jusqu'à ce que la mesure de $p$ n'augmente plus ;

o) calculer les paramètres descriptifs suivants :

- $\gamma_m(t) = f_m(t)$, m = 1, 2, 3, 4, 5 et $\gamma_6(t) = A(t)$): pour $\mathbf{r_i} \in \mathfrak{R}_\mathbf{g}$ ;
- une valeur $\mathbf{I}_{B \to A/C}$ qui quantifie l'influence dirigée de la région B du cerveau ou du coeur sur la région A, qui n'est pas due à l'activité provenant de la région C ;

p) spécifier des groupes de référence de sujets pour lesquels les paramètres descriptifs ont été statistiquement résumés, et calculer :

- les probabilités $p(\gamma_m)$ que les paramètres descriptifs mesurés obtenus dans l'étape o pour le sujet examiné soient typiques d'un groupe de référence donné ;

- les probabilités $p(\mathrm{I}^\mathbf{m}_{\mathbf{A} \to \mathbf{B/C}})$ que les paramètres descriptifs mesurés obtenus dans l'étape o pour le sujet examiné soient typiques d'un groupe de référence donné ;

q) coder les valeurs de $p(\gamma_m)$ et $p(\mathrm{I}^\mathbf{m}_{\mathbf{A} \to \mathbf{B/C}})$ au moyen d'une échelle de couleurs, et afficher lesdits codes en surimpression sur l'image anatomique sélectionnée, en faisant ressortir les régions statistiquement significatives par le fait de définir à un seuil égal à zéro les valeurs qui sont inférieures à un niveau de signification choisi, pour obtenir une carte paramétrique statistique pour la tomographie du CEP.

**2.** Procédé selon la revendication 1, dans lequel le système de référence commun est le système de coordonnées de Talairach.

**3.** Procédé selon la revendication 1, dans lequel l'atlas anatomique est obtenu selon l'une des variantes suivantes :

a) par l'extraction des probabilités des tissus depuis des images anatomiques obtenues pour le sujet spécifique étudié, ces images étant de l'un quelconque des types suivants : une tomodensitométrie (TDM), des images de résonance magnétique (IRM), des sections post-mortem de la tête obtenues par cryotomie, etc., ledit atlas étant appelé "Atlas Anatomique Individuel" ;

b) par une transformation rigide ou élastique du treillis du conducteur volumique du sujet, afin de garantir la meilleure correspondance possible avec un treillis d'un conducteur volumique canonique, pour lequel une distribution des probabilités des tissus a été obtenue à partir d'un échantillon d'images anatomiques normales ou pathologiques, ledit atlas étant appelé "Atlas Anatomique Probabiliste".

**4.** Procédé selon la revendication 1, dans lequel le calcul des matrices de champ électrique évoqué et de champ magnétique évoqué par les versions vectorielles de la Méthode des Eléments Finis (MEF) ou la Méthode des Différences Finies (MDF), pour un conducteur volumique non homogène et anisotrope, comprennent les étapes qui consistent à :

a) spécifier les positions des capteurs pour le treillis des modalités EEG/ECG et MEG/MCG, respectivement $\aleph_1$ et $\aleph_2$, telles que définies dans l'étape b de la revendication 1 ;

b) spécifier les positions $r_g$ et $r_v$ dans le treillis du volume de génération $\mathfrak{R}_g$ et du conducteur volumique $\mathfrak{R}_V$, telles que définies respectivement dans les étapes c et f de la revendication 1 ;

c) spécifier le profil de conductivité, tel que défini dans l'étape g de la revendication 1, au moyen d'une approximation, de telle sorte que chaque ensemble d'étiquettes de tissus constitue une collection de régions incluses N pour lesquelles la valeur de conductivité est constante, $\sigma = \{\sigma_1, ..., \sigma_N\}$ ;

d) calculer numériquement les densités de courants ohmiques $j_{kV} = j_k (r_v)$ et $j_{kV}^\omega = j_k (r_V, \omega)$ sur chaque point $r_v$ du treillis du conducteur volumique $\mathfrak{R}_V$ qui appartient aux surfaces délimitant les régions, au moyen des systèmes d'équations algébriques linéaires suivants : $Dc = c_\infty - \Gamma c$, où $c = (c_1, ..., c_N)$ ; $c_k = \{j_{kV}, V = 1, ..., N_{k,k+1}\}$ pour l'EEC/ECG ou $c_k = \{j_{kV}^\omega, V = 1, ..., N_{k,k+1}\}$ pour le MEG/MCG ; $N_{k,k+1}$ est le nombre de points $r_v$ qui appartiennent à la surface séparant les régions k et k+1 ; les matrices $\Gamma$ et D étant définies par :

e)

$$\Gamma = \frac{1}{4\pi}\begin{pmatrix} \dfrac{(\sigma_1 - \sigma_2)}{\sigma_1}\Gamma_{11} & \dfrac{(\sigma_2 - \sigma_3)}{\sigma_2}\Gamma_{12} & \cdots & \Gamma_{1N} \\[2mm] \dfrac{(\sigma_1 - \sigma_2)}{\sigma_1}\Gamma_{21} & \dfrac{(\sigma_2 - \sigma_3)}{\sigma_2}\Gamma_{22} & \cdots & \Gamma_{2N} \\ \vdots & \vdots & \ddots & \vdots \\ \dfrac{(\sigma_1 - \sigma_2)}{\sigma_1}\Gamma_{N1} & \dfrac{(\sigma_2 - \sigma_3)}{\sigma_2}\Gamma_{N2} & \cdots & \Gamma_{NN} \end{pmatrix}$$

$$D = \begin{pmatrix} \alpha_1 I_{N_{1,2}} & 0 & \cdots & 0 \\ 0 & 0 & & \vdots \\ \vdots & & \ddots & 0 \\ 0 & \cdots & 0 & \alpha_N I_{N_{N,N}} \end{pmatrix} ;$$

f) où $\alpha_j = \dfrac{(2\sigma_j + \sigma_{j+1})}{3\sigma_j}$ ; $\Gamma_{jk} = \begin{pmatrix} \Gamma_1^k(r_1^j) & \cdots & \Gamma_{N_{k,k+1}}^k(r_1^j) \\ \vdots & \ddots & \vdots \\ \Gamma_1^k(r_{N_{j,j+1}}^j) & \cdots & \Gamma_{N_{k,k+1}}^k(r_{N_{j,j+1}}^j) \end{pmatrix}$ ; $r_i^j$ étiquette le $i^{ème}$ point de $\mathfrak{R}_V$

qui appartient à la j$^{\text{ième}}$ surface ; $c_\infty = \left(c_\infty^1;\ldots;c_\infty^N\right)$, avec $c_\infty^k = \{\,j_{kV}^\infty,\ V = 1,\ \ldots,\ N_{k,k+1}\}$ pour l'EEG/ECG et $c_\infty^k = \{\,j_{kV}^{\infty\infty},\ V = 1,\ \ldots,\ N_{k,k+1}\}$ pour le MEG/MCG ; les valeurs de $j_{kV}^\infty = j_{k\infty}(r_V)$ et

$j_{kV}^{\infty\infty} = j_{k\infty}(r_V, \omega)$ sont évaluées à partir des expressions :

g) $\quad j_{k\infty}(r) = -\dfrac{I_{er}}{4\pi}\nabla\cdot(g(r,r_e) - g(r,r_r))$

h) $\quad j_{k\infty}(r,\omega) = \dfrac{i\omega}{4\pi}\sum\limits_{k=1}^{N}(\sigma_k - \sigma_{k+1})\int\limits_{S_{k,k+1}}(g(r,r')\cdot(n_k(r')\times b_c(r',\omega))dr'^2$ , où $b_c(r', \omega)$ est le champ

magnétique généré par le courant alternatif basse fréquence passant à travers une seule spire, dans un conducteur volumique infini et homogène ;

i) la fonction $\quad \Gamma_n^k(r) = \int\limits_{\Delta_n^k}\nabla' g(r,r')\cdot dr'^2 - \int\limits_{\Delta_n^k}\nabla' g(r,r')\circ dr'^2$ , où $g(r_g,\ r')$ est la Fonction de Green du domaine infini ;

j) calculer le champ électrique évoqué et le champ magnétique évoqué dans chaque compartiment d'un IPHC au moyen des théorèmes de réciprocité : $\quad k_{eg} = -\dfrac{j_j(r_g)}{\sigma_j I_{er}}\quad$ au niveau de l'électrode "er" ; et

$k_{cg} = -\dfrac{j_j(r_g,\omega)}{i\omega\sigma_j I_c(\omega)\Delta S_c}\quad$ au niveau de la spire "co" ; les densités de courant ohmiques étant évaluées en tout point $r_g$ du treillis du volume de génération $\Re_g$ au moyen des expressions suivantes :

k) le champ électrique évoqué :

$$j_j(r_g) = j_{k\infty}(r_g) - \frac{1}{4\pi}\sum_{k=1}^{N}\frac{(\sigma_k - \sigma_{k+1})}{\sigma_k}\sum_{V=1}^{N_{k,k+1}}\Gamma_V^k(r_g)j_{kV}$$

l) le champ magnétique évoqué :

$$j_j(r_g,\omega) = j_{k\infty}(r_g,\omega) - \frac{1}{4\pi}\sum_{k=1}^{N}\frac{(\sigma_k - \sigma_{k+1})}{\sigma_k}\sum_{V=1}^{N_{k,k+1}}\Gamma_V^k(r_g)j_{kV}^\infty$$

m) calculer les matrices de champ électrique évoqué $K_1 = \{K'_{eg}\}$ et de champ magnétique évoqué

$K_2 = \{K'_{cg}\}$, en spécifiant une ligne desdites matrices pour chaque capteur (EEG/ECG e = 1, ..., $N_e$ et

MEG/MCG c = 1, ..., $N_c$) et une colonne pour chaque point du treillis du volume de génération $r_g \in \Re_g$.

**5.** Procédé selon la revendication 1, dans lequel le calcul de l'opérateur de noyau $K_m$ est spécifié comme étant dans la résolution spatiale et temporelle associée à la m$^{\text{ième}}$ modalité d'imagerie (i = 3, 4, 5).

**6.** Procédé selon la revendication 1, dans lequel l'estimation du CEP $f_1(t)$ pour un sujet donné comprend les étapes qui consistent à :

   a) attribuer des valeurs initiales aux ensembles de variables suivants :

   1. l'activation des tissus $A(t) = \left[A(r_g, t)\right]_{r_g \in \Re_g}$ pour chaque point $r_g$ de $\Re_g$ ;

   2. les indicateurs fonctionnels $f_m(t) = \left[f_m(r_g, t)\right]_{r_g \in \Re_g}$ pour chaque point $r_g$ de $\Re_g$, où $f(t) = [f_m(t)]_{1 \leq m \leq M}$ désigne la totalité des modalités de mesure obtenues pour le sujet ;
   3. l'hyperparamètre de l'activation $\Theta_A$ ;
   4. l'hyperparamètre du bruit intrinsèque des indicateurs fonctionnels $\Theta_\beta = [\Theta_{\beta_m}]_{1 \leq m \leq M}$ ;
   5. l'hyperparamètre du bruit des instruments $\Theta_e = [\Theta_{e_m}]_{1 \leq m \leq M}$ ;

   b) calculer la probabilité a posteriori :

$$P(A(t), \Theta_A, f(t), \Theta_\beta, \Theta_e | o(t)) \propto \quad \prod_{m=1}^{M} l_{o_m}(o_m(t) - K_m * f_m(t) | \Theta_{e_m}) \times$$

$$\prod_{m=1}^{M} \pi_{f_m}(f_m(t) - H_m \circ A(t) | \Theta_{\beta_m}) \times$$

$$\prod_{m=1}^{M} \left\{ \pi_{\Theta_{e_m}}(\Theta_{e_m}) . \pi_{\Theta_{\beta_m}}(\Theta_{\beta_m}) \right\} \times$$

$$\pi_A(A(t) | \Theta_A) \times \pi_{\Theta_A}(\Theta_A)$$

   qui comprend les termes multiplicatifs suivants :

   1) les termes de vraisemblance $l_{o_m}$ pour chaque modalité m $\quad l_{o_m}(o_m(t) - K_m * f_m(t) | \Theta_{e_m})$

   2) les probabilités a priori pour les indicateurs fonctionnels $\quad \pi_{f_m}(f_m(t) - H_m \circ A(t) | \Theta_{\beta_m})$

   3) la probabilité a priori pour l'activation $\pi_A(A(t) | \Theta_A)$
   4) la probabilité a priori pour les hyperparamètres de l'activation $\pi_{\Theta_A}(\Theta_A)$

   5) les probabilités a priori pour les hyperparamètres du bruit intrinsèque des indicateurs fonctionnels

$$\pi_{\Theta_{\beta_m}}(\Theta_{\beta_m})$$

   6) les probabilités a priori pour les hyperparamètres du bruit des instruments $\pi_{\Theta_{e_m}}(\Theta_{e_m})$

   c) modifier de manière itérative les paramètres A(t), $\Theta_A$, f(t), $\Theta_\beta$, $\Theta_e$ jusqu'à ce qu'une valeur maximale de la probabilité a posteriori soit obtenue, ladite modification étant effectuée selon l'un des mécanismes suivants :

   1. Maximisations successives de $P(A(t), \Theta_A, f(t), \Theta\beta, \Theta e | o(t))$ pour des sous-ensembles de paramètres, tout en maintenant tous les autres fixes, "Maximisation Conditionnelle par Itérations" (MCI) ;
   2. Maximisations successives de $P(A(t), \Theta_A, f(t), \Theta_\beta, \Theta_e | 0(t))$ pour des sous-ensembles de paramètres, en fixant les autres paramètres à leur valeur espérée, "Maximisation par Espérances" (ME) ;
   3. Choix du mode de distribution de $P(A(t), \Theta_A, f(t), \Theta_\beta, \Theta_e | o(t))$ obtenu au moyen des méthode Monte Carlo par Chaînes de Markov (MCCM).

**7.** Procédé selon la revendication 6, dans lequel les observations électrophysiologiques $o_m(t)$ m = 1, 2 pour l'estimation de $f_1(t)$ comprennent l'une quelconque des combinaisons suivantes : EEG seul, MEG seul, EEG et MEG ensemble.

**8.** Procédé selon la revendication 6, dans lequel :

- soit aucune des images fonctionnelles $o_m$(t) m = 3, 4, 5 n'est utilisée pour l'estimation de $f_1$(t), soit, d'une autre manière,
- certaines de ces mesures sont utilisées dans l'une quelconque des combinaisons suivantes : IRMf seule, TEP seule, TEPU seule, IRMf et TEPU, IRMf et TEPU, TEP et TEPU.

**9.** Procédé selon la revendication 6, dans lequel le calcul des vraisemblances $l_{o_m}(o_m(t) - K_m * f_m(t)|\Theta_{e_m})$ comprend les étapes consistant à:

a) sélectionner l'utilisation d'un risque polynomial insensible $\varepsilon$ ou bien d'un risque polynomial généralisé $R_x$ $[x|\Theta_x]$ dans le but spécifique d'évaluer des vraisemblances non gaussiennes ;
b) calculer le vecteur de divergence $\Delta = o_m(t) - K_m * f_m(t)$ ;
c) remettre le $\Delta$ à l'échelle en fonction des hyperparamètres $\Theta_{e_m}$ ;

d) calculer $R_x^c\left[\Delta|\Theta_x\right]$, le risque de $\Delta$ ;

e) calculer $l_{o_m}\left(\Delta|\Theta_{e_m}\right) = \exp\left(-B\,R_x^c\left[\Delta|\Theta_x\right]\right)$, où B est une constante présélectionnée.

**10.** Procédé selon la revendication 6, dans lequel le calcul de la probabilité a priori $\pi_{f_m}(f_m(t)-H_m \circ A(t)|\Theta_{\beta_m})$ pour les indicateurs fonctionnels comprend les étapes qui consistent à :

a) sélectionner les indices m, n, s de l'espace de Besov $B_{n,s}^m$ dans le but de définir le lissé de l'indicateur fonctionnel estimé, un choix particulier mais non exclusif étant m = 1, n = 1, s = 1, ce qui définit "l'algèbre des bosses" ;
b) calculer le vecteur divergence $\Delta = f_m(t) - H_m \circ A(t)$ ;
c) remettre le $\Delta$ à l'échelle en fonction des hyperparamètres $\Theta_{\beta_m}$ ;

d) calculer $\left\|\Delta\right\|_{B_{n,s}^m}$, la norme du $\Delta$ dans $B_{n,s}^m$,

ledit calcul étant effectué tout d'abord par développement de $\Delta$ sous la forme de $\Delta = \sum_k F_k.\psi_k$, où le $\psi_k$

appartient à un Dictionnaire d'Atomes (dont des exemples particuliers mais non exhaustifs sont le dictionnaire des ondelettes, le dictionnaire des fonctions de Fourier, le dictionnaires des fonctions de dirac delta, ou un méga-dictionnaire défini par l'union de plusieurs dictionnaires), puis calcul de $\left\|\Delta\right\|_{B_{n,s}^m} \approx \sum_k \alpha_k.\left\|F_k\right\|^m$ ;

e) calculer $\pi_{f_m}\left(\Delta|\Theta_{\beta_m}\right) = \exp\left(-C\left\|\Delta\right\|_{B_{n,s}^m}\right)$, où C est une constante présélectionnée.

**11.** Procédé selon la revendication 6, dans lequel le calcul de la probabilité a priori de l'activation $\pi_A(A(t)|\Theta_A)$ comprend les étapes qui consistent à :

a) calculer une fonction non décroissante h($\square$) de l'atlas probabiliste h($p_g$);

b) calculer la norme $\left\|A(t)\right\|_{B_{n,s}^m}$, de la manière spécifiée dans la revendication 10 ;

c) calculer $\pi_A\left(A(t)|\Theta_A\right) = \exp\left(-D\,h(p(s,r_g)) - E\left\|A(t)\right\|_{B_{n,s}^m}\right)$, où D et E sont des constantes préalablement spécifiées.

**12.** Procédé selon la revendication 6, dans lequel la probabilité a priori pour le CEP prend la forme spécifique suivante :

$$\pi_{f_1}(f_1(t) - H_1 \circ A(t)|\Theta_{\beta_1}) = \pi_{f_1}(f1(t) - M\,A(t)|\Theta_1)\pi_2(\mu|\Theta_2)$$

où $\pi_1$ et $\pi_2$ sont les probabilités a priori spécifiées selon la revendication 10,

$$M = \begin{bmatrix} \mu_1 & 0 & \cdots & 0 & 0 \\ 0 & \mu_2 & \cdots & 0 & 0 \\ \cdots & \cdots & \mu_i & \cdots & \cdots \\ 0 & 0 & \cdots & \mu_{N_g-1} & 0 \\ 0 & 0 & \cdots & 0 & \mu_{N_g} \end{bmatrix}, \quad \mu = \begin{bmatrix} \mu_1 \\ \mu_2 \\ \cdots \\ \mu_{N_g} \end{bmatrix},$$

où les $\mu_g$ sont les orientation du CEP pour chaque point de $\Re_g$.

13. Procédé selon la revendication 6, dans lequel la probabilité a priori $\pi_2(\mu|\Theta_2)$ définie dans la revendication 12 est calculée par l'une ou l'autre des expressions suivantes :

- $\pi_2(\mu|\Theta_2) = \exp\left(-\left\|\Lambda_s.\mu.\Lambda_m\right\|_{B_{n,s}^m}\right)$, où $\Lambda_s$ est une matrice diagonale qui spécifie le degré de lissé qui sera imposé à chaque point de $\Re_g$, et $\Lambda_m = W.P$, où W est une matrice de pondération préalablement spécifiée et P est une matrice diagonale contenant les $p_g$ pour tous les $r_g$ ;

- au moyen d'évaluations successives des distributions marginales de

$\pi_2(\mu_g|\Theta_2) = p_g.N(0,\sigma_A^2) + (1-p_g)N(0,\sigma_B^2)$, avec $N(0,\sigma^2)$ la distribution gaussienne invariante ayant comme moyenne zéro et comme écart-type $\sigma$, $\sigma_A^2$ et $\sigma_B^2$ étant des constantes sélectionnées de manière à être respectivement grande et petite par rapport à la variation attendue des orientations.

14. Procédé selon la revendication 6, dans lequel les calculs sont effectués pour $o_1(t)$ et/ou $o_2(t)$, dans le domaine fréquentiel, par spécification :

- du fait que les signaux électriques ont été soumis à la transformée de Fourier, t désignant maintenant la fréquence ; $l_{o_m}(o_m(t) - K_m * f_m(t)|\Theta_{e_m}) = N_R^{2,p}(0,\Sigma_e)$, où $N_R^{2,p}(\mu_x,\Sigma_x)$ désigne la distribution gaussienne réelle multivariante, ayant pour moyenne et variance ($\mu x, \Sigma_x$);

- $\pi_{f_m}(f_m(t) - H_m \circ A(t)|\Theta_{\beta_m}) = N_R^{2,p}(0,0), H_m = I$, la matrice identité ;

- $\pi_A(A(t)|\Theta_A) = N_R^{2,p}(0,\Sigma_A)$ ;

- $\pi_{\Theta_A}(\Theta_A) = \left\|\Sigma_A\right\|^{-\alpha/2} \exp(-1/2\,\mathrm{Tr}(Q\Sigma_A^{-1}))$ une distribution a priori conjuguée naturelle pour les matrices de Wishart complexes, où $\alpha$ et Q sont préalablement spécifiés en fonction de connaissances antérieures ;

- $\pi_{\Theta_{\beta_m}}(\Theta_{\beta_m})$ et $\pi_{\Theta_{e_m}}(\Theta_{e_m})$ sont des constantes définissant des valeurs antérieures incorrectes.

15. Procédé selon la revendication 1, dans lequel le calcul de l'élément $I_{B \to A/C}$ qui quantifie l'influence dirigée de la région B du cerveau ou du coeur sur la région A, qui n'est pas due à l'activité provenant de la région C, comprend les étapes qui consistent à :

a) définir les régions A, B, C sous la forme d'ensembles de voxels arbitraires $\mathbf{r_i} \in \mathfrak{R_g}$ choisis dans le volume de génération ;

b) définir, pour toute modalité d'imagerie donnée m, la matrice de passage $\Gamma_i^q = \left[\gamma_m(t-k)\right]_{1 \leq k \leq q}$ ;

c) calculer les résidus $\varepsilon\left(\Gamma_t^q, t\right)$ d'un modèle autorégressif, sur un quelconque cerveau donné, en ajustant le modèle $\gamma_t = F\left(\Gamma_t^q, t\right) + \varepsilon\left(\Gamma_t^q, t\right)$, au moyen d'une fonction non paramétrique F, et en utilisant l'expression :

$$\varepsilon\left(\Gamma_t^q, t\right) = F\left(\Gamma_t^q, t\right) - \gamma_t \ ;$$

d) calculer la matrice $\Sigma_\gamma\left(\Gamma_t^q, t; A | B.C\right)$ sous la forme de la covariance des résidus du modèle autorégressif non paramétrique sur l'ensemble constitué de l'union des régions A, B et C ;

e) calculer la matrice $\Sigma_\gamma(\Gamma_t{}^q, t; A|B.C)$ sous la forme de la covariance des résidus du modèle autorégressif non paramétrique sur l'ensemble constitué de l'union des régions A et C, sans la région B ;

f) calculer l'influence de la région A sur la région B lorsque l'influence de la région C est éliminée, sous la forme de l'expression : $I_{B \to A/C} = \ln\left(\dfrac{\left|\Sigma_\gamma\left(\Gamma_t^q, t; A|\{ \ \}.C\right)\right|}{\left|\Sigma_\gamma\left(\Gamma_t^q, t; A|B.C\right)\right|}\right)$ sous la forme du rapport logarithmique des déter-

minants des matrices $\Sigma_\gamma\left(\Gamma_t^q, t; A|B.C\right)$ et $\Sigma_\gamma\left(\Gamma_t^q, t; A|\{ \ \}.C\right)$.

**16.** Procédé défini dans la revendication 1, dans lequel les calculs mis en jeu comprennent les étapes consistant à :

a) spécifier des groupes de sujets de référence pour lesquels des paramètres descriptifs $\gamma_m(t)$ ont été obtenus et pour lesquels certaines covariantes ont été mesurées ; lesdites covariantes (dont un exemple spécifique mais non exclusif est l'âge) étant désignées par $\bar{\omega}$ ;

b) transformer, vers la gaussianité, les paramètres descriptifs du sujet examiné ainsi que ceux des sujets des groupes de référence au moyen de l'expression : $\gamma'_m(t) = T(\gamma_m(t))$, dans laquelle les paramètres descriptifs $\gamma_m(t)$ sont transformés par une fonction non linéaire T pour donner le vecteur $\gamma'_m(t)$ ;

c) calculer la moyenne conditionnelle $\mu(\gamma'_m ; \bar{\omega})$ et la variance conditionnelle $\sigma(\gamma'_m; \bar{\omega})$ de chaque paramètre descriptif sous la forme d'une fonction des paramètres de réglage $\bar{\omega}$, dans chaque groupe de référence, au moyen d'une régression non paramétrique ;

d) calculer la transformée z de chaque paramètre descriptif au moyen de l'expression :

$$z = \left(\dfrac{\gamma'_m - \mu(\gamma'_m ; \varpi)}{\sigma(\gamma'_m ; \varpi)}\right) \text{ où chaque quantité est évaluée en chaque point du treillis de génération } \mathfrak{R_g} \ ;$$

e) calculer les probabilités que $z_{max}$ et $z_{min}$, les valeurs maximale et minimale de z dans une région d'intérêt C, excèdent respectivement des valeurs seuil $U_{max}$, $U_{min}$ ; lesdits seuils $U_{max}$, $U_{min}$ étant respectivement des seuils choisis en fonction de connaissances antérieures ;

f) calculer les quantités transformées suivantes :

$$\begin{cases} z' = F^{-1}\big(P(z)\big) & \text{si } z \geq U_{max} \\ z' = 0 & \text{si } U_{min} < z < U_{max} \\ z' = F^{-1}\big(P(z)\big) & \text{si } z \leq U_{min} \end{cases},$$

où $F^{-1}$ est la fonction gaussienne inverse ;

g) afficher les courbes z, définies sur des graphes dimensionnels de z' tracés pour un point fixe du treillis de génération $\mathfrak{R}_g$ et comme une fonction du temps ou de la fréquence ; le dernier cas étant appelé "spectre z" de la tomographie du CEP ;

afficher les valeurs z' sous la forme d'images bidimensionnelles ou tridimensionnelles pour un instant fixe ou une fréquence fixe, ladite image étant appelée "image z" de la tomographie du CEP, l'image étant rendue selon un code couleur qui indique le degré de déviation des valeurs par rapport aux groups de référence, ladite image étant superposée sur un atlas anatomique, qui constitue l'arrière-plan de l'image.

Fig. 1

a)

b)

c)

d)

Fig. 2

Fig. 3

Fig. 4

Fig. 5

Figura 6

Fig. 7

Fig. 8

EP 1 005 677 B1

Fig. 9

Figura 10

Figura 11

EP 1 005 677 B1

Fig. 12

Fig. 13